# EUROPEAN PATENT APPLICATION

(11) **EP 4 549 915 A1**
(43) Date of publication of application: **07.05.2025**
(21) Application number: 24210438.8
(22) Date of filing: 01.11.2024
(51) Int. Cl.: G01N 21/65, G01N 21/64, G01N 1/00

(54) **METHODS AND APPARATUS FOR TRACE CHEMICAL AND BIOLOGICAL DETECTION FROM WATER OR OTHER LIQUID SAMPLES**

(30) Priority: 02.11.2023 US 202363595747 P
(71) Applicant: Photonsystems, Inc., Covina, CA 91722 (US)
(72) Inventor: Hug, William F., Covina, 91722 (US); Bhartia, Rohit, Covina, 91722 (US); Reid, Ray D., Covina, 91722 (US); Nguyen, Ken, Covina, 91722 (US); Nguyen, Quoc, Covina, 91722 (US)
(74) Representative: Schlee, Alexander Richard

(57) **Abstract**

Compact and portable, real-time or near real-time, in-line, on-line, at-line, or off-line reagentless detection, identification, and/or quantification spectroscopic methods and sensor systems provide detection and quantification of chemical and biological (CB) materials or analytes (whether harmful or beneficial) at trace quantities and concentrations (e.g., at less than 10 ppb, 10 ppt, or 10 ppq). Some embodiments detect trace volumes of materials of interest that were originally in water or another liquid wherein detection is enhanced by one or more of (1) liquid reduction or elimination, (2) overlaid multi-droplet deposition, (3) analyte spatial separation, and/or (4) creation and storage of analyte sample archives in a readily accessible manner for future re-examination.

## Description

### Field of the Invention

The field of the invention is real-time and near real-time, reagentless sensors for use in detecting, identifying, and quantifying trace quantities of harmful and/or beneficial chemical and biological materials in water or other liquids whether the analytes to be evaluated originated in the liquid or were originally on a surface or in the air and were obtained via entrapment in a liquid (e.g., whether the sample came directly from a liquid source under study or whether the liquids were added as an aid in sample collection of analytes from the air or from a surface). More specifically, the field of the invention involves spectroscopic detection and analysis, and even more specifically, quantitative spectroscopic detection and analysis. In some embodiments, the field more specifically involves one or more of compact and portable detection systems, compact capture and retention of deposition samples, overlaid depositions of precisely known sample volumes, reduction or removal of carrier media prior to analyte detection, use of deep UV, and/or use of deep UV fluorescence detection.

### Background of the Invention

Methods, Apparatus, and Systems of the present invention relate to spectroscopic detection systems and calibration systems such as those taught in the following US Patents.

| **Docket No.** | **Inventors, "Title" (Docket)** |
|---|---|
| **App No. & App. Date** | |
| **Pat. No. & Pat. Date** | |
| 09/250,820 - Feb. 17, 1999 | Hug, William et al., "Analytical Instrument Using a Sputtering Metal Ion Laser". |
| 6,287,869 - Sep 11, 2001 | |
| 11/245,486 - Oct. 5, 2005 | Hug, William et al., "Spectroscopic Chemical Analysis Methods and Apparatus". |
| 7,525,653 - Apr. 28, 2009 | |
| 12/545,772 - Aug. 21, 2009 | Hug, William et al., "Spectroscopic Chemical Analysis Methods and Apparatus". |
| 8,395,770 - Mar. 12, 2013 | |
| 12/628,205 - Nov. 11, 2009 | Hug, William et al., "Native Fluorescence Detection Methods and Detectors for Naphthalene and/or Other Volatile Organic Compound Vapors". |
| 8,759,791 - Jun. 24, 2014 | |
| 14/313,994 - Jun 24, 2014 | Hug, William et al., "Native Fluorescence Detection Methods, Devices, and Systems for Organic Compounds". |
| 9,442,070 - Sep 13, 2016 | |
| 16/290,002 - Mar. 1, 2019 | Reid, Michael et al., "Methods and Apparatus for Spectroscopic Identification and/or Calibrated Quantification of Surface Concentration of Materials". |
| 10,732,037 - Aug 4, 2020 | |
| 17/374,902 - Jul 13, 2021 | Bhartia, Rohit et al., "Methods and Systems for Detection of Biohazard Signatures in Complex Clinical and Environmental Samples". |
| 11,448,598 - Sep. 20, 2022 | |

Many types of instruments presently exist which purport to accurately measure harmful or beneficial materials of interest. These instruments suffer from a wide range of shortcomings including: (1) lack of sensitivity, (2) lack of selectivity, (3) lack of range of chemical and biological application, (4) unapplicable size, (5) unapplicable weight, (6) unapplicable power consumption, (7) unapplicable cost (SWaP-C), and/or (8) limited extended autonomous operation capability.

Chemical and biological analyte materials present themselves for detection while contained in a liquid sample, in air, or on a surface. Materials contained within air or on surfaces are often converted to liquid bearing samples so that the detection process occurs within or from a liquid medium. Trace concentrations of analyte materials in many cases need to be detectable in the part per billion (ppb) to part per trillion (ppt) range, and below. Detection at these levels is presently possible only within a well-equipped chemical laboratory with highly skilled operators, but not by autonomous in-line, on-line, at-line or off-line rapid detection, identification, and quantification instruments. A need exists for improved apparatus and methods that can provide ppb or ppt detection on an in-line, on-line, at-line, or off-line basis particularly for rapid detection, identification, and/or quantification.

### Harmful Materials Detection

Harmful material detection applications include detection and identification of a wide range of inorganic, organic, and biological contaminants in wastewater treatment plants, potable water processing plants, aquatic and factory farming facilities, and other water or liquid related processing plants. Harmful material detection is also needed in the production of food and beverage products, fermented products, pharmaceutical products, semiconductor products, and a wide range of other commercial and consumer products including cell phones, high performance batteries, high end optical components, and the like. Such detection is used in manufacturing facilities, environmental and ecological testing, and research and development activities.

Harmful materials detection in wastewater treatment plants includes detection of nitrates, nitrites, phosphates, sulphates, ammonium, pesticides, pharmaceutical and related drugs, and drug ingredients, polyfluoroalkyl substances (PFAs), plasticizers and microplastics, biological material including bacteria, viruses, hormones, and other harmful organic and inorganic micropollutant materials. Present wastewater treatment plant methods require collection of water grab-samples and testing at remote laboratory facilities, with time periods of days to weeks between tests. During these long periods between gathering and testing, many factors can cause dramatic changes in the actual water contamination content. As a result, overtreatment of wastewater is a common practice to avoid exceeding regulatory levels which in turn results in excessive use of and costs for electrical energy, and consumables such as chemical agents, biological agents, and filtering materials. This overtreatment may also result in excess emissions of harmful gases. A need exists for improved methods of testing and getting test results so that more timely actions can be taken to improve water treatment, make water treatment more efficient, more sensitive, and/or more effective.

Similar problems exist for manufacturing of consumer, commercial, and industrial products, where randomized but infrequent testing is often employed to determine the quality of a product or components that are going into the product. Example application areas include aqua or factory farming, potable water and beverage manufacturing, food manufacturing, fermented products manufacturing including probiotics, yogurts, and other products. As part of a process to provide higher quality products, more frequent chemical and biological testing would be beneficial particularly if it can be provided with a quicker turnaround time, in a more efficient, and potentially a more cost-effective manner. A need exists for enabling technology that would allow commercially viable implementation of such chemical and biological testing.

Detection and identification of harmful materials is also important to cleaning verification at various levels of hardware product manufacturing including product contamination and cleaning of manufacturing equipment in pharmaceutical, food, or beverage manufacturing or manufacturing of sophisticated products like cell phones, watches, optical components, etc. In these applications, as well as in others, detection may be accomplished directly form spectroscopic analysis of a product or by swabbing or wiping to accumulate analytes and then rinsing the swab or wipes with a liquid medium to collect the analyte/liquid combination as a sample for testing. A need exists for enabling commercially viable enhancements in cleaning verification.

Similar contaminants are also of concern in natural environments, such as in soil or in water in lakes, rivers, reservoirs, and wells, etc. and a need exists for enabling improvements in providing detection in natural environments.

### Beneficial Materials Detection

Beneficial materials detection applications include detecting the chemical or biological product itself, instead of possible contaminants. Examples of beneficial detection applications include continuous and near continuous measurement of the concentration of active pharmaceutical and biopharmaceutical materials and/or their precursors, during manufacturing of drugs to improve the consistency and efficacy of drugs provided to consumers. Other beneficial ingredient detection applications include measurement of other manufactured chemical and biological materials such as, for example, brewed products, probiotics, food, and beverages. In some cases, required limits of the detection drop into the parts per billion (ppb) range, the parts per trillion (ppt) range or potentially even into the parts per quadrillion range (ppq). A need exists for improved beneficial material detection to support improved product quality.

### Methods of Detection

The detection of trace amounts of materials has been ongoing for many years and includes technologies such as Rayleigh, Raman, laser induced breakdown spectroscopy (LIBS), laser induced fluorescence (LIF), infrared, photoacoustic, and terahertz spectroscopies. Each of these technologies has specific advantages and disadvantages. However, after elimination of technologies that have unacceptable size, weight, power, and cost (SWAP-C), there is a diminishing list of potential useful candidate technologies for a wide range of reasons. Some principal limitations of these various technologies reside in: (1) a lack of chemical or biological specificity (without the need for recalibration due to changes in matrix or other changes), (2) high limits of detection, (3) sample damage and spectral alteration of samples during excitation, (4) obscuration of target spectral signatures due to interference from fluorescence emission or other mechanisms, (5) obscuration of target spectral signatures due to interference from other adjacent chemical species within or surrounding the target, and/or (6) obscuration of target spectral signatures due to interference from ambient natural and artificial light, heat, and other sources of interferences. These technologies cover a wide range of the electromagnetic spectrum from about 200 nanometers (nm) to 30 millimeters (mm) or 30 million nm. Technologies in the near UV, visible, and IR (including near, mid, and far IR and mm-waves) have the greatest limitations. Near UV, visible and near IR Raman methods have problems with fluorescence interference or obscuration of Raman emissions by many materials found in many applications. Mid IR and far IR methods have problems with depth of penetration in water or liquids as well as interference from ambient light and heat interferences. Millimeter-wave methods have the greatest interference since thermal radiation from non-targeted materials occurs within the range of detection.

Technologies in the UV wavelength range have limitations due to damage and spectral alteration of samples during excitation as well as obscuration due to fluorescence from target material and its surroundings. Compared to the limitations of other detection wavelength regions, these are more tractable and resolvable with excitation in the deep UV below 280 nm and especially below 250 nm.

Both LIBs and Raman spectroscopy methods push the excitation energy density to limits of sample damage. However, LIBS damage is in the extreme while Raman is considered minimal. In the extreme case of LIBs, the method by its nature is to evaporate, ionize, and excite samples to temperatures of tens of thousands of degrees centigrade to produce an ionized plasma of the combined materials within the excitation volume. This includes not only target but also surrounding materials within the excitation volume. The decay of this plasma burst produces Bremsstrahlung radiation from which the atomic elemental emissions within the plasma can be identified. With trace concentration of target material within this plasma, it is difficult to determine the molecular composition of the original sample which leads to high error rates of detection. Thermal/electrical breakdown of a sample is caused by direct breaking of bonds by incident radiation, or the adiabatic heating of a sample volume produced when laser excitation pulse widths are less than a few nanoseconds (ns) and excitation power levels are many 10s of kilowatts (kW). This process is typical of Q-switched and excimer lasers where sample damage can occur with only a few nanojoules (nJ) of energy, even when the excited volume is imbedded in media with high thermal diffusivity. The drawbacks of these approaches can be addressed by careful application of excitation energy which is provided for in some embodiments of the invention. A need exists for improved methods of trace material identification and quantification.

Needs for improved methods, systems, and apparatus exist that can be used in providing one or more of: (1) real-time or near real-time autonomous detection that can be measured in periods of hours or minutes or less as opposed to days or weeks, (2) improved autonomous operation capability, (3) detection with improved efficiency, convenience, lower cost, smaller size, lower power consumption, improved industrial design and durability, and the like, (4) detection with measurements providing improved detection sensitivity limits down ten of parts per billion (ppb) or less, tens of parts per trillion (ppt) or less, and even down to tens of parts per quadrillion (ppq) or less, (5) improved sample manipulation, (6) improved autonomous run time, and/or (7) improved retention and storage reliability of samples, more efficient storage of such samples, and/or easier accessibility of such samples for retesting.

### Summary of the Invention

It is an object (a first object) of some embodiments of the invention to provide a reliable, compact, and portable sensor system to provide real-time or near real-time detection of trace and ultra-trace amounts of chemical or biological materials having an in-situ presence in water at the time a sample is being taken or gathered for testing purposes.

It is an object (a second object) of some embodiments of the invention to provide a reliable, compact, and portable sensor system to provide real-time or near real-time detection of trace and ultra-trace amounts of chemical or biological materials that did not have a presence in water at the time of sample gathering (e.g., it was in a liquid other than water, it was in air or some other gaseous medium, or it was on a surface of a solid at the time of same gathering).

It is an object (a third object) of some embodiments of the invention to provide a method, apparatus, and/or system for providing the detection according to one of the first or second objects along with permanently capturing and recording trace and ultra-trace amounts of chemical or biological materials from each of multiple samples taken to form a sample history where the samples are each capable of being retested for verification purposes or as retested as part of completely new evaluations. As used herein, permanent refers to capturing and recording analyte samples for a period of time so that such samples may be re-examined one or more times in the future after completion of an initial examination where retention may extend for weeks or longer, months or longer, or even years in some cases.

It is an object (a fourth object) of some embodiments of the invention to provide the capture and recording of the third object in the form of a chemical and/or biological (CB) structure (CBS) device or disc (CBD) or hard drive (CBHD) (hereafter generally referred to as CBD) on which physical archives of each of many sample depositions are collected and stored (e.g. hundreds to tens of thousands of 50 to 500 micron or micrometer (µm or um) diameter analyte depositions - e.g. nominally 150 µm diameter analyte depositions) to provide a historical physical record of analyte concentrations taken over many days, weeks, months, or years, to be used to enable future re-testing and validation, potentially using improved future detection hardware and software. Along with the physical sample record, additional information is preferably captured and retained, e.g., as a separate digital record held on a separate digital memory or hard drive that may provide both spectroscopic information and relevant ancillary information related to each physical CB sample and/or as additional encoded markings or depositions applied directly to the CBD which can be decoded and interpreted by an appropriate optical, magnetic, or other reader.

It is an object (a fifth object) of some embodiments of the invention to provide analyte concentration, analyte separation, carrier media reduction or complete media removal in a rapid manner (e.g. in sub-second, seconds, or minutes) while still allowing for accurate quantity or surface/volume concentration determination of the analyte as it originally existed and wherein this object may be achieved separately or in conjunction with one or more of the first to fourth objects.

It is an object (a sixth object) of some embodiments to implement some features of the fifth object to automatically reduce or eliminate background signals from a liquid media that transports an analyte, wherein the elimination occurs by evaporation of all or part of the liquid media quickly as a result of it being deposited in small volumes (e.g. droplets of precisely known volume) on a substrate covering an area similar in size to the optical detection area (e.g., less than about 50 to 500 µm diameter), thereby enhancing the signal to background noise ratio and thus reducing the limit of detection (LOD) to enhance the ability to detect smaller concentrations of an analyte using a deep UV, near-UV, visible, or IR spectroscopic measurement.

It is an object (a seventh object) of some embodiments of the invention to implement some features of the fifth object to automatically concentrate samples in a sequential process of evaporation and resuspension into a lesser volume (in the same media or possibly in a different media) to increase concentration to improve the LOD with reduced, or even without, interference from the original liquid media.

It is an object (an eighth object) of some embodiments of the invention to implement some features of the fifth object to automatically concentrate samples in a sequential process of depositing picoliter (pL), nanoliter (nL), or microliter (µL or uL) volumes (e.g. 100s of pLs, 1s nLs, 10s of nLs, or 100s of nL) of sample material onto a substrate, including rapidly drying the deposited sample due to the high surface to volume ratio of the deposited droplet, and sequentially depositing more volumes within the same deposition area on a substrate to increase concentration to effectively reduce the LOD by reducing or eliminating interference from the original liquid media.

It is an object (a ninth object) for some embodiments of the invention to have the detections and a preliminary analysis, and perhaps a final analysis completed in real-time (e.g. within seconds or minutes) or near real-time (e.g. within tens of minutes or possibly hours) using spectroscopic methods using in-line, on-line, at-line, and/or off-line monitoring and analysis paradigms, where in-line refers to sensing or measuring while a material is undergoing a process without sample removal, where on-line refers to automatically taking samples to be analyzed without stopping the process where the sample is intended for substantially immediate (e.g. within seconds or minutes) and proximal testing (for example adjacent to the processing area or within feet, tens of feet, or within the same building), where at-line refers to taking samples from the process stream and taking those samples to separate but local detection and analysis stations (e.g. a separate lab or building or perhaps a mobile station which is outside the building or facility in which the process is occurring), and where off-line refers to taking samples from the process stream and sending them to a lab for analysis perhaps tens of hours or days after original acquisition of the sample. Real-time and near real-time may fall within the time period noted above but they may alternatively, be defined by a time-scale or time constant that exists for a given process where the real-time results are provided within a time period such that a parameter under investigation can't change by more than 10%, 5%, 2%, or even 1% while near-real time may refer to period of time greater than real-time but less than 20%, 10%, 4%, or 2% respectively.

In a first aspect of the invention a method of spectroscopic analysis includes: (a) obtaining a liquid sample to be tested; (b) providing a substrate; (c) depositing a known volume of the liquid sample to a predefined location on the substrate; (d) allowing or causing at least a portion of the liquid to evaporate from the sample to provide an analyte sample for testing; (e) directing excitation radiation on to the analyte sample; (f) detecting resulting emission radiation coming from the analyte sample; and (g) analyzing emission radiation spectra to provide a determination concerning the presence of any biological or chemical analytes of interest.

In a second aspect of the invention a method of spectroscopic analysis includes: (a) obtaining a liquid sample to be tested; (b) providing a substrate; (c) depositing a defined volume of the liquid to a predefined location on the substrate where the defined volume is less than a total volume to be deposited; (d) thereafter repeating the depositing operation one or more times so as to deposit the total volume on to the predefined location on the substrate while allowing time between depositing operations or after the total volume has been deposited to allow at least a portion of the liquid to evaporate to leave an analyte sample (e.g., a sample including a concentrated amount of analyte which may be dry or which still may be contained in a reduced volume of liquid); (e) directing excitation radiation on to the analyte sample; (f) detecting resulting emission radiation coming from the analyte sample; and (g) analyzing emission radiation spectra to provide a determination concerning the presence of any biological or chemical analytes of interest.

In a third aspect of the invention a method of spectroscopic analysis includes: (a) obtaining a liquid sample to be tested; (b) providing a substrate; (c) depositing a known volume of the liquid sample to a predefined location on the substrate and allowing or causing different analytes to spatially separate after deposition to create a distributed analyte sample; (d) allowing or causing at least a portion of the liquid to evaporate from the sample to provide an analyte sample for testing; (e) directing excitation radiation on to the analyte sample; (f) detecting resulting emission radiation coming from different regions of the distributed analyte sample; and (g) analyzing emission radiation spectra from the different regions of the distributed analyte sample to provide an integrated determination concerning the presence of any biological or chemical analytes of interest within the total sample.

In a fourth aspect of the invention a method of spectroscopic analysis for a plurality of different liquid samples includes creation of a multi-sample analyte storage device or disc, includes: (a) obtaining a liquid sample to be tested; (b) providing a substrate; (c) depositing a known volume of the liquid sample to a predefined location on a substrate; (d) allowing or causing at least a portion of the liquid to evaporate from the sample to provide an analyte sample for testing; (e) directing excitation radiation on to the analyte sample; (f) detecting resulting emission radiation coming from the analyte sample; (g) analyzing emission radiation spectra to provide a determination concerning the presence of any biological or chemical analytes of interest; and (h) repeating the operations of (a) - (g) for a plurality of additional liquid samples wherein the depositing of each liquid sample will occur at a different defined location on the substrate relative to previously dispensed droplets and wherein the substrate with its plurality of depositions will form a permanent chemical and biological storage device or disc (CBD) that may be accessed in the future for providing updated analysis.

In a fifth aspect of the invention a method of spectroscopic analysis for a plurality of different liquid samples includes creation of a multi-sample analyte storage device, including: (a) obtaining a liquid sample to be tested; (b) providing a substrate; (c) depositing a known volume of the liquid sample to a predefined location on a substrate, wherein a volume of the liquid sample is selected from the group of: (1) a volume equal to a total volume to be deposited and (2) a volume less than the total volume to be deposited and wherein positions of different analytes in the deposited volume is selected from a group of: (1) substantially overlapping positions or symmetric positioning driven primarily by deposition and drying and (2) nonsymmetric, spatially offset positions driven by a non-symmetric motive force in combination with deposition and drying; (d) repeating (c) as necessary until the total volume is deposited while allowing time between depositing operations or after the total volume is deposited to allow or cause at least a portion of the liquid to evaporate to provide an analyte sample; (e) directing excitation radiation on to the analyte sample; (f) detecting resulting emission radiation coming from the analyte sample as a whole or from multiple regions of the sample; (g) analyzing emission radiation spectra to provide a determination concerning the presence of any biological or chemical analytes of interest; and (h) repeating the operations of (a) - (g) for a plurality of additional liquid samples wherein the depositing of each liquid sample occurs at a different defined location on the substrate relative to previously dispensed droplets and wherein the substrate with its plurality of depositions will form a permanent chemical and biological storage device or disc (CBD) that may be accessed in the future for providing updated analysis.

In a sixth aspect of the invention a sensing method for trace and ultra-trace concentrations of chemical and biological materials includes: (a) providing an addressable substrate capable of receiving a plurality of spatially separated deposits of liquids from samples to be tested; (b) depositing a first sub-microliter (e.g. 5-50 nL) volume of liquid that may contain one or more analytes of interest on a selected deposition location on the substrate; (c) sequentially depositing a plurality of additional volumes (e.g. droplets) vertically upon previously dispensed deposits, wherein a drying time between dispensing successive droplets allows for at least partial evaporation of the liquid so that liquid at the dispensing location does continue to increase in volume with each successive droplet, and wherein the overlaying of depositions allows for thickening of the analyte deposition wherein a diameter of analyte deposition is between 50 µm and 500 µm; (d) providing excitation radiation onto the thickened analyte material deposition wherein the excitation radiation has a wavelength in the deep ultraviolet, near ultraviolet, visible, or infrared; (e) receiving at least one of Rayleigh, Raman, resonance Raman, surface plasmon resonance enhanced Raman native fluorescence, or phosphorescence emission radiation and the time decay results of each of these modes of detection from the analyte deposition location arising from the excitation radiation, onto at least one optical element which directs the radiation along at least one detection path; (f) detecting the emission at least one location along the detection path; and (g) determining whether the detected radiation corresponds to a chemical or biological material of interest.

A first variation of the sixth aspect of the invention includes the substrate having sufficient size to collect at least one hundred (more preferably at least one thousand, and even more preferably at least ten thousand) distinct and separated analyte depositions. A second variation includes the substrate being structured. A third variation includes the substrate being unstructured. A fourth variation includes the substrate being configured to enable controlled access to specifically selected analyte deposition locations to allow future re-examination of previously deposited analyte samples. A fifth variation provides a history of the detected chemistry for each analyte deposition location that has received deposition of analyte and has been previously examined and wherein the medium for storing samples and storing history may be the same or different.

In a seventh aspect of the invention, a system for spectroscopic analysis, includes: (a) a substrate; (b) means for depositing a known volume of the liquid sample to a predefined location on the substrate; (c) means for allowing or causing at least a portion of the liquid to evaporate from the sample to provide an analyte sample for testing; (d) means for directing excitation radiation on to the analyte sample; (e) means for detecting resulting emission radiation coming from the analyte sample; and (f) means for analyzing emission radiation spectra to provide a determination concerning the presence of any biological or chemical analytes of interest.

In an eighth aspect of the invention, a system for spectroscopic analysis, includes: (a) a substrate; (b) means for depositing a defined volume of the liquid to a predefined location on the substrate where the defined volume is less than a total volume to be deposited; (c) means for operating the means for depositing multiple times to deposit the total volume on to the predefined location on the substrate while allowing time between depositing operations or after the total volume has been deposited to allow at least a portion of the liquid to evaporate to leave an analyte sample; (d) means for directing excitation radiation on to the analyte sample; (e) means for detecting resulting emission radiation coming from the analyte sample; and (f) means for analyzing emission radiation spectra to provide a determination concerning the presence of any biological or chemical analytes of interest.

In a ninth aspect of the invention, a system for spectroscopic analysis, includes: (a) a substrate; (b) means for depositing a known volume of the liquid sample to a predefined location on the substrate and allowing or causing different analytes to spatially separate after deposition to create a distributed analyte sample; (c) means for allowing or causing at least a portion of the liquid to evaporate from the sample to provide an analyte sample for testing; (d) means for directing excitation radiation on to the analyte sample; (e) means for detecting resulting emission radiation coming from different regions of the distributed analyte sample; and (f) means for analyzing emission radiation spectra from the different regions of the distributed analyte sample to provide an integrated determination concerning the presence of any biological or chemical analytes of interest.

In a tenth aspect of the invention, a system for spectroscopic analysis for a plurality of different liquid samples including creation of a multi-sample analyte storage device or disc, includes: (a) a substrate; (b) means for depositing a known volume of the liquid sample to a predefined location on a substrate; (c) means for allowing or causing at least a portion of the liquid to evaporate from the sample to provide an analyte sample for testing; (d) means for directing excitation radiation on to the analyte sample; (e) means for detecting resulting emission radiation coming from the analyte sample; (f) means for analyzing emission radiation spectra to provide a determination concerning the presence of any biological or chemical analytes of interest; and (g) means for controlling (b) - (f) for depositing, allowing or causing, directing, detecting, and analyzing for a plurality of additional liquid samples wherein the depositing of each liquid sample will occur at a different defined location on the substrate relative to previously dispensed droplets and wherein the substrate with its plurality of depositions will form a permanent chemical and biological storage device or disc (CBD) that may be accessed in the future for providing updated analysis.

In an eleventh aspect of the invention, a system for spectroscopic for a plurality of different liquid samples taken from at least one source including creation of a multi-sample analyte storage device, includes: (a) a substrate; (b) means for depositing a known volume of the liquid sample to a predefined location on a substrate, wherein a volume of the liquid sample is selected from the group of: (1) a volume equal to a total volume to be deposited and (2) a volume less than the total volume to be deposited and wherein positions of different analytes in the deposited volume is selected from a group of: (1) substantially overlapping positions or symmetric positioning driven primarily by deposition and drying and (2) nonsymmetric, spatially offset positions driven by a non-symmetric motive force in combination with deposition and drying; (c) means for repeatedly operating the means for depositing of element (b) as necessary until the total volume is deposited while allowing time between depositing operations or after the total volume is deposited to allow or cause at least a portion of the liquid to evaporate to provide an analyte sample; (d) means for directing excitation radiation on to the analyte sample; (e) means for detecting resulting emission radiation coming from the analyte sample as a whole or from multiple regions of the sample; (f) means for analyzing emission radiation spectra to provide a determination concerning the presence of any biological or chemical analytes of interest; (g) means for operating elements (c) - (g) for a plurality of additional liquid samples wherein the depositing of each liquid sample occurs at a different defined location on the substrate relative to previously dispensed droplets and wherein the substrate with its plurality of depositions will form a permanent chemical and biological storage device or disc (CBD) that may be accessed in the future for providing updated analysis.

In a twelfth aspect of the invention, a system for sensing trace and ultra-trace concentrations of chemical and biological materials including: (a) an addressable substrate capable of receiving a plurality of spatially separated deposits of liquids from samples to be tested; (b) means for depositing a first sub-microliter (e.g. 5-50 nL) volume of liquid that may contain one or more analytes of interest on a selected deposition location on the substrate; (c) means for sequentially depositing a plurality of additional volumes (e.g. droplets) vertically upon previously dispensed deposits, wherein a drying time between dispensing successive droplets allows for at least partial evaporation of the liquid so that liquid at the dispensing location does continue to increase in volume with each successive droplet, and wherein the overlaying of depositions allows for thickening of the analyte deposition wherein a diameter of analyte deposition is between 50 µm and 500 µm; (d) means for providing excitation radiation onto the thickened analyte material deposition wherein the excitation radiation has a wavelength in the deep ultraviolet, near ultraviolet, visible, or infrared; (e) means for detecting at least one of Rayleigh, Raman, resonance Raman, surface plasmon enhanced Raman, native fluorescence, or phosphorescence emission radiation and their subsequent time decay rates arising from the analyte deposition location at least one location along a detection path; and (f) means for determining whether the detected radiation corresponds to a chemical or biological material of interest.

In a thirteenth aspect of the invention, spectroscopic analysis apparatus, includes:(a) a substrate; (b) a computer-controlled dispenser for depositing a known volume of the liquid sample to a predefined location on the substrate; (c) an evaporation promoter selected from the group of at least one of : (1) a substrate heater, (2) an air heater, (3) an air chiller, (4) a desiccant, (5) at least one air filter, (6) an exhaust fan, and (7) an intake fan, and (8) a wait time for promoting evaporation of the liquid from the deposited liquid sample to provide an analyte sample for testing; (d) a source of excitation radiation controlled to direct the excitation radiation at the predefined location on the substrate when the analyte sample is present; (e) at least on optical component and a detector for capturing emission radiation in a plurality of selected spectral bands arising from an interaction between the excitation radiation and the analyte sample; and (f) a computer programmed to compare detected emission radiation with spectral emission data from samples of known materials of interest to determine presence of and concentration of such known materials in the analyte sample.

In a fourteenth aspect of the invention, spectroscopic analysis apparatus, includes:(a) a substrate; (b) a computer-controlled dispenser configured for depositing a plurality of overlaid known volume droplets to obtain a total dispensed volume wherein a time between successive dispensing is controlled; (c) an evaporation promoter selected from the group of at least one of: (1) a substrate heater, (2) an air heater, (3) an air chiller, (4) a desiccant, (5) at least one air filter, (6) an exhaust fan, and (7) an intake fan, and (8) a controlled wait time for promoting evaporation of the deposited liquid to provide an analyte sample for testing; (d) a source of excitation radiation controlled to direct the excitation radiation at the predefined location on the substrate when the analyte sample is present; (e) at least on optical component and a detector for capturing emission radiation in a plurality of selected spectral bands arising from an interaction between the excitation radiation and the analyte sample; and (f) a computer programmed to compare detected emission radiation with spectral emission data from samples of known materials of interest to determine presence of and concentration of such known materials in the analyte sample.

In a fifteenth aspect of the invention, spectroscopic analysis apparatus, includes: (a) a substrate including an analyte separation promoter; (b) a computer-controlled dispenser for depositing a known volume of the liquid sample to a predefined location on the substrate; (c) an evaporation promoter selected from the group of at least one of: (1) a substrate heater, (2) an air heater, (3) an air chiller, (4) a desiccant, (5) at least one air filter, (6) an exhaust fan, and (7) an intake fan, and (8) a wait time for promoting evaporation of the liquid from the deposited liquid sample to provide an analyte sample for testing, wherein the evaporation promoter and the analyte separation promoter are configured to operate in conjunction to provide an analyte sample with reduced or eliminated liquid and with a desired level of separation of different analytes; (d) a source of excitation radiation controlled to direct the excitation radiation at the predefined location on the substrate when the analyte sample is present; (e) at least on optical component and a detector for capturing emission radiation in a plurality of selected spectral bands arising from an interaction between the excitation radiation and the analyte sample; and (f) a computer programmed to compare detected emission radiation with spectral emission data from samples of known materials of interest to determine presence of and concentration of such known materials in the analyte sample.

In a sixteenth aspect of the invention, spectroscopic analysis apparatus, includes: (a) a substrate capable of permanently holding a plurality of spaced deposited analyte samples; (b) at least one reservoir for holding at least one liquid sample to be tested; (c) a computer-controlled dispenser capable of receiving at least a portion of the liquid sample from the reservoir; (c) at least one computer- controlled stage for relatively positioning the substrate and the dispenser for aligning the dispenser with a predefined substrate location and thereafter depositing a known volume of the liquid sample to the predefined location; (d) an evaporation promoter selected from the group of at least one of: (1) a substrate heater, (2) an air heater, (3) an air chiller, (4) a desiccant, (5) at least one air filter, (6) an exhaust fan, and (7) an intake fan, and (8) a wait time for promoting evaporation of the liquid from the deposited liquid sample to provide an analyte sample for testing; (d) a source of excitation radiation controlled to direct the excitation radiation at the predefined location on the substrate when the analyte sample is present; (e) at least one optical component and at least one detector for capturing emission radiation in a plurality of selected spectral bands arising from an interaction between the excitation radiation and the analyte sample; and (f) a computer programmed to compare detected emission radiation with spectral emission data from samples of known materials of interest to determine presence of and concentration of such known materials in the analyte sample; (g) a drain for receiving excess liquid sample material from the dispenser after completing creation of the analyte sample; and (h) a computer control system for repeatedly loading the at least one dispenser with fresh sample liquid, for defining new predefined locations on the substrate for receiving at least deposition of fresh liquid samples, for operating the evaporation promoter, and for holding fresh analyte samples formed from the fresh liquid samples, and for operating the source of excitation radiation, the at least one detector, and the programmed computer to provide presence and concentration information for the analytes presence in each fresh analyte sample.

In a seventeenth apparatus aspect of the invention, an apparatus for sensing trace and ultra-trace concentrations of chemical and biological materials includes: (a) an addressable substrate capable of receiving a plurality of spatially separated deposits of liquids from samples to be tested; (b) a dispenser (e.g., a digital micro syringe or pipette with a dispensing needle) for depositing a first sub-microliter (e.g. 5-50 nL) volume of liquid that may contain one or more analytes of interest on a selected deposition location on the substrate; (c) a programmed controller issuing a series of dispending commands to the dispenser (e.g. a programmed microcontroller that sends repeated commands to a plunger of the dispenser to push out commanded volumes of material at selected times or upon input from selected sensors and as necessary repeated commands to move the dispenser vertically and/or horizontally to aid in the dispensing of droplets) to deposit a plurality of volumes (e.g. droplets) vertically upon previously dispensed deposits, wherein a drying time between dispensing successive droplets allows for at least partial evaporation of the liquid so that liquid at the dispensing location does continue to increase in volume with each successive droplet, and wherein the overlaying of depositions allows for thickening of the analyte deposition in the region of deposition wherein a diameter of analyte deposition is between 50 µm and 500 µm; (d) a source of excitation radiation that can be supplied onto the thickened analyte material deposition wherein the excitation radiation has a wavelength in the deep ultraviolet, near ultraviolet, visible, or infrared; (e) at least one optical element for receiving at least one of Rayleigh, Raman, resonance Raman, native fluorescence, or photoluminescence emission radiation from the analyte deposition location arising from the excitation radiation and for directing the emission radiation along at least one detection path; (f) at least one detector for detecting the emission radiation at at least one location along the detection path; and (g) a programmed computer for determining whether the detected radiation corresponds to a chemical or biological material of interest which includes comparing spectrum signatures of known materials to the detected emission radiation.

A first variation of the sixth apparatus aspect of the invention includes the substrate having sufficient size to collect at least one hundred (more preferably at least one thousand, and even more preferably at least ten thousand) distinct and separated analyte deposition areas. A second variation includes the substrate being structured. A third variation includes the substrate being unstructured. A fourth variation includes the substrate being configured to enable controlled access to specifically selected analyte deposition locations to allow future re-examination of previously deposited analyte samples. A fifth variation includes a memory for holding a history of the detected chemistry for each analyte deposition location that has received deposited analyte material and has been previously examined.

In an eighteenth aspect of the invention, a method of spectroscopic analysis, includes: (a) obtaining a liquid sample to be tested from at least one source; (b) providing a substrate; (c) depositing a total volume of the liquid sample to a predefined location on the substrate; (d) allowing or causing at least a portion of the liquid to evaporate from the sample to provide an analyte sample for testing; (e) directing excitation radiation on to the analyte sample; (f) detecting resulting emission radiation coming from the analyte sample; and (g) analyzing emission radiation spectra to provide a determination concerning the presence of any biological or chemical analyte of interest.

Numerous variations of the eighteenth aspect of the invention exist and include, for example: (1) the depositing of (c) including depositing a defined volume of the liquid to a predefined location on the substrate where the defined volume is less than the total volume to be deposited and thereafter repeating the depositing operation one or more times to deposit the total volume on to the predefined location on the substrate, and wherein the allowing or causing of (d) occurs between depositing operations or after the total volume has been deposited; (2) the depositing of (c) including (i) making one or more deposits, and (ii) allowing or causing different analytes to spatially separate after deposition to create a distributed analyte sample, wherein the detecting of (f) includes detecting resulting emission radiation coming from different regions of the distributed analyte sample, and wherein the analyzing of (g) includes analyzing emission radiation spectra from the different regions of the distributed analyte sample to provide an integrated determination concerning the presence of any biological or chemical analytes of interest; and (3) repeating the depositing of (c) and the allowing or causing of (d) to form a plurality of additional liquid samples wherein the depositing of each liquid sample will occur at a different defined location on the substrate relative to previously formed analyte samples and wherein the substrate with its plurality of analyte samples will form a permanent chemical and biological storage device or disc (CBD) that may be accessed in the future for providing updated analysis;

Numerous additional variations of the eighteenth aspect of the invention exist and include, for example: (4) the depositing the total volume of (c) includes depositing a known volume of the liquid sample to a predefined location on a substrate, wherein the known volume of the liquid sample is selected from the group of: (A) a volume equal to a total volume to be deposited, and (B) a volume less than the total volume to be deposited and repeating the deposition as necessary until the total volume is deposited, wherein the allowing or causing of (d) includes allowing time between depositing operations or after the total volume is deposited, wherein different analytes in the deposited volume have positionings selected from a group of: (A) substantially overlapping positions or symmetric positioning driven primarily by deposition and drying and (B) spatially offset positions driven by a separating motive force in combination with deposition and drying, wherein the detecting of (f) includes detecting resulting emission radiation coming from the analyte sample as a whole or separately from multiple regions of the sample; and wherein the method further includes repeating at least operations of the depositing of (c) and allowing of (d) for a plurality of additional liquid samples wherein the depositing of each liquid sample occurs at a different defined location on the substrate relative to previously dispensed analyte sample locations and wherein the substrate with its plurality of analyte samples form a permanent chemical and biological storage device or disc (CBD); and (5) the substrate is capable of receiving a plurality of spatially separated deposits of liquids from samples to be tested, wherein the depositing of the total volume of (c) occurs via deposition of a plurality of sub-microliter volumes which are overlaid on locations of previously deposited volumes, wherein the allowing or causing of (d) includes providing a drying time between dispensing successive droplets to allow for at least partial evaporation of the liquid so that liquid at the dispensing location does not continue to increase in volume with each successive overlaid volume, wherein the overlaid volumes provide thickening of the analyte deposition wherein a diameter of analyte deposition is between 50 µm and 500 µm, wherein the excitation radiation of (e) includes radiation selected from the group of deep ultraviolet, near ultraviolet, visible, and infrared, and wherein the detected emission radiation of (f) includes radiation selected from the group of Rayleigh, Raman, resonance Raman, native fluorescence, and photoluminescence emission radiation.

Still numerous additional variations of the eighteenth aspect of the invention exist and include, for example: (6) any of the aspect itself or its first through fourth variations wherein the determination includes a quantification of any biological or chemical analytes of interest; (7) any of the aspect itself, its first through fourth or sixth variations wherein (g) provides a preliminary determination and wherein the method additionally includes repeating operations at least once and analyzing the emission radiation spectra from the repeated operations to provide a refined determination concerning the presence and quantification of any biological or chemical analytes of interest, wherein the repeated operations include operations selected from the group of (A) at least (e) and (f); (B) at least (d), (e), and (f); and (C) at least (c), (d), (e), and (f); (8) seventh variation of the aspect wherein at least one repeating of (f) includes detection of a different type of emission radiation than used during the initial operation of (f), and wherein at least one repeating of (e) includes use of a different wavelength range of excitation radiation than was used during the initial operation of (e); (9) any of the aspect itself, its first through fourth, or sixth through eighth variations wherein the excitation radiation includes radiation selected from the group of: (A) between 200 nm and 12 microns, (B) between 1046 nm and 200 nm, (C) less than 400 nm, (D) less than 300 nm, (E) less than 275 nm, and (F) less than 250 nm; and wherein the detecting of emission radiation includes detection of radiation selected from the group of: (A) Raman emission, (B) resonance Raman emission, (C) surface plasmon resonance enhancement Raman emission, (D) native fluorescence emission, (E) Rayleigh emission, and (F) phosphorescence emission (10) any of the aspect itself, its first through fourth, or sixth through ninth variations wherein substantially complete evaporation of the liquid from the sample has occurred at a time of detecting; (11) any of the aspect itself, its first through fourth, or sixth through ninth variations wherein at a time of detecting, the amount of liquid remaining in the analyte sample (as compared to the original amount of liquid present) being tested is selected from the group of: (A) less than 50%, (B) less than 20%, (C) less than 10%, (D) less than 5%, (E) less than 2%, (F) less than 1%, (G) less than 0.5 percent, (H) less than 1%, and (I) less than 0.1%; (12) any of the aspect itself, its second through fourth, or sixth through eleventh variations wherein the total volume is deposited in a single deposition; and (13) any of the aspect itself, its second through fourth, or sixth through eleventh variations wherein the deposited volume is created from a plurality of separate depositions wherein at least one deposition has a volume selected from the group of: (A) less than 1 nL, (B) less than 2 nL, (C) less than 5 nL, (D) less than 10 nL, (E) less than 20 nL, (F) less than 50 nL, (F) less than 100 nL, (H) less than 200 nL, (I9) less than 500 nL, (J) less than 1 uL, and (K) less than 2 uL.

Still further variations of the eighteenth aspect of the invention exist and include, for example: (14) any of the aspect itself, its second through fourth, sixth through eleventh, or thirteenth variations wherein the deposited volume is created from a plurality of separate depositions and wherein a number of the plurality of depositions is selected from the group of: (A) at least 2 depositions, (B) at least 4 depositions, (C) at least 8 depositions, (D) at least 16 depositions, (E) at least 32 depositions, (F) at least 64 depositions, (G) at least 128 depositions, and (H) at least 256 depositions; (15) either of the thirteenth or fourteenth variations wherein successive depositions have targeted deposition locations that are selected from the group of (A) more than 90% of subsequent droplets have deposition areas that overlay the combination of areas occupied by prior depositions by an amount selected from the group of: (i) more than 50%, (ii) more than 70%, (iii) more than 90%, (iv) more than 95%, and (v) more than 99%, and (B) more than 90% of subsequent droplets have central droplet locations selected from the group of (i) within 8 um of the average center location for previously dispensed droplets, (ii) within 4 um of the average center location for previously dispensed droplets, (iii) within 2 um of the average center location for previously dispensed droplets, and (iv) within 1 um of the average center location for previously dispensed droplets; (16) any of the thirteenth through fifteenth variations of the aspect wherein at least one successive deposition has a substantially smaller volume than that of a previous deposition, where the smaller volume is selected from the group of: (A) no more than 70% of that of the previous deposition, (B) no more than 50% of that of the previous deposition, (C) no more than 30% of that of the previous deposition; (17) any of the aspect itself, its second through fourth or sixth through sixteenth variations wherein the substrate has a property selected from the group of: (A) a smooth surface, (B) depressions defining potential droplet locations, (C) regions where analyte depositions are to exist have higher hydrophilicity than surrounding than surrounding peripheral regions, (D) regions where analyte depositions are to exist have lower hydrophobicity than surrounding peripheral regions, (E) no production of interfering background emissions during exposure so excitation radiation, (F) regions where analyte deposition are intended have chromatographic surfaces, and (G) structured plasmonic features; (18) any of the aspect itself, its second through fourth, sixth through seventeenth variations wherein the liquid includes an aqueous solution; (19) any of the aspect itself, its second through fourth, sixth through eighteenth variations wherein the analyte sample includes a plurality of analyte samples; (20) the nineteenth variation of the aspect wherein the plurality of analyte samples are extracted from a material undergoing a process and wherein the determination made for each analyte sample is used, at least in part, to make a decision selected from the group of: (A) leaving processing parameters and operations unchanged, (B) making a change to at least one processing parameter, (C) making a change to at least one processing operation, (D) making a determination as to when to perform a subsequent test, (E) making a determination concerning ending the process; (21) the third variation of the aspect wherein the spatial separation includes chromatographic separation; (22) any of the aspect itself, its second through fourth, or sixth through twenty first variations wherein the sample includes water extracted during a wastewater treatment operation; and (23) any of the aspect itself, its second through fourth, or sixth through twenty-second variations wherein liquid sample includes a plurality of samples which originate from a plurality of different sources.

In a nineteenth aspect of the invention, a system for spectroscopic analysis, includes:(a) a substrate; (b) means for depositing a total volume of a liquid sample to a predefined location on the substrate; (c) means for allowing or causing at least a portion of the liquid to evaporate from the liquid sample to provide an analyte sample for testing; (d) means for directing excitation radiation on to the analyte sample; (e) means for detecting resulting emission radiation coming from the analyte sample; and (f) means for analyzing emission radiation spectra to provide a determination concerning the presence of at least one biological or chemical analyte of interest.

Numerous variations of the nineteenth aspect of the invention exist and include, for example: (1) the means for depositing of (b) including means for depositing a defined volume of a liquid to a predefined location on the substrate where the defined volume is less than a total volume to be deposited and means for operating the means for depositing multiple times to deposit the total volume on to the predefined location on the substrate while allowing time between depositing operations or after the total volume has been deposited to allow at least a portion of the liquid to evaporate to leave an analyte sample; (2) the aspect additionally including means for allowing or causing different analytes to spatially separate after deposition to create a distributed analyte sample, wherein the means of depositing of (b) includes means for depositing a known volume of the liquid sample one or more times to deposit the total volume to a predefined location on the substrate, wherein the means for detecting of (e) includes means for detecting resulting emission radiation coming from different regions of the distributed analyte sample; and wherein the means for analyzing or (f) includes means for analyzing emission radiation spectra from the different regions of the distributed analyte sample to provide an integrated determination concerning the presence of any biological or chemical analytes of interest; (3) the aspect additionally including means for operating the means of depositing of (b) and the means for allowing or causing of (c) a plurality of times to create a plurality of additional analyte samples at different defined locations on the substrate relative to previously created analyte samples wherein the substrate with its plurality of depositions includes a permanent chemical and biological storage device or disc (CBD); (4) the means for depositing of (b) includes means for depositing a known volume of the liquid sample one or more times to deposit the total volume to a predefined location on the substrate wherein a position of different analytes in the deposited volume is selected from a group of: (A) substantially overlapping positions or symmetric positions driven primarily by deposition and drying, and (B) spatially offset positions driven by a non-symmetric motive force in combination with deposition and drying, and wherein the means for detecting of (e) includes means detecting emission radiation coming from the analyte sample as a whole or from multiple regions of the sample, and wherein the system additionally includes means for operating the means of (b) and (c) a plurality of times to create a plurality of additional analyte samples at different defined locations on the substrate relative to previously created analyte samples wherein the substrate with its plurality of depositions includes a permanent chemical and biological storage device or disc (CBD); and (5) the substrate of (a) includes being capable of receiving a plurality of spatially separated deposits of liquids from samples to be tested, wherein the means for depositing of (b) includes means for depositing a first sub-microliter volume of liquid and a means for sequentially depositing a plurality of at least partially overlaying additional volumes upon previously dispensed deposits, wherein the means for allowing or causing of (c) provides a drying time between successive deposits that allows for at least partial evaporation of the liquid so that liquid at the dispensing location does increase in volume with each successive deposition, and wherein the overlaying of depositions provides for thickening of the analyte deposition wherein a diameter of analyte deposition resulting from the deposition of the total volume is between 50 µm and 500 µm, wherein the means for directing excitation radiation of (d) provides radiation having a wavelength in the deep ultraviolet, near ultraviolet, visible, or infrared portions of the spectrum, and wherein the means for detecting detects at least one of Rayleigh, Raman, resonance Raman, native fluorescence, or photoluminescence emission radiation.

Numerous additional variations of the nineteenth aspect of the invention exist and include, for example: (6) any of the aspect itself or its first through fourth variations wherein the system additionally includes means for receiving a testable liquid which can be accessed by the means for dispensing and can thereafter be deposited by the means for dispensing; (7) any of the aspect itself, its first through fourth variations, or its sixth variation wherein the means for determining is further configured to quantify an amount of at least one detected biological or chemical analyte of interest; (8) any of the aspect itself, its first through fourth variations, or its sixth or seventh variations wherein the determination of the means of (f) provides a preliminary determination and the system is configured to repeat operations at least once to provide a refined determination concerning the presence and quantification of at least one biological or chemical analyte of interest wherein the means to undergo repeated operation includes means selected from the group of (A) at least (e) and (f); (B) at least (d), (e), and (f); and (C) at least (c), (d), (e), and (f); (9) the eighth variation wherein the system is configured such that at least one repeating of the operation of the means of (e) includes use of a different type of emission radiation than used during a previous operation of the means of (e) and at least one repeating of the operation of the means of (d) includes providing a different wavelength range of excitation radiation than was used during a previous operation of the means of (d); (10) any of the aspect itself, its first through fourth variations, or its sixth through ninth variations] wherein the means of (d) is configured to provide excitation radiation selected from the group of: (A) between 200 nm and 12 microns, (B) between 200 nm and 1046 nm, (C) less than 400 nm, (D) less than 300 nm, less than 275 nm, and (E) less than 250 nm, and wherein (e) is configured to detect emission radiation selected from the group of: (A) Raman emission, (B) resonance Raman, (C) surface plasmon resonance enhancement Raman, (D) fluorescence emission, (E) Rayleigh emission, (F) phosphorescence emission; (11) any of the aspect itself, its first through fourth variations, or its sixth through tenth variations wherein the system is configured such that substantially complete evaporation occurs prior to an operation of the means of (d) and (e); (12) any of the aspect itself, its first through fourth variations, or its sixth through eleventh variations wherein the system is configured to operate the means of (d), (e), and (f) when, the amount of liquid remaining in the analyte sample (as compared to the original amount of liquid present) being tested is selected from the group of: (A) less than 50%, (B) less than 20%, (C) less than 10%, (D) less than 5%, (E) less than 2%, (F) less than 1%, (G) less than 0.5 percent, (H) less than 1%, and (I) less than 0.1%; (13) any of the aspect itself, its first, third, fourth, or its sixth through twelfth variations wherein the system is configured to operate the means of (b) only once to provide the total volume from a single deposition; and (14) any of the aspect itself, its first variation, its third or fourth variations, or its sixth through twelfth variations wherein the system is configured to operate the means of (b) a plurality of times to provide a deposited volume that is created from a plurality of depositions, wherein at least one of the plurality of depositions is configured to have a volume selected from the group of: (A) less than 1 nL, (B) less than 2 nL, (C) less than 5 nL, (D) less than 10 nL, (E) less than 20 nL, (F) less than 50 nL, (G) less than 100 nL, (H) less than 200 nL, (I) less than 500 nL, (J) less than 1 uL, and (K) less than 2 uL.

Still additional variations of the nineteenth aspect of the invention exist and include, for example: (15) any of the aspect itself, its first variation, its third or fourth variations, or its sixth through fourteenth variations wherein the system is configured such that a plurality of depositions is used in depositing the total volume, wherein the plurality is selected from the group of: (A) at least 2 depositions, (B) at least 4 depositions, (C) at least 8 depositions, (D) at least 16 depositions, (E) at least 32 depositions, (F) at least 64 depositions, (G) at least 128 depositions, and (H) at least 256 depositions; (16) either of the fourteenth or fifteenth variations of the aspect wherein the system is configured to provide successive depositions having targeted deposition locations that are selected from the group of (A) more than 90% of subsequent droplets have deposition areas that overlay the combination of areas occupied by prior depositions by an amount selected from the group of: (i) more than 50%, (ii) more than 70%, (iii) more than 90%, (iv) more than 95%, and (v) more than 99%, and (B) more than 90% of subsequent droplets have central droplet locations selected from the group of (i) within 8 um of the average center location for previously dispensed droplets, (ii) within 4 um of the average center location for previously dispensed droplets, (iii) within 2 um of the average center location for previously dispensed droplets, (iv) within 1 um of the average center location for previously dispensed droplets; (17) any of the fourteenth through sixteenth variations of the aspect wherein the system is configured such that the at least one successive deposition has a substantially smaller volume than that of a selected previous deposition, where the smaller volume is selected from the group of: (A) no more than 70% of that of the previous deposition, (B) no more than 50% of that of the previous deposition, (C) no more than 30% of that of the previous deposition; (18) any of the aspect itself, its first through fourth variations, or its sixth to seventh variation wherein the substrate has a property selected from the group of: (A) a smooth surface, (B) depressions defining potential droplet locations, (C) regions where analyte depositions are to exist having higher hydrophilicity than the surrounding peripheral regions, (D) regions where analyte depositions are to exist have lower hydrophobicity than surrounding peripheral regions, (E) no production of interfering background emissions during exposure to excitation radiation, (F) at least some regions where analyte deposition are intended have chromatographic surfaces, and (G) structured plasmonic features; (19) any of the aspect itself, its first through fourth variations or its sixth through eighteenth variations wherein the liquid includes an aqueous solution; (20) any of the aspect itself, its first through fourth variations or its sixth through ninth variations wherein the analyte sample includes a plurality of analyte samples; (21) the twentieth variation wherein the analyte sample is extracted from a material undergoing a process and wherein the system is configured such that the determination made for the analyte sample is used, at least in part, to make a decision selected from the group of: (A) leaving processing parameters and operations unchanged, (B) making a change to at least one processing parameter, (C) making a change to at least one processing operation, (D) making a determination as to when to perform a subsequent test, (E) making a determination concerning ending the process; (22) the second variation of the aspect wherein the means for allowing or causing provides for chromatographic separation; (23) any of the aspect itself, its first through fourth variations or its sixth through twenty-second variations wherein the spectroscopic analysis system is incorporated into a wastewater treatment facility and wherein a sample to undergo spectroscopic analysis includes water extracted during a wastewater treatment operation; (24) any of the aspect itself, its first through fourth variations or its sixth through twenty-third variations wherein the system is configured such that the sample to be tested include a plurality of samples which originate from a plurality of different sources.

In a twentieth aspect of the invention, a spectroscopic analysis apparatus, includes:(a) a substrate; (b) a computer-controlled dispenser for depositing a total volume of a liquid sample to a predefined location on the substrate; (c) an evaporation promoter selected from the group of at least one of : (1) a substrate heater, (2) an air heater, (3) an air chiller, (4) a desiccant, (5) at least one air filter, (6) an exhaust fan, (7) an intake fan, and (8) a wait time for promoting evaporation of the liquid from the deposited liquid sample to provide an analyte sample for testing; (d) a source of excitation radiation controlled to direct the excitation radiation at the predefined location on the substrate when the analyte sample is present; (e) at least one optical component and a detector for capturing emission radiation in a plurality of selected spectral bands arising from an interaction between the excitation radiation and the analyte sample; and (f) a computer programmed to compare detected emission radiation with spectral emission data from samples of known materials of interest to determine presence of at least one material of interest in the analyte sample.

Numerous variations of the twentieth aspect of the invention exist and include, for example: (1)
the computer-controlled dispenser of (a) being configured for depositing a plurality of overlaid known volume droplets to obtain a total deposited volume wherein a time between dispensing of successive droplets is controlled, and wherein the evaporation promoter of (c) includes a promoter selected from the group of at least one of: (A) a substrate heater, (B) an air heater, (C) an air chiller, (D) a desiccant, (E) at least one air filter, (F) an exhaust fan, and (G) an intake fan, and (H) a controlled wait time for promoting evaporation of the deposited liquid to provide an analyte sample for testing; (2) the substrate of (a) includes at least one of an analyte separation promoter, and wherein the evaporation promoter and the analyte separation promoter are configured to operate in conjunction to provide an analyte sample with reduced or eliminated liquid and with a desired level of separation of different analytes; (3) the system additionally including (g) at least one reservoir for holding at least one liquid sample to be tested, (h) at least one computer-controlled stage for relatively positioning the substrate and the dispenser for aligning the dispenser with a predefined substrate location and thereafter depositing a known volume of the liquid sample to the predefined location using one or more deposition operations to deposit the total volume;(i) a drain for receiving excess liquid sample material from the dispenser after dispensing the total volume; and (j) a computer control system configured for loading sample liquid from the reservoir into the dispenser, for depositing the fresh liquid samples at predefined locations on the substrate using the dispenser, for operating the evaporation promoter, for operating the source of excitation radiation, and for operating the at least one detector, and wherein the substrate of (a) is capable of permanently holding a plurality of spaced apart, deposited analyte samples; (4) wherein the substrate of (a) includes a substrate capable of receiving a plurality of spatially separated deposits of liquids from samples to be tested, wherein the dispenser of (b) includes a digital micro syringe or pipette with a dispensing needle for depositing defined sub-microliter volumes of liquid, wherein the apparatus additionally includes a programmed controller for providing dispensing commands to the dispenser and for controlling the evaporation promoter to allow at least partial drying of deposited liquid between dispensing successive droplets so that liquid at the predefined location does continue to increase in volume with the dispensing of each successive droplet, wherein a diameter of analyte deposition is between 50 µm and 500 µm after dispensing the total volume, wherein the source of excitation radiation of (d) provides at least one of deep ultraviolet, near ultraviolet, visible, or infrared radiation, and wherein the emission radiation includes at least one of Rayleigh, Raman, resonance Raman, native fluorescence, or photoluminescence emission radiation wherein the at least one optical component aids in directing the emission radiation along an optical path from the analyte deposition location to the detector; and (5) the apparatus additionally including a liquid flow system that provides a liquid sample from a process chamber or a storage chamber to a reservoir that allows access to the liquid by the dispenser such that the dispenser can be loaded with the liquid and thereafter made to deposit the liquid onto the selected location of the substrate.

Numerous additional variations of the twentieth aspect of the invention exist and include, for example: (6) the twentieth aspect itself or any of its first to third or fifth variations wherein the computer is further programmed to quantify an amount of detected biological or chemical analytes of interest; &) the twentieth aspect itself or any of its first to third or fifth variations wherein the determination of (f) provides a preliminary determination concerning a material of interest and wherein the apparatus further includes a controller programmed to operate at least one of the detector and the computer multiple times to provide a refined determination concerning the presence and quantification of any biological or chemical analytes of interest; (8) the twentieth aspect itself or any of its first to third or fifth to seventh variations wherein the determination of (f) provides a preliminary determination concerning a material of interest and wherein the apparatus includes a controller programmed to operate at least the source, the detector, and the programmed computer multiple times to provide a refined determination concerning the presence and quantification of any biological or chemical analytes of interest; (9) the twentieth aspect itself or any of its first to third or fifth to seventh variations wherein the determination of (f) provides a preliminary determination concerning a material of interest and wherein the apparatus includes a controller that is programmed to operate at least the dispenser, the source, the detector, and the programmed computer multiple times to provide a refined determination concerning the presence and quantification of any biological or chemical analytes of interest; (10) the twentieth aspect itself or any of its first to third or fifth to seventh variations wherein the apparatus is configured such that a repeating of operation of the detector at least once includes detection of a different type of emission radiation than used during a previous operation of the detector; (11) the twentieth aspect itself or any of its first to third or fifth to seventh variations wherein the apparatus is configured such that a repeating of the operation of the source and the detector includes the source providing a different wavelength range of excitation radiation than was used during a previous operation of the source and the detector; (12) the source providing excitation radiation selected from the group of: (A) between 200 nm and 12 microns, (B) between 1046 nm and 200 nm, (C) less than 400 nm, (D) less than 300 nm, less than 275 nm, and (E) less than 250 nm, and wherein the at least one optical element and the detector are configured to detect emission radiation selected from the group of: (A) Raman emission, (B) resonance Raman, (C) surface plasmon resonance enhancement Raman, (D) fluorescence emission, (E) Rayleigh emission, and (F) phosphorescence emission; (13) the twentieth aspect itself or any of its first to third or fifth to twelfth variations wherein the apparatus is configured such that substantially complete evaporation occurs prior to an operation of the source of (d) and the detector of (e); (14) the twentieth aspect itself or any of its first to third or fifth to thirteenth variations wherein the apparatus is configured to operate the source, the detector, and the programmed computer when the amount of liquid remaining in the analyte sample (as compared to the original amount of liquid present) being tested is selected from the group of: (A) less than 50%, (B) less than 20%, (C) less than 10%, (D) less than 5%, (E) less than 2%, (F) less than 1%, (G) less than 0.5 percent, (H) less than 1%, and (9) less than 0.1%; (15) the twentieth aspect itself or either of its second or fifth variations wherein the apparatus is configured to operate the dispenser only once to provide a deposited total volume; and (16) the twentieth aspect itself or any of its first to third or fifth to fourteenth variations wherein the apparatus is configured to operate the dispenser a plurality of times to provide a deposited total volume that is created from a plurality of smaller depositions, wherein at least one of the plurality of depositions is configured to have a volume selected from the group of: (A) less than 1 nL, (B) less than 2 nL, (C) less than 5 nL, (D) less than 10 nL, (E) less than 20 nL, (F) less than 50 nL, (G) less than 100 nL, (H) less than 200 nL, (I) less than 500 nL, (J) less than 1 uL, and (K) less than 2 uL.

Further additional variations of the twentieth aspect of the invention exist and include, for example: (17) the twentieth aspect itself or any of its first to third or fifth to fourteenth, or sixteenth variations wherein the apparatus is configured such that a plurality of depositions is used in depositing the total volume, wherein the plurality is selected from the group of: (A) at least 2 depositions, (B) at least 4 depositions, (C) at least 8 depositions, (D) at least 16 depositions, (E) at least 32 depositions, (F) at least 64 depositions, (G) at least 128 depositions, and (H) at least 256 depositions; (18) the sixteenth or seventeenth variation wherein the apparatus is configured to provide successive depositions having targeted deposition locations that are selected from the group of (A) more than 90% of subsequent depositions have deposition areas that overlay the combination of areas occupied by prior depositions by an amount selected from the group of: (i) more than 50%, (ii) more than 70%, (iii) more than 90%, (iv) more than 95%, and (v) more than 99%, and (B) more than 90% of subsequent droplets have central droplet locations selected from the group of (i) within 8 um of the average center location for previously dispensed depositions, (ii) within 4 um of the average center location for previously dispensed droplets, (iii) within 2 um of the average center location for previously dispensed droplets, (iv) within1 um of the average center location for previously dispensed droplets; (19) the sixteenth through eighteenth variation wherein the apparatus is configured such that at least one successive deposition has a substantially smaller volume than that of a selected previous deposition, where the smaller volume is selected from the group of: (A) no more than 70% of that of the previous deposition, (B) no more than 50% of that of the previous deposition, (C) no more than 30% of that of the previous deposition; (20) the twentieth aspect itself or any of its first to third or fifth to nineteenth variations wherein the substrate has a property selected from the group of: (A) a smooth surface, (B) depressions defining potential droplet locations, (C) regions where analyte depositions are to exist having higher hydrophilicity than the surrounding peripheral regions, (D) regions where analyte depositions are to exist have lower hydrophobicity than surrounding peripheral regions, (E) no production of interfering background emissions during exposure to excitation radiation, (F) at least some regions where analyte deposition are intended have chromatographic surfaces, and (G) structured plasmonic features; (21) the twentieth aspect itself or any of its first to third or fifth to twentieth variations wherein the liquid includes an aqueous solution; (22) the twentieth aspect itself or any of its first to third or fifth to twenty-first variations wherein the analyte sample includes a plurality of analyte samples; (23) the twenty-second variation of the twentieth aspect, wherein the plurality of analyte samples are extracted from a material undergoing a process and wherein the apparatus is configured such that the determination made for the analyte sample is used, at least in part, to make a decision selected from the group of: (A) leaving processing parameters and operations unchanged, (B) making a change to at least one processing parameter, (C) making a change to at least one processing operation, (D) making a determination as to when to perform a subsequent test, (E) making a determination concerning ending the process; (24) the second variation of the twentieth aspect wherein the substrate provides for chromatographic separation; (25) the twentieth aspect itself or any of its first to twenty-fourth variations wherein the spectroscopic analysis apparatus is incorporated into a wastewater treatment facility and wherein a sample to undergo spectroscopic analysis includes water extracted during a wastewater treatment stage; and (26) the twentieth aspect itself or any of its first to twenty-fifth variations wherein the apparatus is configured such that the sample to be tested includes a plurality of samples which originate from a plurality of different sources.

### Brief Description of the Drawings

FIG. 1A provides a block diagram illustrating a detection and analysis method embodiment that uses at least partial removal of the water media of an aqueous solution that is to be tested for the presence of any analytes whereby the removal of media reduces interfering emission signals from the media to ultimately improve the LOD of a detection and analysis process for analytes of interest.
FIG. 1B provides a graph illustrating the Raman spectrum of water with potassium nitrate contamination where the spectral background from the water masks portions the Raman signature of the potassium nitrate at a concentration of 100 parts per million and makes a portion of it completely unreadable at a concentration of 10 parts per million thus making concentrations in the 1 ppm, 100 ppb, and 10 part per billion unreadable (i.e., the concentrations are smaller than the effective limits of detection).
FIG. 1C provides a plot of normalized Raman intensity versus wavenumber illustrating the intensity improvement that can be achieved when the water background is sufficiently reduced for two example analytes (sodium nitrate and potassium dihydrogen phosphate).

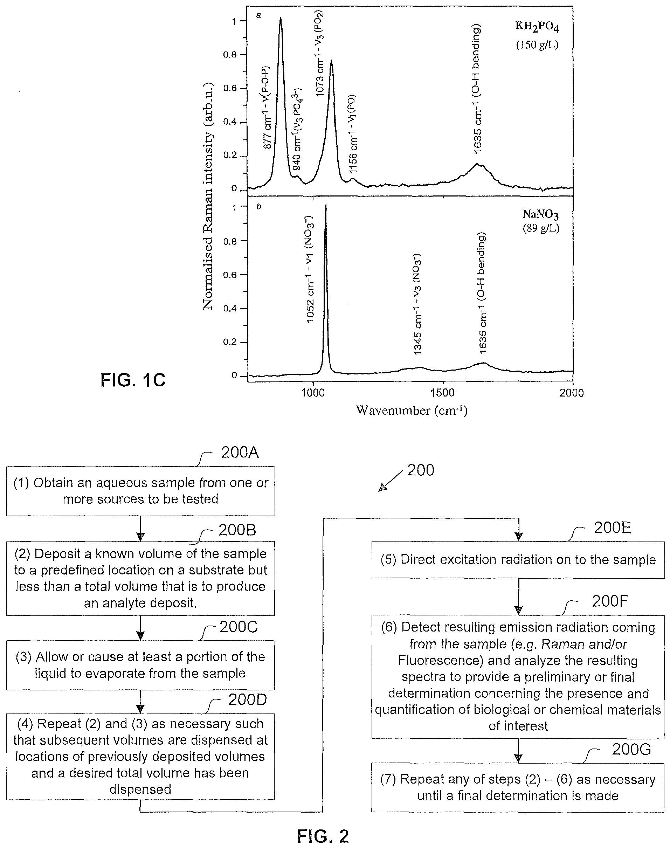

FIG. 2 provides a block diagram illustrating a detection and analysis method embodiment using repeated overlaying depositions of small volumes of an aqueous solution so as to provide smaller deposition areas for individual droplets and faster drying times or evaporation rates thus allowing rapid buildup of higher concentrations of analytes on the substrate surface with substantial removal, if not complete removal, of the water media prior to overlaying droplets thus providing smaller overall analyte deposition areas, higher analytes emission signals due to larger amounts of analytes, and reduced interference media emission signals thus providing an improved LOD with a short processing time.
FIG. 3 provides a block diagram illustrating a detection and analysis method embodiment using a physical analyte separation method to reduce any interference that might exist when a resulting deposit has multiple analytes to ultimately provide improve detection limits.
FIG. 4 provides a block diagram illustrating a detection and analysis method embodiment that uses a deposition substrate in the form of a storage structure or disc on to which a plurality of different test sample depositions can be individually located for permanent storage and subsequent re-evaluation.
FIG. 5 provides a block diagram illustrating a detection and analysis method providing for partial or complete media removal, for formation of a final analyte deposit from a single deposition or multiple depositions, for allowing the final deposit to retain its deposition configuration or to undergo physical separation of analytes and using a multi-sample CBD substrate to provide a physical record of analyte presence over time.
FIG. 6 provides a flowchart of another spectroscopy detection and analysis method for trace chemical or biological material detection from a liquid sample including some anomaly recognition and potential processing alternatives.

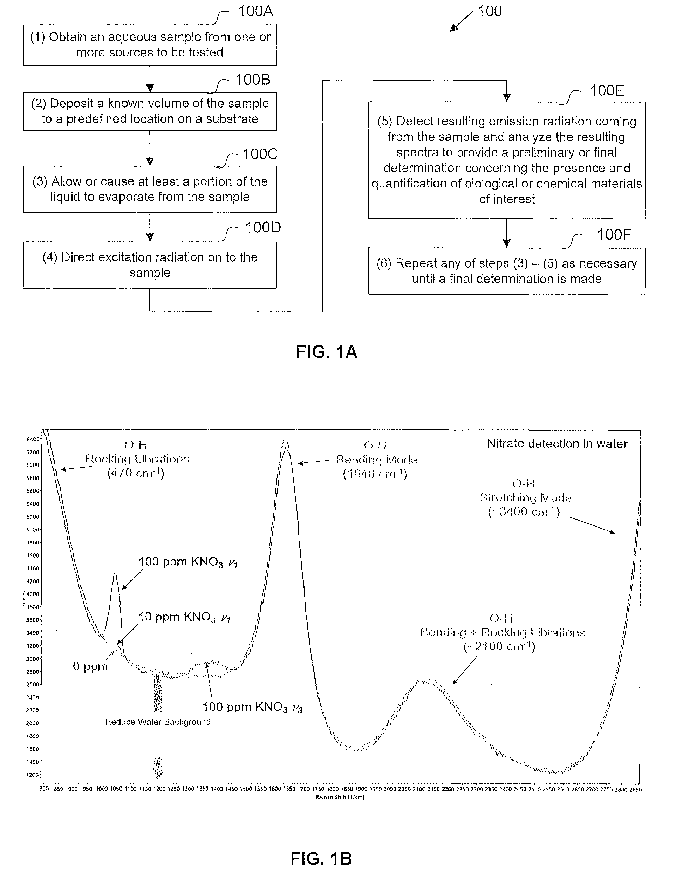

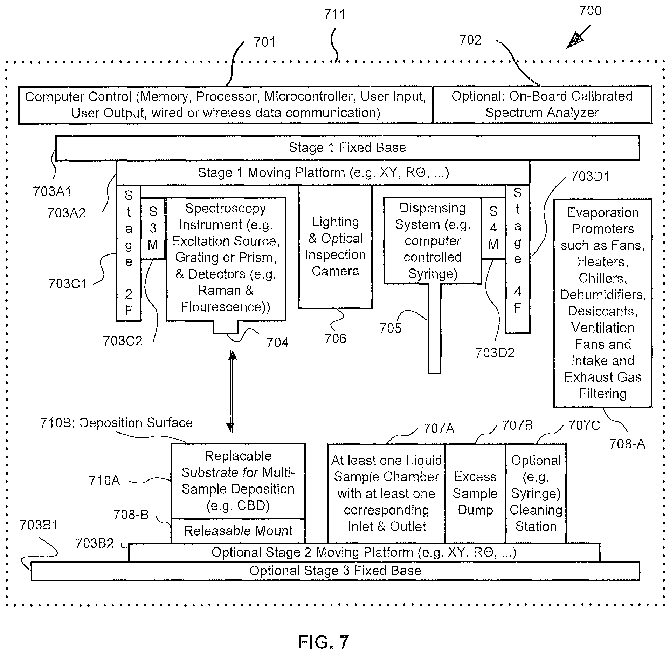

FIG. 7 provides a schematic illustration of a system (e.g. a system in a box), with various necessary and optional components, for sequentially capturing one or more liquid samples from different aqueous or other liquid sources into the dispensing system (e.g. including a syringe, or other controlled dispensing device), depositing analyte containing droplets (e.g., nanoscale droplets), removing at least part of the liquid carrier, and then providing spectroscopic analysis of the remaining analyte material to determine presence of selected or general chemical and biological content along with providing a quantification of that content, wherein the system may be programmed, or otherwise configured, to implement one of the methods set forth in FIGS. 1A or 2 - 6, a combination of such methods, or some other method.
FIG. 8 provides a schematic representation of a spectroscopic instrument that may be used in some embodiments of the system of FIG.7.
FIG. 9A provides a listing of example deposition devices that may be used in the system of FIG. 7.
FIG. 9B provides a listing of example deposition methods that may be implemented in different embodiments of the system of FIG. 7.
FIG. 9C provides a listing of example detailed deposition procedures that may be implemented in some variations of the system of FIG. 7
FIGS. 9D-1 and 9D-2 to 9G-1 to 9G-2 provide both block diagrams and schematic representations of four example processes for controllably dispensing known volumes of material in the form of individual droplets from computer-controlled syringes.

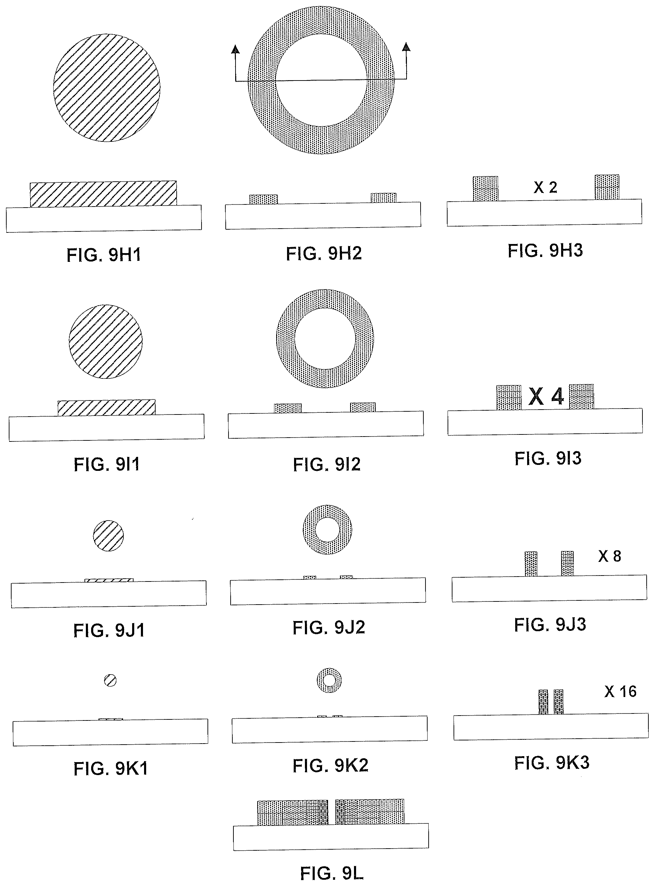

FIGS. 9H1 - 9K3 illustrate four hypothetical example droplets of different sizes (FIGS. 9H1, 9I1, 9J1, and 9K1) and resulting analyte deposits that might occur from single deposit (FIGS. 9H2, 9I2, 9J2, and 9K2) and multiple droplets (FIGS. 9H3, 9I3, 9J3, and 9K3), respectively, while FIG. 9L illustrates a net deposit which might occur from multiple overlaid or adjacent deposits including from deposit combinations that result from different sized droplets (e.g., from a single FIG. 9H1 droplet, four FIG. 9I1 droplets, eight FIG. 9J1 droplets, and sixteen FIG. 9K1 droplets).

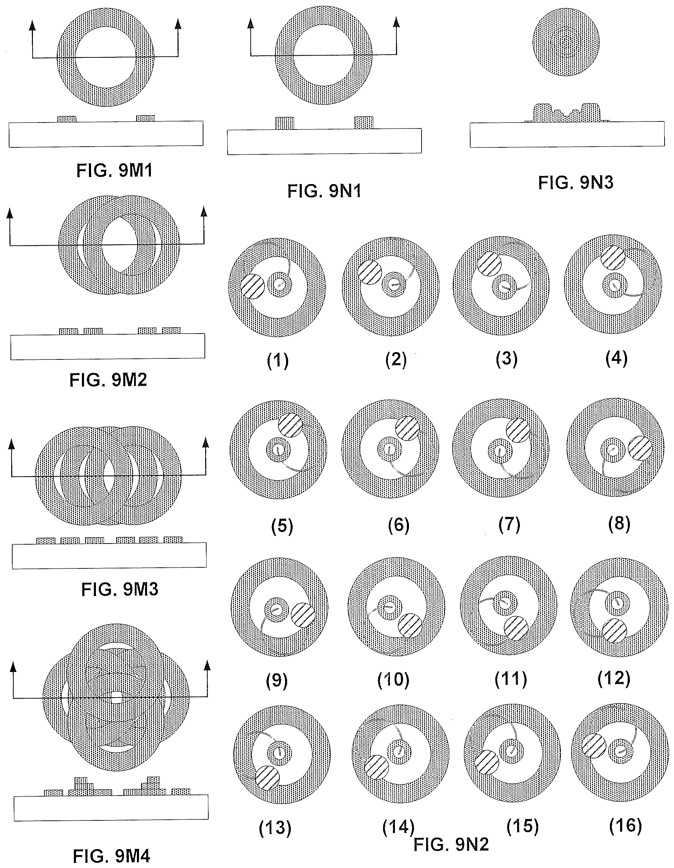

FIGS. 9M1 - 9M4 provide hypothetical top views and cut views of one or more droplets that are dispensed at slightly offset positions (for FIGS. 9M2 to 9M4) to illustrate another example of analyte stacking.
FIGS. 9N1 - 9N3 provides another example of analyte stacking but also provides an example of how lateral analyte concentration may be made to occur.

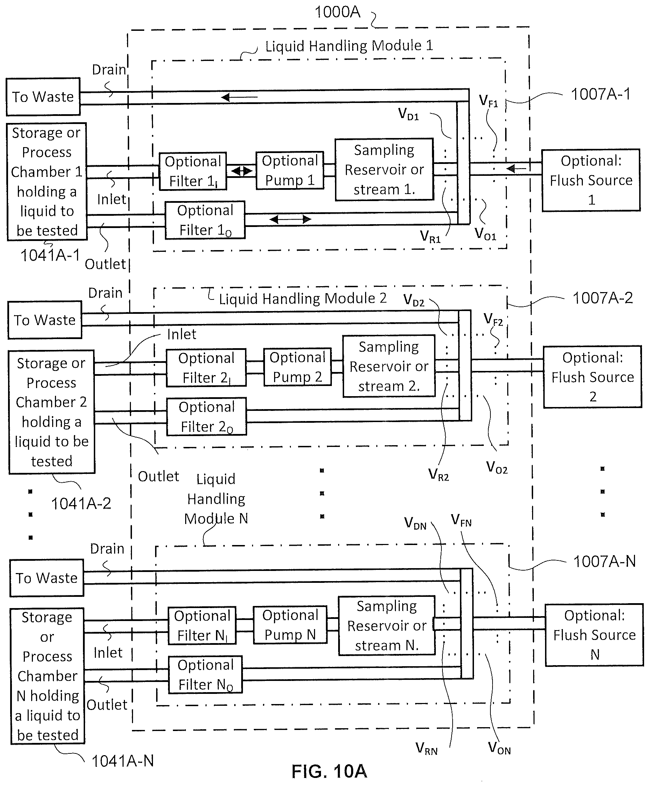

FIG. 10A provides a schematic illustration of a multi-source modular liquid handling system that can be used in the system of FIG. 7 that can supply 1 to N liquids independently to 1 to N reservoirs which can be accessed by the dispensing system wherein the modular system includes valves for controlling liquid flow, a flushing system, an inlet, and multiple outlets for each module.

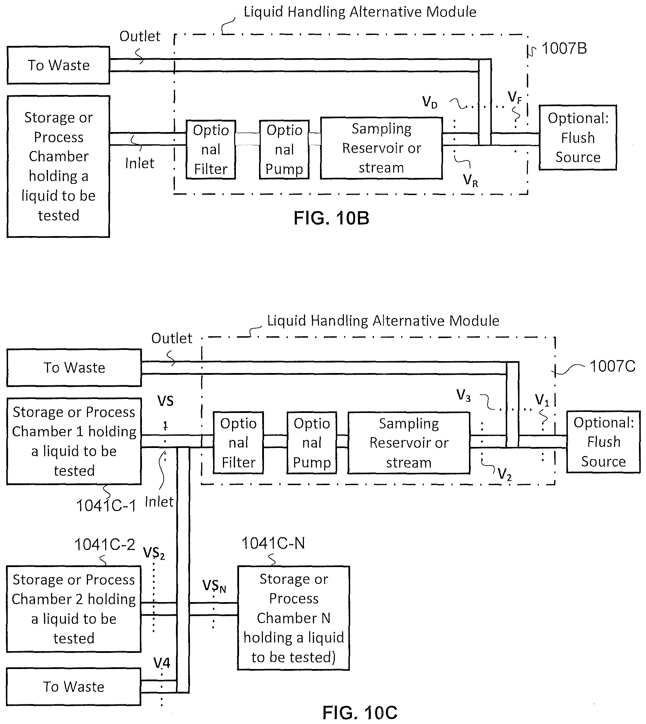

FIG. 10B provides a simplified module with only a single outlet that can be used to replace one or more of the modules of FIG. 10A.
FIG. 10C illustrates another example of a multi-source liquid handling system where the different sources share a common reservoir.

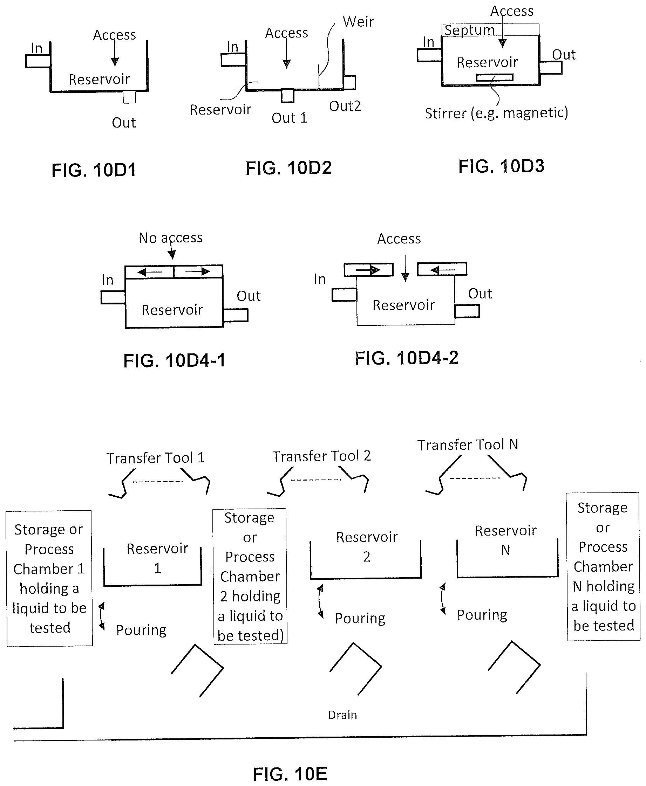

FIGS. 10D1 - 10D4-2 illustrate five different example reservoirs that might be used in the embodiments of FIGS. 10A - 10C.
FIG. 10E provides a different type of reservoir and fluid transfer system that is also capable of handling liquid from multiple sources.

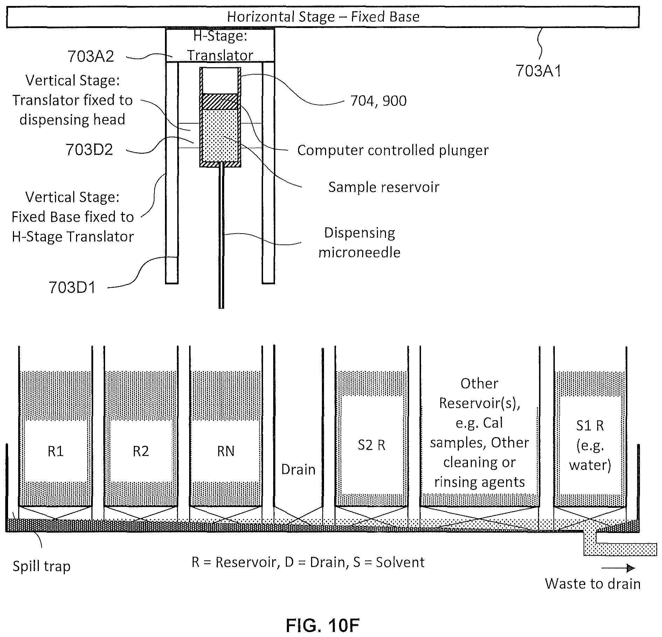

FIG. 10F illustrates the relationship between a dispensing system and multiple reservoirs that can be accessed by a dispensing system of some embodiments.

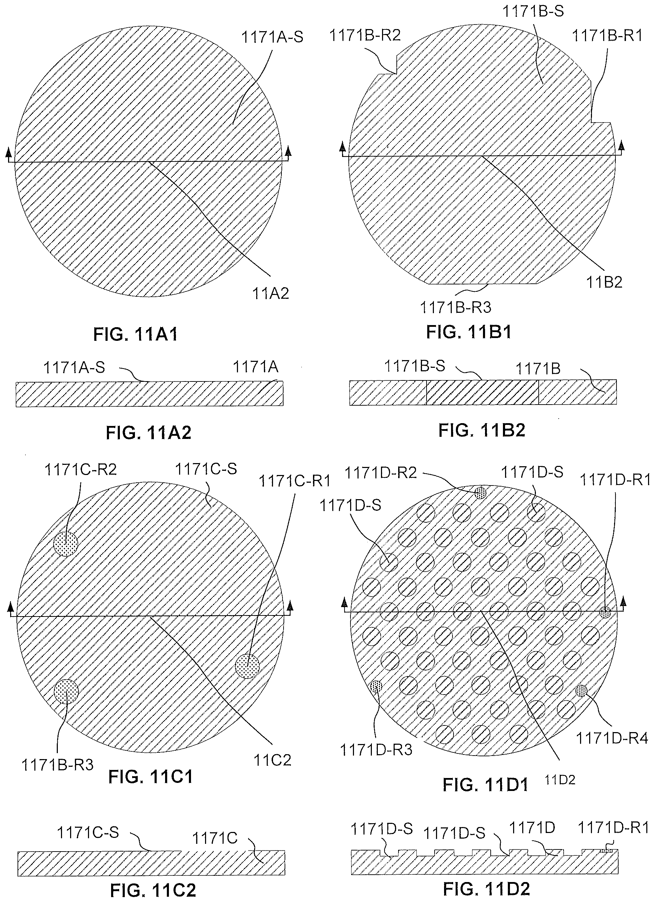

FIGS. 11A1 - 11A2 provide a top view and a side cross-sectional view, respectively, of a first example of a chemical biological disk (CBD) that may be used in some embodiments of the invention.
FIGS. 11B1 - 11B2 provide a top view and a side cross-sectional view, respectively, of another example of a CBD that may be used in some embodiments of the invention wherein the CBD includes registration surfaces for engaging a mounting structure that provides for upright loading into the system as well as proper rotational loading.
FIGS. 11C1 - 11C2 provide a top view and a side cross-sectional view, respectively, of another example of a CBD that may be used in some embodiments of the invention where alignment or registration features are marked or printed on the disk by writing or printing thereto when the disk is first loaded or formatted for use.
FIGS. 11D1 - 11D2 provide a top view and a side cross-sectional view, respectively, of another example CBD that include registrations marks as well as deposition location areas that take the form of indentations into which deposits can be made and analytes retained.

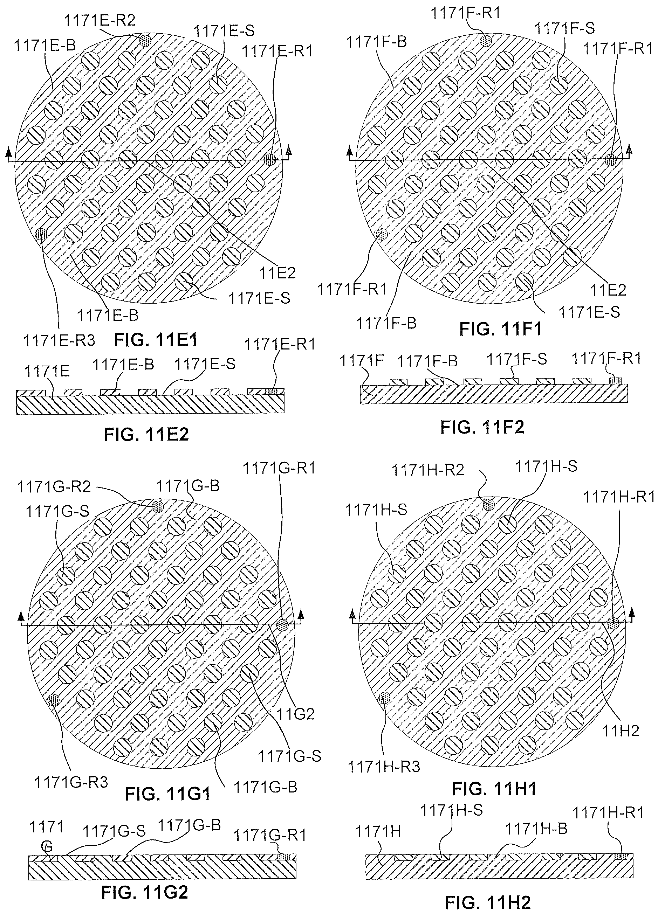

FIGS. 11E1 - 11E2 provide a top view and a side cross-sectional view, respectively, of a further example CBD but where the substrate is formed of a hydrophilic material but deposition areas are surrounded by a coating of a hydrophobic material that can be used to help ensure preferential placement of droplets and associated analyte deposits.
FIGS. 11F1 - 11F2 provide a top view and a side cross-sectional view, respectively, of a CBD having a substrate that is hydrophobic but has deposition locations that are provided with a hydrophilic or less hydrophobic coating.
FIGS. 11G1 - 11G2 provide a top view and a side cross-sectional view, respectively, of a CBD having a hydrophilic substrate that is etched in locations where a hydrophobic material will be deposited.
FIGS. 11H1 - 11H2 provide a top view and a side cross-sectional view, respectively, of a CBD having a hydrophobic substrate that is etched in locations where a hydrophilic material will be deposited or structured plasmonic features will be formed on the surface to produce signal enhancement.

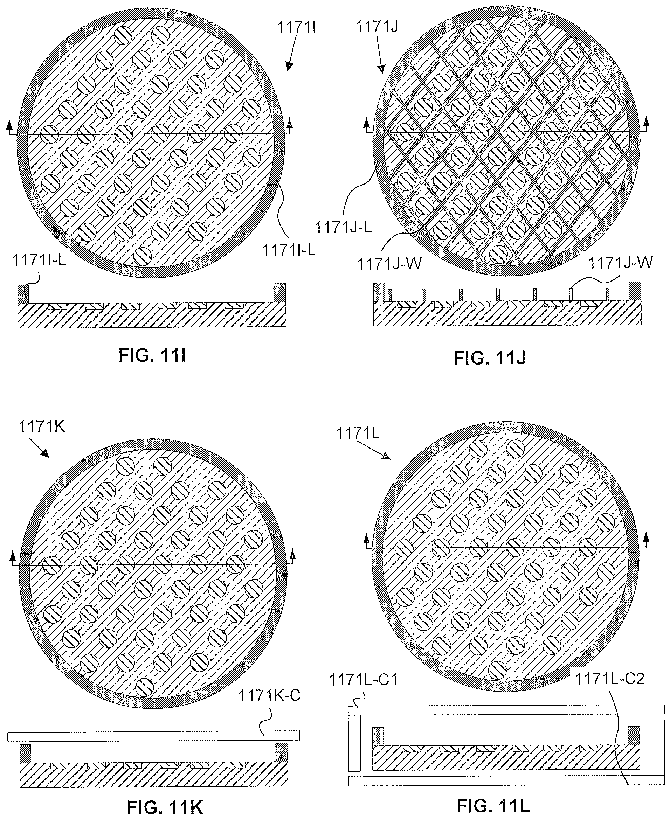

FIG. 11I provides a top view and a side cross-sectional view of a CBD that includes a protective lip around its perimeter to help ensure that deposits are not damaged by inadvertent scrapping of the surface wherein the CBD is otherwise similar to that of the CBD of FIGS. 11H1 and 11H2 with the exception of registration marks are not being shown and some of the deposition locations have been eliminated in favor of the protective lip.
FIG. 11J provides a top view and a side cross-sectional view of a CBD that includes not only a raised protective perimeter like that of FIG. 11I but where each deposition location has its own elevated perimeter wall that protects it during formation of neighboring deposits and against inadvertent contact.
FIGS. 11K provides a top view and a side cross-sectional view of a CBD with a simple cover or lid that protects the deposition surface from contamination when not in use.
FIGS. 11L provides a top view and a side cross-sectional view of another example CBD that is protected not only by an upper lid but also by a lower base structure and side walls which can be separated to provide access to the CBD surface when necessary.

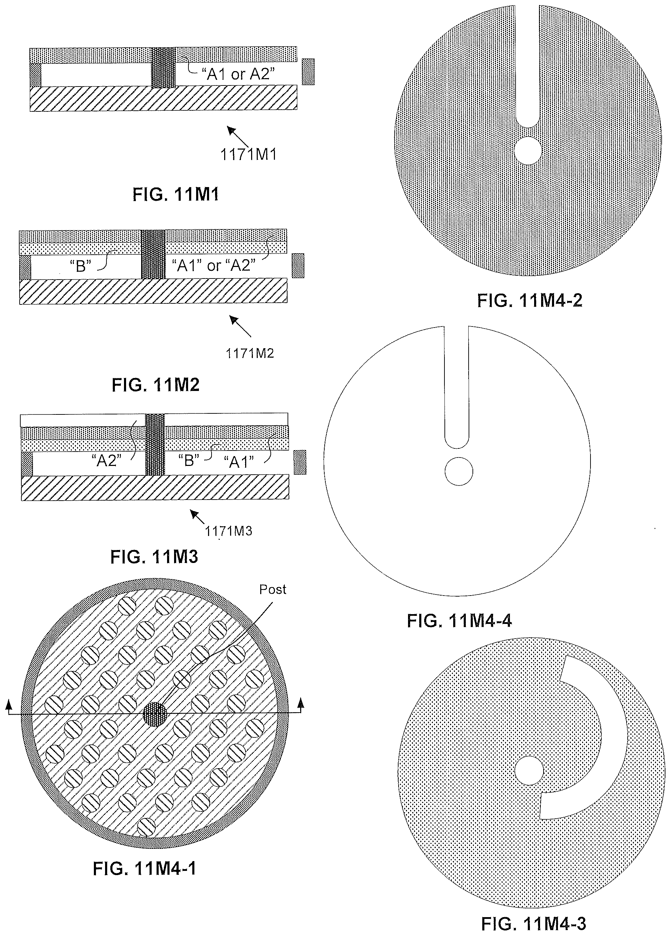

FIGS. 11M1 - 11M3 provide side cross-sectional views of three additional example CBDs which include different rotatable protective lid structures
FIG 11M4-1 provides a top view of the CBD without any lid but where a rotational shaft for supporting rotation of a lid can be seen while FIGS 11M4-2 to 11M4-4 provide three different types of lids that may be used alone or in combination where rotation of the lid is used to expose locations for deposition or reading.

### Detailed Description of Preferred Embodiments

Embodiments of present invention provide spectroscopic methods, apparatus, and/or systems for the detection of analytes found in liquids, in the air or other gases, or on surfaces. The methods involve the manual or automatic taking of, or capture of, samples from liquid, gases, or surfaces of interest which are used to create concentrated dried or semi-dried deposits to be tested. For example, a sample may be an extracted part of a liquid of interest that is being held or processed. It may include a material taken from a surface, potentially of known area, by swabbing, by application of a solvent, or by another extraction technique whereafter the extracted material may combined with a liquid to create a known volume. As a further example, material may be extracted from the air, potentially of known volume by direct entrapment in a liquid or by condensation on a surface followed by transfer to a liquid such that a known volume is obtained. An entire extracted sample, or a known volume of the sample, may then undergo controlled deposition in the process of creating a dried or semi-dried analyte deposit from a single deposition or from multiple overlaid depositions which can act as a test sample for spectroscopic analysis. In some variations the extracted volume may be used to create more than one independent deposition with the sample potions undergoing separate processing between successive independent depositions/tests.

Embodiments of the invention recognize several independent methods for contributing to (1) lowering of limits of detection for analytes, (2) reducing sample preparation time, (3) providing quantitative surface or volume concentration levels that existed in the original samples, and (4) providing permanent and easily accessible retention of analyte samples for record keeping and for potential future re-evaluation. Each of these various methods have independent usefulness by themselves and provide even greater benefits when used in combination regardless of excitation wavelength and spectroscopic detection method used, or combination of methods used. It is believed that the largest advantages come from combining two or more of these improvement methods along with use of deep UV (wavelengths between 200 and 280 nm and even more particularly with wavelengths below 250 nm) along with native fluorescence and phosphorescence detection and concurrent or subsequent and selective Raman detection methods.

Each individual method, combinations of methods, specific example implementation details, systems for implementing the methods and benefits are discussed further hereafter.

FIG. 1A provides a block diagram 100 illustrating a detection method that uses at least partial removal of the water media of an aqueous solution that is to be tested for the presence of any analytes. This removal or elimination will reduce interfering emission signals from the water carrier media to provide an improvement in detection limit (i.e., allow detection of lower concentrations of analytes). The method includes obtaining a sample of the water to be tested where the sample may be obtained manually or by an automatic extraction process (Block 100A). The obtaining of the sample may directly provide the sample into a dispensing apparatus such as a computer controlled digital syringe with a needle or micropipette as taught in US Patent No. 10,732,037, or using a different dispensing apparatus that can provide the small volumes of liquids of interest. Alternatively, the sample may be first extracted and located in a reservoir that the dispenser can access to load in a volume of the sample liquid.

Next a volume of the liquid sample is deposited onto a known location of a substrate such that the dispensed volume has desired width or configuration (Block 100B). As with the sample extraction and loading, the dispensing may occur manually or more preferably in an automated manner using a programmed controller (e.g., a microcontroller or group of microcontrollers that handle the entire process). Deposition volumes of interest may range, for example, from as little as 10 nanoliters (10 nL) or less, up to 5 microliters (5 uL) or more. In the present embodiment, a single droplet may be used to provide a total volume which will form the basis for supplying the analytes or multiple droplets may be dispensed on top of one another to provide for increased local buildup of analytes in a smaller area (e.g., and array with a smaller diameter than may be provided by a single droplet of the same total volume). Parameters of most interest in the present embodiment may include, for example: droplet volume, number of droplets, droplet size uniformity, volume precision, and target precision.

Droplet volume is the volume of each droplet as it is created by the dispenser. The droplet volume is not necessarily the same as the volume that is delivered to the substrate surface, as some amount of evaporation may occur prior to releasing the droplet from the dispenser. Each droplet will preferably have a known size, which will preferably be less than 5 uL, 3uL, 2uL, 1 uL, 0.5 uL, 0.2 uL, 0.1 uL, 50 nL, 20 nL, 10nL, 5 nL, 2nL, or even 1nL or less. As used herein uL means microliters and nL means nanoliters. Larger and fewer droplets (even a single droplet) may be used, or smaller more numerous droplets may be used to create a desired total volume. In some variations, net deposits may be made from a combination of droplets of different sizes with identical dropping locations or with somewhat offset droplet locations such that the net deposit is made from overlaying stacks material from multiple depositions. Optimal droplet volume and number of droplets to use may depend on a number of variables, including: (1) how rapidly dispensed droplets are to evaporate, (2) the dispensed droplet diameter, (3) growth in dispensed diameter if insufficient evaporation occurs between successive depositions, (4) accumulated volume error, (5) variation in droplet location precision, (6) total time to effectively dispense the required number of droplets and the time for evaporation to reach a desired level of completion, and the like. For a given set up, it is within the level of skill in the art to determine an appropriate range of droplet volumes, dispensing rates, and other parameters.

It may be desirable to hold the volume size of each droplet to a target size within a desired tolerance such as, for example, ± 20%, ± 10%, ± 5%, ± 1%, ± 0.5%, or ± 0.1%, or even less with more precise tolerances becoming more critical as the number of droplets increase and if the volume of dispensed liquid will be used in determining ultimate concentration of the analytes as found in the original sample.

The diameter of the droplet deposit depends on droplet volume, surface tension of the liquid, and hydrophobicity of the substrate surface. The residue left by single or multiple droplets typically form a set of "coffee ring" patterns of the evaporated materials in solution as well as particles including inorganic, organic, and biological particles. The desired range of droplet diameters are typically less than 5 mm, 2 mm, 1 mm, 500 um, 200 um, 100 um, 50um, 20 um, 10 um, or even less.

Desiccation or drying time of each droplet depends on droplet volume, surface area, substrate temperature, and possibility humidity, and is preferably less than 10 s, 5 s, 2 s, 1 s, 500 ms, 200 ms, 100 ms, 50 ms, 10 ms, or even less with quicker drying time being more preferred as the number of droplets increase. Droplet drying or desiccation rate depends on the surface area to volume ratio (A/V) of a droplet, with smaller droplets drying faster due to the higher ratio since A/V is a constant (K) divided by the droplet diameter. It also depends on the droplet temperature, substrate temperature, ambient humidity, and other factors. The desire for a faster drying time may need to be balanced with allowing a sufficient flow time to achieve spatial separation of different analytes if such separation will be used to lower the any relevant limit on detection.

Dispensing location precision may be important in achieving an ultimate analytic area that will be examined. It is preferred that single or multiple droplets are dispensed within a radius of <1 mm, 500 um, 200 um, 100 um, 50 um, 20 um, 10 um or 5 um from an identified spatial location on a substrate.

In some embodiments, it may be desirable to set droplet dispensing rates based on a certain level of liquid removal from a previously dispensed droplet prior to dispensing an overlaying droplet. Such criteria may be set by ascertaining a water removal time constant for a droplet as the time to reach 1/e water level. It may be desirable to set an average dispensing rate so that it is correlated to a certain number of time constants. For example, the time may be set to less than 7, 5, 3, 2, 1, 0.5, 0.3 or even 0.1 or less time constants. Alternatively, it may be set to lie within a particular range of time constants, e.g., between 5 and 7, 3 and 5, 2 and 3, 1 and 2, or 0.5 and 1.

Substrate materials and features may vary between embodiments and the types of samples that are intended to be analyzed. The substrate surface is most preferably a clean metal, fused silica, sapphire, or other surface which produces no background emissions to interfere with the Raman or fluorescence emissions from the analyte. Alternatively, the surface may be a chromatographic surface which enables lateral separation of multiple analytes within a droplet for further identification, or some other surface, including constrained fabrics or other porous material used to accumulate and concentrate samples for testing. Substrate temperatures may be regulated to control drying rates wherein the controlled temperatures may be above or below ambient temperatures. In other embodiments, substrates may be formed from other materials and/or coated with other materials.

The substrate may be spatially unstructured, physically, or plasmonically structured, or structured with coatings to control hydrophobicity, hydrophilicity, or other properties in a matrix of patterned areas on the substrate. A goal of spatial structuring may be to confine droplets to be formed on the surface to specific locations and/or to enable a suitable mapping arrangement to enable the substrate to become a physical recording medium for multiple samples to allow creation of a historical archive of samples evaluated. Such an archive enables subsequent testing to be done whether it be tests to revalidate prior conclusions, or to develop new insights using different excitation wavelengths, different emission types, updated software, updated algorithms (e.g., enhanced AI or machine learning algorithms), or updated spectral libraries of different materials. Such repeated tests might look for contaminants that were previously unknown or not considered of interest. In some embodiments, the substrate may be provided with one or more transparent or opaque covers that may physically protect the deposited samples from contamination during storage or non-use. Such covers may be manually or automatically removable via sliding or rotation and provide access for deposition and optical interrogation but otherwise protect the substrate surface from contamination from the environment. Such sliding or rotation may, alternatively, be used to provide access to selected portions of the substrate while other portions remain covered. Substrates may be provided with alignment or engagement features.

Turning back to the process of FIG. 1A, after the deposition of Block 100B, the process moves to Block 100C and the liquid in the deposited droplet is allowed to, or is caused to, at least partially evaporate and in some embodiments to substantially evaporate (e.g., removal of greater than 90%, 95%, 99%, or even 99.9%, or more of the liquid) or even completely evaporate prior to initiating spectroscopic detection. In some cases, a balance must be achieved between a desired or required extent of desiccation, a time to achieve it, and a limit of detection that is to be achieved so that appropriate detections can be made in reasonable times.

Next, the process proceeds to Blocks 100D and 100E, which call for exposing the analyte sample to excitation radiation (Block 100D), detecting resulting emission radiation (e.g., using Raman, Fluorescence, Rayliegh, or phosphorescence), and analyzing the resulting spectra to provide a preliminary or final determination concerning the presence and quantification of biological or chemical materials of interest (Block 100E). In some variations, for example, fluorescence and Raman may be performed over all portions of the deposited material, Raman may be performed only when certain fluorescence results are present, or Raman and/or fluorescence may only be performed in selected areas that are dictated by a manual or automatic visual imaging and analysis.

If necessary, the process proceeds to Block 100F which calls for repeating the operations of Blocks 100C - 100E as necessary until a final determination is made. Block 100F may be skipped in some implementations while it might be necessary in others. For example, one such situation might occur when analysis of fluorescence emission from an initial operation of Blocks 100D and 100E requires repeating to provide a Raman analysis (as noted above). Alternatively, if the examination yielded an indication of excess background water from Raman emission signals, further evaporation might be allowed to proceed followed by a further spectroscopic examination. In another alternative, if an examination showed that some low-level signals were of potential interest, it may be concluded that additional deposits should be made in hopes of obtaining enhanced emission signals.

FIG. 1B provides a graph illustrating the Raman spectrum of water with potassium nitrate contamination where the spectral background from the water masks portions the Raman signature of the potassium nitrate at a concentration of 100 parts per million (PPM or ppm) and makes portion of it completely unreadable at a concentration of 10 parts per million. This makes concentrations in the ranges of 10 ppm, 1 ppm, 100 parts per billion (ppb), and 10 ppb unreadable. This figure illustrates the problem with the presence of water when trying to achieve trace or ultra-trace level of detection.

Although the use of UVRS and UVRRS has eliminated many of the shortcomings of Raman detection for many materials of interest, Limits of detection (LOD) in water or other liquid backgrounds are still limited to the ppm and high ppb range (e.g., 100s of ppb), while emerging detection requirements are often in the low ppb (parts per billion), ppt (parts per trillion) range, and below. To achieve LODs in these ranges requires increasing the signal and/or reducing the noise or background. The methods proposed herein (e.g., in the first embodiment), while particularly useful with UVRS and UVRRS, are not limited only to the use of UVRS or UVRRS, but also benefit detection in the near UV, visible, or IR.

Water is a primary carrier for many of the trace materials of interest. However, although water is considered a good matrix for analytes at concentrations above ppm, water in fact produces significant signal both at the primary Raman bands of water and a broad background continuum spectrum of signal that spans a range from a few hundred wavenumbers to over 4000 wavenumbers as can be seen in FIG. 1B. The source of this "water background" signal is not fully understood but is a fact of careful measurement. This background is of primary interest when making measurement of trace contaminants in water backgrounds where the signals of interest are exceptionally low ppb or ppt range and where any background is problematic. This continuum background from water is a function of excitation wavelength, being nearly equal to the HOH scissors water band at 1635 cm-1 with excitation at 785 nm, about 70% of the HOH band at 532 nm, about 40% at 248 nm, and about 35% at 229 nm. For perspective, the 1635 cm-1 HOH scissors Raman band of water is only a few percent of the signal level of the OH stretch band near 3400 cm-1. However, it remains a significant factor for trace detection in water, along with the related shot noise. This broad background noise limits LOD to about 1 to 10 ppm for nitrates and does not allow detection of less concentrated, but also important, micropollutant substances like pharmaceuticals, pesticides, PSFSs, and the like.

Separately, but equally important, are fluorescence emissions from the target analyte or other dissolved or suspended particulate material that interfere with Raman detection within the excitation volume of a laser particularly when the excitation wavelength is not below 250 nm. When below about 250 nm, Raman emissions are in a separate spectral region from fluorescence for most substances of interest within a sample and thus fluorescence does not interfere or obscure detection of Raman emissions when deep UV below about 250 nm is used.

Limits of detection can be improved only by increasing the detected signal or decreasing the noise and/or background signal and its related shot noise. Improving the signal is accomplished by both the design of the spectrometer to increase throughput efficiency and/or increasing the excitation energy. For detection of trace materials immersed in a water background, the excitation volume produced by the laser traversing the liquid encompasses primarily the liquid, and only a trace amount of the targeted material. Increasing laser excitation energy linearly increases signal from the target material, but also linearly increases the signal of the dominant background liquid, leaving no improvement in signal to background. The background provides a threshold above which the analyte signal competes with the shot noise of the background plus any other noise induced during detection. Background is significant for materials in the low ppb, ppt, and ppq concentration, and addressing the background signal is important to achieving very low limits of detection.

A focus of the present patent application is on reagentless methods and systems. Concentration methods include physical separation of the analyte from background and/or various forms of chromatography. For automated systems with no manual intervention, physical separation methods include evaporation, filtration, centrifugation, and various forms of chromatographic separation. Water born analytes of interest are both dissolved components as well as suspended particulates. In both cases, evaporation methods for concentration apply. Filtration methods are only for particulates, where large quantities of air or liquids are passed through a filter, and either detected directly on the filter or extracted from the filter and detection in a static or flowing stream. Larger particulates, whether suspended or not, may be identified by the methods and systems of the present invention so long as the particulates are not so large as to interfere with the dispensing process that is used.

Concentration by evaporation provides many possibilities ranging from partial evaporation to total evaporation. In partial evaporation, the sample may be heated but only to an extent that no damage occurs to the analyte during the time required to reduce the volume of the matrix by factors from 2 to 100 or more. This may be followed by resuspension of the analyte with added matrix material (which may be the same or different from the original liquid) to remove any analyte condensed or remaining on the wall of the sample container. This process can be repeated several times to further concentrate the analyte. The final sample is then measured with DUV-RS, DUV-RRS, DUV-SERS, DUV-LIF, or other methods in the near UV, visible, or IR. For many IR applications, reducing the amount of water or other liquid within a spectroscopic interrogation region around the analyte is beneficial for reducing or eliminating absorption rather than emissions from the water or other fluid. An example of this is with Mid-IR methods, where the primary limitation is water absorption even in very thin water features.

Another approach is using total evaporation of the liquid matrix, leaving the analytes as materials deposited and dried on a substrate surface which is then detected, again using deep UV or other methods, as described above. Within the dried deposit are all the ingredients contained in the water sample, except for any material evaporated along with the water. For the total evaporation approach, there are several alternate methods for achieving desiccation and concentration: (1) a single dropped and dried droplet may be the analyte source or a stack of smaller dried droplets may be the analyte source wherein the overall deposit diameter may be smaller and the amount of deposited analyte may be larger; (2) a single larger droplet that is partially dried or completely dried may be followed by one or more a smaller droplets that is/are deposited over the larger drop to concentrate the previously deposited material into a smaller area deposit, or alternatively the one or more smaller droplets are swept over the dried or partially dried droplet to absorb the analyte deposit and concentrate it into a smaller area; and (3) a continuous drop and dry method where droplet formation is continuous with sample constantly drying during deposition.

FIG. 1C provides another graphic, this time illustrating the intensity improvement that can be achieved when the water background is sufficiently reduced for two example analytes (sodium nitrate and potassium dihydrogen phosphate). Such reductions in background can lead to significant improvement in the limits of detection.

FIG. 2 provides a block diagram 200 illustrating a detection method embodiment using repeated overlaying depositions of relatively small volumes of an aqueous solution so as to provide smaller deposition areas and fast drying times or evaporation rates thus allowing rapid buildup of higher concentrations of analytes on the substrate surface with substantial removal, if not complete removal, of the water media prior to overlaying droplets. This provides smaller analyte deposition areas, higher analytes emission signals due to larger amounts of analytes, and reduced interference media emission signals, thus providing an improved limits of detection with a short processing time.

The process of FIG. 2 has some process steps, elements, or operations like those of the process of FIG. 1. Block 200A of FIG.2 calls for obtaining an aqueous sample to be tested. Block 200B of FIG. 2 calls for the depositing of a known volume of the sample to a predefined location on a substrate, but unlike Block 100B of FIG. 1A, less than a total volume that is to produce an analyte deposit for analysis is required to be deposited. Blocks 100C and 200C of FIGS. 1A and 2 provide for allowing or causing at least a portion of the liquid to evaporate from the sample. Block 200D of FIG. 2 is new and is a consequence of the incomplete deposit associated with each operation of Block 200B. Block 200D provides for repeating the operations of Blocks 200B and 200C one or more times, such that incomplete volumes are dispensed on top of one another, and such that the total volume dispensed from all deposits provide the total volume intended to produce the analyte deposit that is to be examined. Blocks 200E, 200F, and 200G of FIG. 2 are similar to Blocks 100D, 100E, and 100F, respectively, of FIG. 1A and they call for the application of excitation radiation (Block 200E), detection of emission radiation, analysis, and reaching either a preliminary conclusion or a final conclusion (Block 200F), and if a final conclusion is not reached from the operations of Block 200F, then repeating the operations of other blocks as necessary to reach a final conclusion (Block 200G).

In a first example of the process of FIG. 2, complete desiccation may be achieved. A very small droplet of the analyte within a liquid matrix or media is dropped onto a substrate surface with droplet volume in the nanoliter range (e.g., 1s to 100s of nL per droplet), producing, for example, totally evaporated analyte droplet deposit sizes in the order of 50 to 250 micrometers within seconds. At an LOD detection goal of 100 parts per billion (10-7 g/mL) for an exemplary sample of NO3-N analyte, this is approximately 10-13 g/nL or 1.6 pg (picograms) per single 16 nL droplet, a mass for which detection has been demonstrated in a miniature deep UV Raman spectroscopy instrument. Detection of NO3, which has a 4.43 times larger mass than that of NO3-N, has been demonstrated with signal to noise ratio (SNR) values significantly over 200:1. Therefore, limits of quantification (LOQ) of NO3 with SNR of 10:1 would be 80 fg (femtograms) of NO3, or a concentration in the parts per trillion range for a single 16 nL droplet of water doped with 100 ppb of NO3-N.

To quantify the concentration of an analyte in the liquid sample, the measured mass of the analyte deposit is divided by the volume of the sample deposited. The volume deposited per droplet needs to be highly controlled to accurately determine the concentration of the material in the source fluid. One example of volume control is the use of a syringe pump where the droplet volume is accurately measured by the linear displacement of a plunger. Droplet volumes by this method have demonstrated accuracy within a few percent at volumes down to about 10 nL. This enables quantification of each analyte within a sample. Other droplet formation and deposition methods are possible including ultrasonic and other methods, where the droplet volume is measured during the process.

Small droplet deposit size is preferrable for two reasons: (1) to provide for rapid evaporation at or near room temperature due the high surface to volume ratio of small volumes; and (2) to produce evaporated deposit diameters compatible with the typical diameter of a focused laser or LED beam used to collect one or more of Rayleigh, Raman, fluorescence emission and/or photoluminescence spectra from the sample and related decay rates. Some methods, for example, may be limited to just fluorescence, just Raman, a combination of fluorescence and Raman, fluorescence followed by Raman as appropriate while others may involve other emission types, or combinations thereof. The lateral dimensions of the droplet are determined by the surface tension of the fluid and the hydrophobicity of the surface. For example, surface tension in wastewater treatment plants have a range from about 30 to 70 dyne/cm according to some sources, producing a range of droplet diameters at the same volume. To ensure signal from all of the deposited and desiccated sample is collected, the deposit can then be scanned with the laser beam to ensure that optical emissions from the entire deposit are collected. Alternatively, epi-illuminated visible or UV imaging can be used to reduce the scan area to the regions of interest within a deposit. Areal integration yields the total mass of the deposit with the spectral composition used to determine the mass of each CB component of the deposit. In some cases, it may not be necessary to scan a sample, depending on the overlap of the condensed spot and laser interrogation spot diameters and the error that is willing to be accepted.

This drop-and-dry process of this first implementation can be repeated many times to increase the mass of analyte deposited within a small area of 50 to 500 micrometers in diameter, or perhaps several times lower or several times larger (e.g., 20 to 2000 micrometers). By either of these methods, the limits of detection can be improved by many orders of magnitude, enabling detection of analytes in the low ppb and ppt range, or below. Setting the temperature of the substrate to be above ambient but low enough to avoid any damage, alteration, or evaporation of any analyte within the sample will help to ensure consistent deposit sizes and reduce the dehydration time. In some cases, lowering pressure, lowering the temperature toward freezing, air circulation, and/or otherwise lowering humidity (e.g., by inclusion of nearby desiccants) may aid in promoting more rapid dehydration as well.

Since the deposit sizes are so small, many deposits may be collected and formatted on a small substrate, e.g., of the order of a several cm or tens of centimeters in diameter (e.g., 20 cm, 10 cm, 5 cm, 2 cm or even less). This in turn enables collecting a permanent physical chemical and biological archive record of many hundreds, thousands, tens of thousands of samples and measurements on a single replaceable disc for future re-measurement using enhanced deep UV detection hardware or software of other testing methods. Each of the individual deposit includes a complete collection of the full set of chemical and biological analyte materials found at the time that respective analyte sample was created. This chemical/biological "hard drive" (CBHD) provides a formatted, and re-analyzable history of the entire chemistry and biology at the time of the original deposition and thus allows retaking of spectral data and re-analysis as well as reexamination of prior conclusions if a water quality event needs to be further studied, which may be done with original testing parameters or with different parameters and potentially improved hardware and software detection methods. For example, one such CBHD may be able to record the entire CB history for over 3 years of operation of a facility with sampling occurring every hour, i.e., over 30,000 CB deposits on a 10 cm diameter hard drive. Shorter or longer recording periods can be accomplished with smaller or larger CBHD structures or devices as well as with different sized depositions and/or with different spacings between such depositions.

This implementation enables production of a physical, desiccated chemical/biological archive of events. In addition to depositing test samples, CBHDs may also include deposited calibration standards of known analyte chemistry, quantity, and original concentration as well as deposited or otherwise marked alignment or positioning features. Since detection measurements are conducted without the presence of water or any other liquid, there is no possibility of biofouling or other optical throughput degradation to alter the quantitative results.

In another method variation, a larger droplet may be swept with a smaller droplet to decrease deposition area and improve concentration. In this approach, one or more larger droplets may be deposited to locate a larger total mass of analyte on the substrate. In this case one or more initial droplets may have volumes of perhaps several microliters, or 250 - 1000 times the amount of the droplets of the prior method, causing deposit sizes of perhaps 1 mm or more in diameter. After this droplet, or these droplets, are dispensed, released, and dried, a syringe with a small, 5 - 50 nL droplet (e.g., 10 nL) is suspended from the dispenser tip and is dragged over the larger dried droplet or droplets to sweep or gather the analyte contents spread over a larger area into a much smaller area, where the smaller droplet dries, leaving analyte in a concentrated form. Control of this method may be guided by visible or UV optical imaging of the region encompassing the droplet formation. In some variations, additional sweeping may be performed sequentially with additional small droplets.

Another approach involves a continuous drop and dry method which involves a syringe pump continuously feeding the source water at a rate sufficiently slow that drying occurs, on average at approximately the same rate as deposition, enabling increasing concentration of analytes in a small, dried droplet size (i.e., analyte deposition area). In variations of this approach, periodic pauses in deposition may occur to allow account for any difference in deposition rate and drying rate.

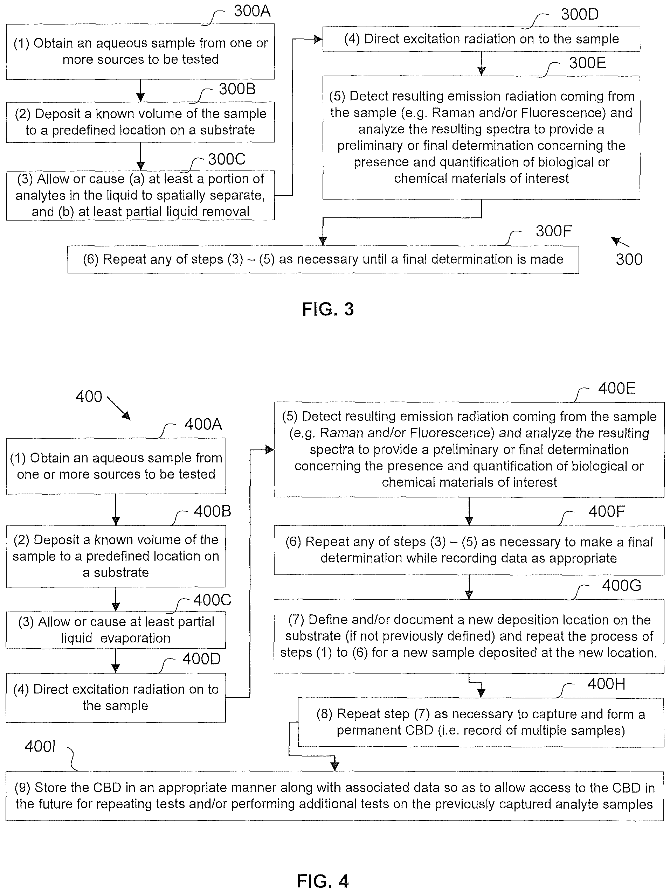

FIG. 3 provides a block diagram 300 illustrating another analysis method embodiment. This method uses a physical analyte separation method to reduce any interference that might exist when a resulting deposit has multiple analytes to provide improved detection limits. The process starts with Block 300A, which includes obtaining an aqueous sample to be tested. It then moves to Block 300B, which calls for depositing a known volume of a liquid sample to a predefined location on a substrate, and then moves on to Block 300C, which calls for allowing or causing at least a portion of analytes in the liquid to spatially separate along with at least partial liquid removal to occur. In some variations of the process, repeated overlaying depositions may occur along with repeated spatial separations and liquid evaporations. As with the embodiments of FIGS. 1A and 2, the process then proceeds to supplying excitation radiation to the analyte sample (Block 300D), detecting emission radiation, analyzing the resulting spectra, and making initial or final determinations concerning the sample (Block 300E), and if necessary repeating steps in the event a final determination was not made as part of the operations of Block 300E (Block 300F).

FIG. 4 provides a block diagram illustrating another analysis method embodiment that uses a deposition substrate in the form of a storage structure or disc to which a plurality of different test sample depositions can be located and remain spatially distinct such that permanent storage is provided for a plurality of analyte deposits, which in turn may be used to provide enhanced insights from future spectroscopic examinations. In this embodiment, the process begins with securing a first aqueous liquid sample to be tested (Block 400A). The process then moves forward to depositing a known sample volume at a predefined location on a substrate (Block 400B). The operation(s) of Block 400B may involve, for example, deposition via a single droplet, multiple overlaid droplets of similar sizes, overlaid droplets of difference sizes, or a deposition via continuous deposition stream. The process then moves forward to Block 400C, which provides for allowing or causing at least partial liquid evaporation to occur. In variations of this process, the activities of Blocks 400B and 400C may be interleaved as a series of alternating operations or as a substantially parallel set of processes or operations. As with the embodiments of FIGS. 1 and 2, the process then proceeds to supplying excitation radiation to the analyte sample (Block 400D), detecting emission radiation, analyzing the resulting spectra, and making initial or final determinations concerning the sample (Block 400E), and if necessary repeating steps as necessary in the event a final determination was not made previously (Block 400F). The process them moves on to Block 400G, which calls for defining and/or documenting a new deposition location for placement of a new sample to be tested (if not previously defined or documented) and then repeating the operations of Blocks 400A - 400F for the new sample at the new location. Next, the process moves to Block 400H which calls for repeating the operations of step (Block 400G) for any additional samples to be analyzed and recorded such that a permanent multi-sample CBD is created. Finally, the process moves to Block 400I and concludes with the CBD being stored in an appropriate manner along with associated data to allow future access to the CBD as needed for repeating tests or performing additional tests on the previously captured analyte samples.

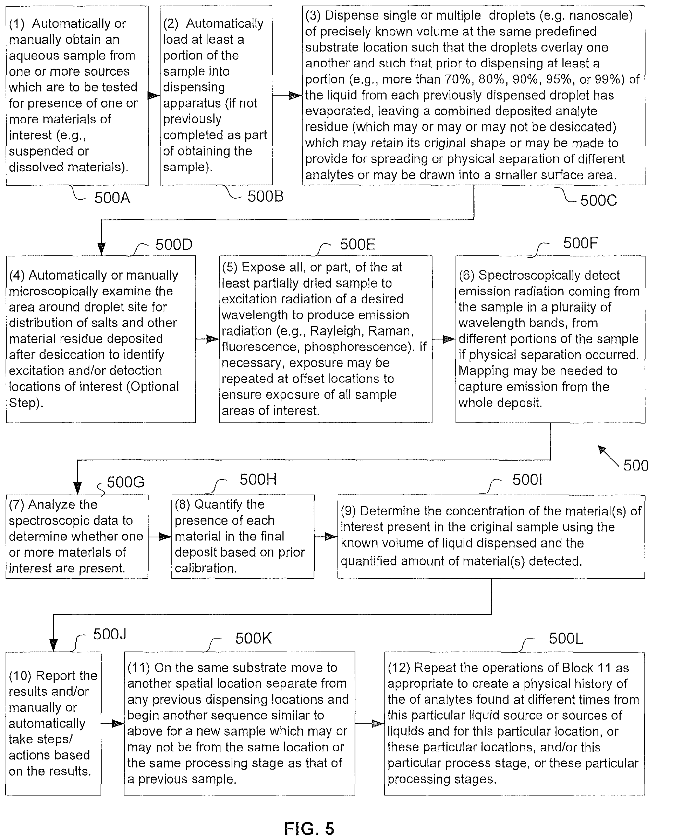

FIG. 5 provides a block diagram 500 illustrating a detection and analysis method providing for partial or complete aqueous media removal, for formation of a final analyte deposit from a single deposition or multiple depositions, for allowing the final deposit to retain its deposition configuration or to undergo physical separation of analytes, and for using a multi-sample CBD substrate to provide a physical record of analyte presence over time. The spectroscopy method of FIG. 5 provides for chemical or biological material detection from an aqueous or water-based sample. In variations of the process, the material may be a non-water-based liquid. The process starts with automatically or manually obtaining a liquid sample which is to be tested for the presence of one or more materials of interest as set forth in the operation(s) of Block 500A. The sample may be extracted as part of an in-line, on-line, at-line, or off-line process. The process may be part of a feedback loop that uses the determinations made, or conclusions drawn, to make or aid in the making automated or manual changes to the process that the liquid may be undergoing (e.g., contamination removal in a water treatment plant, ensuring proper concentration level in a beverage, food, or pharmaceutical processing or manufacturing facility, confirming adequacy or inadequacy of cleaning steps already taken, and the like). Such changes may add in additional process operations, may remove unnecessary operations, and/or may change process parameters associated with existing operations.

If not already completed as part of the operation(s) of Block 500A, the process automatically loads at least a portion of the sample into dispensing apparatus as set forth in the operation(s) of Block 500B.

The operation(s) of Block 500C call for the dispensing of one or more nanoscale droplets a target sample location on a substrate (e.g. a single location or plurality of closely positioned locations) such that the dispensed droplets largely overlay the deposition area of one another, and such that prior to dispensing a subsequent discrete volume of liquid, a substantial portion of the liquid from each previously dispensed droplets has evaporated (e.g., more than 70%, 80%, 90%, 95%, or 99%), leaving a deposited analyte residue which may retain its original shape or may be made or allowed to spread to provide for spatial separation of different analytes or may be drawn into a smaller surface area. In alternative embodiments, the dispensing of droplets may be replaced with a continuous flow of liquid or a series of discrete flows. In the present embodiment, the substrate is configured for receiving depositions from multiple samples at distinct locations and for use of the substrate as a permanent, or re-readable, archive of the deposited analytes. In alternative embodiments, a single sample substrate may be used and the substrate may not be treated as permanent or re-readable archive.

Next, the operation(s) of Block 500D calls for the optional, automatic, or manual, microscopic visual, UV, IR, or other examination of the area around target location. The examination may inspect the distribution of salts and other material residue deposited after desiccation to identify and potentially flag specific excitation and/or detection locations of interest.

After the operation(s) of Block 500D, the process moves to Block 500E, which calls for the exposure of all, or part, of the at least partially dried sample (i.e., the analyte sample) to excitation radiation of a desired wavelength or wavelength band to produce emission radiation (e.g., Rayleigh, Raman, fluorescence, phosphorescence).

In Block 500F, the process provides for the spectroscopic detection of emission radiation coming from the sample in a plurality of wavelength bands and possibly from multiple exposures of different portions of the sample if necessary to read the entire deposit (e.g., if physical separation occurred). If multiple exposures are necessary, mapping and data manipulation or integration may be used to capture and interpret the emission signals from the whole deposit.

In Block 500G, analysis of the spectroscopic data is performed (either locally or by a remote analyzer) to determine whether one or more analytes of interest are present, and if so, the operation(s) of Block 500H quantify the presence of each such analyte that was identified in the deposit based on a prior calibration of the system, post-detection calibration of the system, or a combination of multiple such calibrations. Such calibrations may be based on data extracted by the spectrometer of the system from one or more deposits of known quantity and/or concentration.

If not already performed as part of the operations of Block 500G or Block 500H, the operation(s) of Block 500I are performed. In the operation(s) of Block 500I, the concentration(s) of the analyte(s) of interest found in the original liquid sample are determined. This determination is made based, at least in part, on the known volume of liquid dispensed and the amount of each material that was found from the spectroscopic analysis. In some variations of this embodiment, the determination of concentration is optional or it may be performed directly without necessarily initially performing the quantification of Block 500H.

Block 500J sets forth operation protocol for reporting the results of the analysis and/or manually or automatically using the results to take steps/actions. Such steps or actions may cause modification to the processes that are being used in processing the liquid where the process may increase treatment, decrease treatment, maintain a current treatment plan, otherwise modify the treatment, and/or call for an immediate or expedited resampling (perhaps after a testing system recalibration or cleaning), and the like.

Next, as part of operation(s) of Block 500K, the substrate is incremented to allow deposition at a new sampling location and/or is otherwise made ready for receiving a next sample. Relative movement of the substrate and dispensing system occurs to position the dispenser over and/or in proximity to a next dispensing or target location on the substrate wherein the target location is different and spatially separated from any previously used dispensing locations. In some variations, the new sample location may be cleaned, or it may undergo exposure, detection, and analysis to confirm it readiness to receive a next sample and/or to record any background data can be subtracted from any sample readings that are produced. After appropriate positioning (and any other readiness) is achieved, the operations of Blocks 500A - 500J are repeated for the new sample and the new dispensing location. In addition to depositing the sample on the substrate and making any preliminary detections and analysis, additional information is captured and stored on the substrate or on a separate storage device such as information that correlates the location to a specific sample, the source of the sample, the time of the test, any relevant processing, deposition, or detection parameters, spectral information, and conclusions reached, and possibly any process changes that were initiated as a result of the testing. Finally, in Block 500L, the process of Blocks 500A - 500K are repeated for any additional samples that are to be captured on the substrate to create a physical history of the chemistry of water-borne analytes found in the water source (whether they be contaminants, intended components, hazardous, or beneficial).

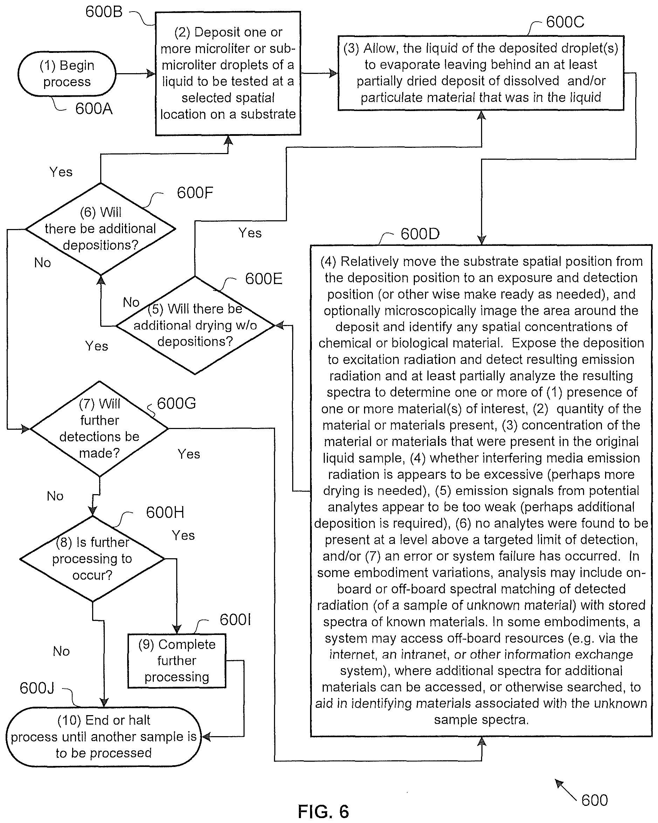

FIG. 6 provides a flowchart of another method for trace chemical or biological material detection from a liquid sample, but with a focus on detecting and handling example anomalies that might occur. The process begins with Block 600A, which assumes that a liquid sample to be tested has been loaded into a dispensing apparatus and is ready for dispensing. The dispensing apparatus may take on a variety of different forms such as, for example, a computer-controlled syringe or micropipette. It is also assumed that all relevant dispenser components (e.g., syringe, micropipette, needle, and the like) have been cleaned and are ready to receive such material. Cleaning may take a variety of forms with preferred forms potentially being dependent on the type of liquid being tested and the purpose of the test. Most particularly, the purpose of cleaning is generally to ensure that a sample is not contaminated by material remaining in or on the dispenser from a prior test. Such contamination may cause inaccurate conclusions to be drawn, particularly when materials of interest are being evaluated in the PPM, PPB, PPT or even in the PPQ ranges. In some embodiments, preparation of the dispenser might involve a separate cleaning process followed by a rinsing process, or it might just a rinsing process. Cleaning may involve the initial use of a detergent and/or one or more solvents which may be, for example, heated or agitated (e.g., ultrasonically). It may also involve subjecting the dispenser to chemically reactive agents or radiation such as ozone, plasma, deep UV radiation, steam, and the like. Rinsing may involve loading the dispenser, one or more times, with sterile water, with alcohol, with other solvents, or combinations thereof followed by operating the dispenser. Cleaning or rinsing may also involve insertion of a needle portion of the dispenser into and out of a cleaning solution or solvent one or more times without necessarily drawing the solution into the needle channel to remove any residual material from the outside of the needle. Such operation may be done with ultrasonic agitation of the solution or of the dispenser itself along with heating or other exposures as noted above. In some embodiments, the cleaning may simply involve rinsing the dispenser one or more times with the sample material that is to be tested wherein it may be assumed that one or more such rinses would remove any material not found within the sample itself. Alternatively, spectroscopic testing may be performed multiple times with two or more separate deposits from the same liquid source, wherein the multiple analyses may confirm that the determinations made by the latest two or more tests produce substantially identical results, thus providing an indication any presence of contamination has been adequately reduced. In some embodiments, at the end of the testing process, the dispenser may undergo immediate cleaning and possibly reloading with analyte-free liquid, particularly if there will be a significant delay before a next test. In other embodiments, the dispenser may be loaded with sample material that may be replaced with fresh sample material prior to subsequent testing.

In the process of FIG. 6, it is also assumed that a sampling station exists from which a sample of the material may be accessed either manually or automatically. In some embodiments, a single instrument may be responsible for testing material from different sources (e.g., from the same type of material but at different processing stages). Such an instrument might use different dispensers for each source or use a common dispenser that accesses material from the separate sources. Such a system would include separate sampling stations with each station, for example, being emptied and refilled with fresh sample material periodically or with each stage being continuously refreshed with a stream of material that can be sampled. In variations of the process of FIG. 6, the above functions may be specifically added to the process or components and controls may be added to systems for implementing such functions. In some embodiments, components and controls might include, reservoirs, pumps, filters, flow paths, drains, UV exposure sources, ozone production sources, fans, heaters, sensors, shields, control chambers, and the like, along with either open loop or closed loop controls that may incorporated learned and trained AI of ML implemented processes or controls.

After Block 600A, the process immediately moves to Block 600B, which calls for the depositing of one or more microliter or sub-microliter droplets (of a liquid that is to be tested, e.g., water) at a selected location on a substrate.

The process then moves to Block 600C, which calls for allowing the liquid of the deposited droplet or droplets to evaporate to leave behind an at least partially dried deposit of dissolved and/or suspended material that was in the liquid. Depending on the deposition method used, the operation(s) of Block 600C may not be necessary as sufficient evaporation may have already occurred by the time the last droplet is dispensed or will have occurred by the time spectroscopic detection is ready to be made.

The process then moves to Block 600D, which calls for relatively moving the substrate spatial position from a dispenser-based deposition position to a spectrometer-based exposure and detection position. Optionally, the deposited analyte may be microscopically imaged, manually, or automatically, to identify any spatial concentrations of chemical or biological material. Such identification may be used to align the sample and spectrometer and/or in setting a multi-exposure and detection process. The exposure of the deposition to excitation radiation and the detecting of resulting emission radiation and the at least partial analysis of the resulting spectra may be used to determine, for example, one or more of: (1) presence of one or more material(s) of interest, (2) quantity of the material or materials present, (3) concentration of the material or materials that were present in the original liquid sample, (4) whether interfering media (e.g., water or other liquid) emission radiation appears to be excessive (perhaps more drying is needed), (5) emission signals from potential analytes appears to be too weak (perhaps additional deposition is required), (6) no analytes were found to be present at a level above a targeted limits of detection, and/or (7) an error or system failure has occurred which might require user invention or repetition of one or more of the deposition, detection, or analysis processes. In some embodiment variations, analysis may include on-board or off-board spectral matching of detected radiation (of a sample of unknown material) with stored spectra of known materials. In some embodiments, a system may access off-board resources (e.g., via the internet, an intranet, or other information exchange system), where additional spectra for additional materials can be accessed, or otherwise searched, to aid in identifying materials associated with the unknown sample spectra.

Next, the process moves to decision Block 600E, which asks whether additional drying will occur without additional depositions. If not, the process moves to Block 600F, but if so, the process moves back to Block 600C and after its completion moves back to Block 600D and again to Block 600E.

Next, the process moves to decision Block 600F and asks if there will be additional depositions on the sample location. If not, the process moves to Block 600G, but if so, the process moves back to Block 600B and then again through Blocks 600C, 600D, and 600E before re-encountering Block 600F.

At Decision Block 600G, an enquiry is made as to whether further detections will be made (e.g., Raman detections will be made following a previous fluorescence detection). If the answer is no, the process moves to Block 600H, but if not, the process moves back to Block 600D.

At decision Block 600H, an enquiry is made as to whether additional processing (of an unspecified nature) will be made. If the answer is no, the process moves forward to Block 600J, but if the answer is yes, the process moves forward to Block 600I which calls for the performance of the further processing. From Block 600I the process moves forward to Block 600J which calls for the end of the process or at least a halting of the process until another sample is to be processed.

Preceding or following the operation of Block 600J, sample analysis will include digital signal processing of the radiation emitted from the sample to include spectral matching of the unknown spectra with known spectra held in an on-board library or extracted from another source using a connection to the internet, or other communication link. When a match is found and the unknown sample is identified and concentration determined, the process may proceed to the handling and examination of a next sample. Such processing may occur as part of the operation of Block 600D, as part of Block 600I, or alternatively it may be performed after Block 600J particularly if valuation or confirmation of prior results is desired or if enhanced analysist is to be performed.

FIG. 7 provides a schematic illustration of a system for implementing a method for trace chemical or biological material detection from a liquid sample. The system of FIG. 7 may be used in implementing the methods of any of FIGS. 1A or 2 - 6, or combinations or variations thereof.

The system includes a housing 711 that holds the various components of the system with some possible exceptions, including for example: (1) a power source for providing energy to the system, (2) inlet and outlet feed line extensions and drain lines that may provide for intake and removal of liquid samples and possibly solvents and other cleaning materials, (3) filters for liquid sample materials that ensure that no particulates can clog or otherwise interfere with dispenser operation, and (4) environment control systems. The housing may provide protection for the system components and isolation of the processes from external influence (e.g., contamination, environmental changes, and the like) as well as protection of users and others from exposure to radiation, ozone, or other materials or process operation risks. The housing may include windows, access ports, as well as input controls and output devices for interfacing with users and other equipment or systems. The housing itself may provide support for system components, or it may simply function as shell that surrounds system components, which may be supported by a base and/or other framing elements. If the system is small and portable, the housing may include handles, wheels or other features that promote movement of the system.

The system also preferably includes computer control components 701 and associated operation components that are held within the housing However, in some embodiments some of these control and operation components may be located outside the housing with wireless or wired interfaces extending through the housing. Such components may include, for example, one or more programmed computers for controlling operation of the system based on one or more predefined programs. The programs may operate based solely on programmed parameters or may be partially controlled by input parameters entered by a system operator and/or based on enhanced processes generated from ML and/or AI algorithms that have been trained using data gathered by the system or data gathered by other systems. The computer systems may include operational memory and permanent program and data storage systems as well as programmed input and output hardware. The components may also include various wired or wireless communication systems and sensors such as temperature sensors, humidity sensors, contact sensors, force sensors, continuity sensors, capacitive sensors, inductive sensors, magnetic sensors, rotation sensors, flow sensors, optical sensors, rotational and linear motion sensors, and the like, which may be used to indicate motions, positions, and status of various components. The computer and sensor components may work in conjunction with one or more microcontrollers to provide open loop, closed loop, or combination control of the system components. The microcontroller(s) may be configured to operate motors, servos, steppers, solenoids, valves, sensors, lights, detectors, lasers, heaters, coolers, fans, and the like to provide operation and integrated control of various system components.

In some embodiments, the system will include one or more XY (linear) or R Θ (rotational) stages with fixed and moving platforms (e.g., 703A1 & 703A2 and 703B1 & 703B2) for lateral or horizontal movement of the dispensing device(s) (705) and/or the spectroscopy exposure and detection system (704) and/or the lighting and camera system (706) relative to the deposition substrate 710-A and the various liquid reservoirs or chambers (707A), dump or drain regions (707B), cleaning station components (707C), and potentially for interacting with any movement calibration elements (not shown), and the like. The system will also include one or more Z-stages with fixed and moving platforms (e.g., 703C1 & 703C2 and 703D1 & 703D2) for vertical or perpendicular movement of the dispensing device(s) (705) and/or the spectroscopy exposure and detection system (704) and/or lighting and camera system (706) relative to the deposition substrate 710A and the various liquid reservoirs or chambers (707A), dump or drain regions (707B), cleaning stations (707C), and possibly for interacting with any movement calibration elements (not shown). In some variations, the system may use or include rotary motion elements (not shown) that move components (dispensers, lighting systems, camera systems, detectors, substrates, and the like) in and out of operational positions. For example, a dispensing syringe or other device may be located on a robotic arm that provides rotational and/or translational motion or rotary arm that swings the dispenser or device to an operating position (e.g., a dispensing position or a collection position) with any required vertical movement moving the arm and the dispenser or device or just the dispenser or device itself.

The system may also include a releasable mounting and alignment system 710B for the substrate. The releasable mount may include a heater or other elements to aid in the evaporation of liquid upon deposition.

In other embodiments, additional components and functionality may be provided by the system such as additional evaporation promoters like desiccants, dehumidifiers, fans, surface heaters, air heaters and chillers, and air intake and exhaust filters and fans, and the like (708-A). For example, the system may include components and controls for providing access to a covered or protected substrate on a location-by-location basis or upon insertion of a substrate into the system or for covering the substrate when not being accessed or upon removal from the system (not shown). In some cases, fewer components or functionalities may be incorporated based on the functional requirements of the system. In some variations, additional liquid sample chambers may be included that can hold different liquids originating from different liquid sources (e.g., a single liquid source but pulled from different processing stages as the liquid undergoes treatment or modification), multiple spectrometers, and/or multiple dispensing systems, and even multiple substrates and handling stages so that parallel processing of multiple samples may occur. In some variations, data may be transferred from the system as illustrated to a separate system for data storage purposes or providing analysis of the detected emissions. In still other variations, alarms or lights or other operator signaling devices may be provided in the event of system errors or detection of dangerous materials or concentrations of materials. In some variations, the system may provide storage for full and unused CBDs along with automatic robotic handling, loading, unloading, and moving of CBDs. In some embodiments, the system may provide sample volumes of known concentration which can be loaded into the dispenser and deposited to provided calibrations samples. In other embodiments, the system may hold one or more CBHD that contain samples of known concentration which can be periodically and preferably automatically read and processed to verify existing calibration or to provide recalibration of the system.

Methods, apparatus, and sensor systems of various selected embodiments may make use of deep UV Raman or Resonance Raman (having excitation wavelengths below 280 and more preferably below 250 nm), deep UV fluorescence, or a combination of them. Other embodiments may use Raman, resonance Raman, fluorescence, Rayleigh scattering, and phosphorescence. In some embodiments excitation radiation may be provided at wavelengths ranging from 200 nm to 12 um. In some embodiments, samples containing analytes in solution or suspended in a liquid, may be automatically desiccated as a series of overlaying nanoliter droplets of the carrier liquid are dispensed onto a substrate for detection using above optical methods. Such methods will reduce or eliminate any background signal levels from the fluid medium, whether Rayleigh, Raman, fluorescence, or phosphorescence and enables low limits of detection of analytes in the ppm (parts per million range), ppt (the parts per trillion range), and potentially even in the ppq (parts per quadrillion) range.

In other embodiments, the substrate may provide a recording surface that includes many droplet locations, with single droplets deposited at each location or with a plurality of droplets stacked at each location. The depositions at the different locations may originate from different samples to enable collection of hundreds to thousands of spectroscopic data sets taken over periods of hours, days, and months, without intervention by an outside operator. The system or a user could replace the substrate with a new substrate so additional data can be accumulated without, or with minimal, user interaction.

Though a wide range of excitation wavelengths may be used in different embodiments of the invention, deep UV excitation radiation offers some advantages. There are eight main advantages of excitation below 250 nm compared to near-UV, visible or near-IR counterparts: (1) Fluorescence-free Raman and Raman-free fluorescence enabling enhanced detection and identification of target materials without mutual interference or obscuration; (2) resonance Raman signal enhancement for improved Raman sensitivity; (3) Rayleigh law signal enhancement due to shorter excitation wavelength; (4) simultaneous high levels of chemical and biological specificity over a wide range of chemical and biological materials; (5) simplification of Raman spectra due to resonance or pre-resonance enhancement, (6) ability to detect trace biological materials such as bacterial cells and spores, and viruses; (7) eye retina safety; and (8) solar blind detection enabling standoff operation in full daylight or artificial light.

An especially important advantage of deep UV operation below 250 nm is the ability to separate the spectral regions of Raman and fluorescence for over 95% of all materials. This is important since even low levels of fluorescence from the target material of other materials within the excitation volume of the laser can easily obscure the much weaker Raman emissions. It has been shown in many publications that fluorescence from even the simplest organic molecules emit fluorescence down to about 270 nm. As a result, to detect Raman emissions free from fluorescence, excitation needs to be below 250 nm or preferably a somewhat shorter wavelength. Too short a wavelength leads to self-absorption in a target material and matrix with major loss of signal strength. The Raman region occupies about 25 nm of wavelength above the excitation wavelength with excitation at 250 nm and would be obscured by fluorescence at longer excitation wavelengths, which can occur in many or most environments from a wide range of ubiquitous materials in nature. Many systems have been developed that employ diode-pumped solid-state lasers with harmonic generation into the UV emitting at 266 nm or 262 nm. Raman emissions using these excitation wavelengths lead to massive obscuration by fluorescence from any material within the excitation volume from the target or other materials.

When the emission bands of Raman and native fluorescence emissions from targeted analyte materials are separated into separated regions of the spectrum, the benefits of both types of emissions provide additional information about the targeted material. Raman and resonance Raman provides highly specific information about the presence of resonances with molecules, while native fluorescence provides information about the overall electronic configuration of a molecule, easily differentiating the basic form of an organic material. Native fluorescence cross-sections and signal strengths are typically many tens of thousands to millions of times larger and stronger than Raman, providing the opportunity to detect molecules at much lower concentration than Raman methods. Both provide complementary information about the targeted material. Fluorescence detection methods are important for detection of micropollutants, including microplastics and biological material due to the ability of fluorescence spectra to classify many of these materials and concentrations much lower than Raman in the ppt and ppq range and below. Fluorescence methods are often used as a precursor to Raman methods due to this much higher sensitivity.

Raman and resonance Raman spectroscopy provide the highest level of specificity or selectivity of any method since they typically measure many spectrographic features or Raman bands uniquely associated with each different molecule. Within a collection of many combined analytes, there are not only single Raman bands, but the collection of Raman bands from each molecule that can be differentiated to determine the identity and concentration of each analyte present. With the methods taught here, these capabilities are enabled for extremely low concentrations of material.

Whether hazardous or beneficial material, targeted materials can be detected directly in water/liquids, in air, or on surfaces or extracted from air or surfaces and into a water/liquid carrier prior to detection. In the case of air samples, the air is flowed through an air to liquid sampler where particulates and other substances in the air are transferred into a liquid. Surface contaminants are typically obtained by swabbing or wiping of a surface and vortexing or sonicating the swab or wipe to remove particles or substances into a liquid. Liquid samples come directly from in-line, on-line, at-line, off-line sampling of a liquid source under test. Whatever process is used, benefits arise from it being implemented in an automated and autonomous manner without the need for manual processing or intervention. Water or other liquids are often the transport medium for measurements.

Testing of the liquid samples can be done with any of a range of instrument types. Often, these tests utilize flow cells, liquid chromatography, mass-spectrographs, or some combination thereof. Other instruments include Raman, fluorescence, or UV/Visible spectrometers employed at a wide range of operating wavelengths from the deep UV though the near UV, visible, and IR. Raman instruments operated in the near UV, visible, and near IR are typically not employed in wastewater treatment applications due to fluorescence emissions from the many organics typically contained with wastewater, which obscure the Raman emissions.

Limits of detection (LOD) of the target substances contained in the liquid are typically limited by the signal level of the target material of interest compared to the signal level of background or matrix materials within the excitation or interrogation volume of the detection system in the liquid. Optical noise of the measurement also limits the LOD including shot noise from the background, read noise, and electronics noise. But frequently the largest factor in limiting the achievable LOD is signal levels from the transport liquid itself that is carrying the materials of interest.

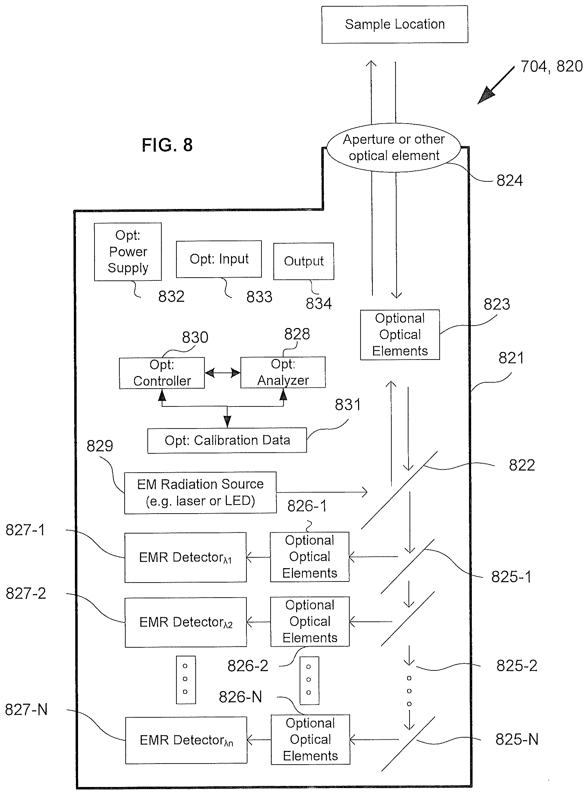

FIG. 8 provides a schematic representation of a spectroscopic instrument that may be used in some embodiments of the invention. In some embodiments, the instrument may include optical elements that provide the device with an elongated sampling appendage so that a sampling orifice, window, or lens may reach into confined spaces as well as providing access to large flat areas. In some variations the device may include a pistol-like handle and one or more trigger-like interface elements, other input elements, a conveniently located display screen or other output device so that it may be used has a handheld device as well as a system mounted device.

The device 820 (referenced as 704 in FIG. 7) includes a housing 821 that can hold a variety of optical elements and electronic elements such as: (1) an optional power supply 832; (2) an optional input device 833, such as a trigger, a key pad, a touch screen, switches, capacitive or inductive elements, wireless connections or wired connections to a separate device with manual or programmed input capability, or the like; (3) an output device 834, such as a visual screen, an auditory speaker or alarm, a wireless connection or wired connection to a separate device with output functionality, a vibrator or other tactile element, (4) an optional controller 830 such as an ASIC, a microprocessor including memory elements and hard coded or software implemented fixed or selectable functionality including for example sensing, calibration, analysis, uploading, downloading and other functional routines; (5) a source 829 of excitation radiation 829 such as a hollow cathode metal ion laser (such as those set forth in US Patent 6,693,944), an LED, or an LET, or a semiconductor laser or the like (such as those set forth in US Patent No. 7,590,161) or other source that provides the required excitation energy whether it be IR, visible, or UV; (6) an optional analyzer 828 which may be part of the controller or a separate component or module that performs or aids in determining what substances have or have not been detected and may be used to implement built in chemometric algorithms and/or trained machine learning (ML) or artificial intelligence (Al) algorithms to provide a wide range of detection capability or focused detection for specific applications where the analyzer may have access to stored analyte spectral data or be able to access such data from a remove source, to search for such data if the spectral analysis requires it, or even to download spectral data and other relevant data to a remote computer system for more intensive analysis; (7) one or more optical elements 822 for directing excitation radiation onto the sample location and for passing emission radiation, which may include, for example, filters, splitters and the like (in some variations of this embodiment these elements are optional); (8) one or more optional optical elements 823 for shaping the excitation radiation prior to reaching the sample location or for providing a fixed size aperture for sealed window 824 of the housing; (9) one or more optical elements 825-1, 825-2, ..., and 825-N (such as, for example dichroic filters, diffraction gratings, prisms, or the like) for receiving emission radiation and for directing it along different optical paths for detection by different detector elements; (10) one or more optional optical elements 826-1, 826-2, ..., and 826-N for filtering and/or shaping emission radiation (e.g. bandpass filters, focusing lenses, and the like) that is being directed along each optical path for each separate spectral detection band or channel; (11) one or more detectors 827-1, 827-2,..., and 827-N (e.g. photodiodes, photomultiplier tubes (PMT), CCD, CMOS, and combinations of such detectors, and arrays of such detectors) for detecting the quantity of emission radiation present within each of the separate spectral detection bands; and (12) optional stored calibration data 831 or ability to look up such calibration data from a remote source that can be used in a process to convert raw data associated with a given material to a calibrated value such as an amount of material per unit area based on an original known sampling area and quantity of liquid used in obtaining the sample, a concentration that was present in a known volume of liquid that originally held the sample or volume or air from which the sample was extracted (e.g. micrograms, nanograms, picograms, or femtograms per unit area or volume, or alternatively PPMs, PPBs, PPTs, or even PPQ). In some embodiments, the number of detectors and associated filters and lens may be less than three (e.g., 1 or 2), while in other embodiments they may number slightly or significantly more than three (e.g., 10 or more). In some alternative embodiments, one or more secondary sources of excitation radiation may be included in the device. In some alternative devices, the device size may be larger or smaller than that of the most preferred embodiments, may be heavier or lighter than that of the most preferred embodiments, or may use power at a lower rate or higher rate than that of the most preferred embodiments. For example, in some embodiments, the device may have a volume that is up to ½ liters, 1 liter, 2 liters, or even 4 liters or more, and may have a weight up to one pound, up to two pounds, up to five pounds, more than ten pounds, or even more than 20 pounds. In some embodiments, the instrument may take the shape of a badge sensor that is mobile phone sized with a 300 g weight. The device may operate on different power sources including, for example, rechargeable batteries or line power. In some embodiments the device may, for example, use a source operating at a wavelength less than 280 nm may be preferred or even less than 250 nm though in other embodiments other sources may be used. Sources having shorter wavelengths may be preferred in some embodiments while longer radiation sources may be preferred in other embodiments.

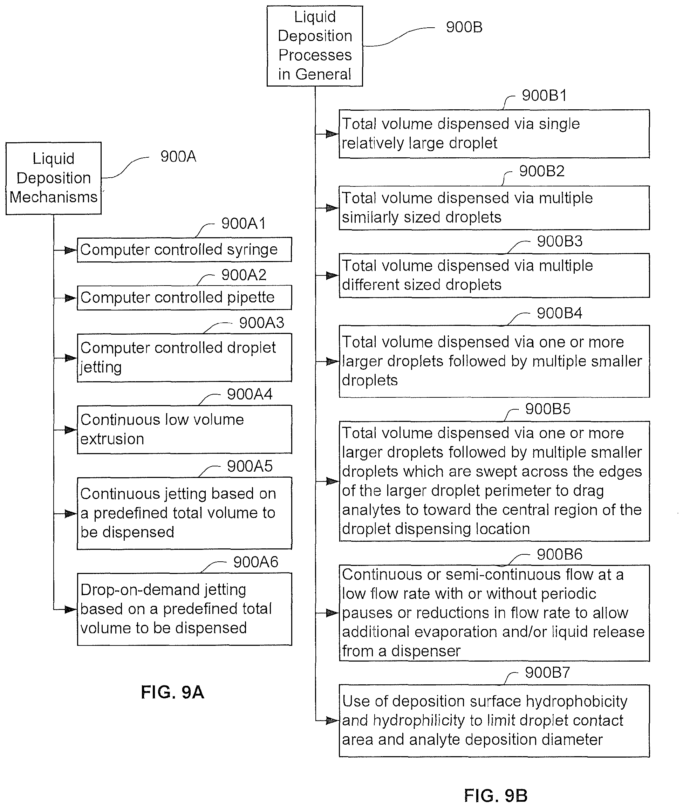

FIG. 9A provides a listing of example deposition devices 900A that may be used in the system of FIG. 7, including: (a) a computer controlled syringe 900A1, (b) a computer controlled micropipette 900A2, (c) a computer controlled droplet jetting device such as piezo electric dispenser 900A3, (d) a continuous low volume extrusion dispenser 900A4, (e) continuous jetting based on a predefined total volume to be dispensed 900A5, and (f) drop-on-demand jetting based on a predefined total volume to be dispensed via a plurality of drops 900A6.

FIG. 9B provides a listing of example deposition methods that may be implemented in different embodiments of the system of FIG. 7: (a) total volume dispensed via single relatively large droplet 900B1, (b) total volume dispensed via multiple similarly sized droplets 900B2, (c) total volume dispensed via multiple different sized droplets 900B3, (d) total volume dispensed via one or more larger droplets followed by multiple smaller droplets 900B4, (e) total volume dispensed via one or more larger droplets followed by multiple smaller droplets which are swept across the edges of the larger droplet perimeter to drag analytes to toward the central region of the droplet dispensing location 900B5, (f) continuous or semi-continuous flow at a low flow rate with or without periodic pauses or reductions in flow rate to allow additional evaporation and/or liquid release from a dispenser 900B6, and (g) use of deposition surface hydrophobicity and hydrophilicity to limit droplet contact area and analyte deposition diameter 900B7.

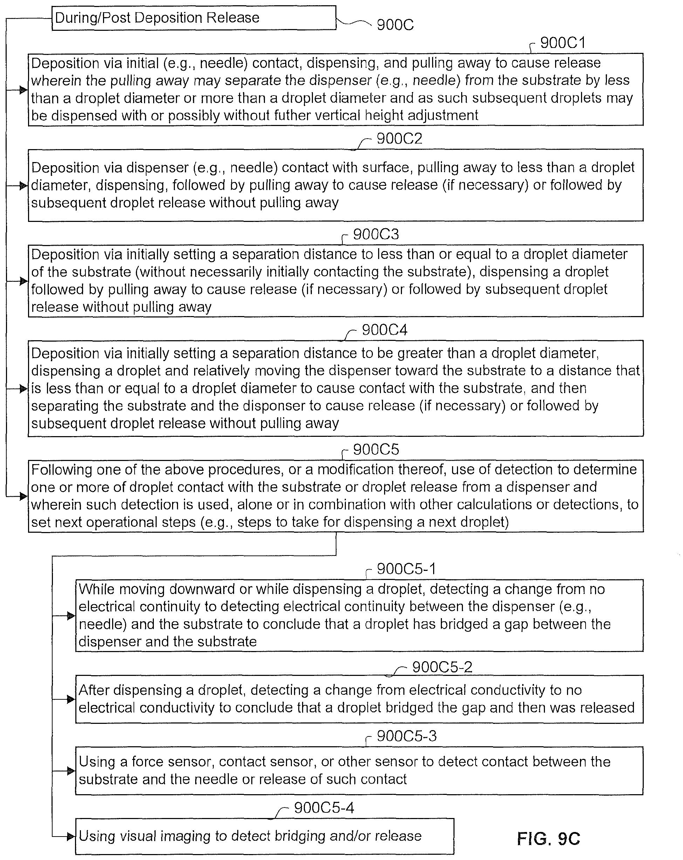

FIG. 9C provides a listing of example detailed deposition procedures that may be implemented in some variations of the system of FIG. 7: (a) deposition via initial (e.g., needle) contact, dispensing, and pulling away to cause release wherein the pulling away may separate the dispenser (e.g., needle) from the substrate by less than a droplet diameter or more than a droplet diameter and as such subsequent droplets may be dispensed with or possibly without further vertical height adjustment 900C1, (b) deposition via dispenser (e.g., needle) contact with surface, pulling away to less than a droplet diameter, dispensing, followed by pulling away to cause release (if necessary) or followed by subsequent droplet release without pulling away 900C2, (c) deposition via initially setting a separation distance to less than or equal to a droplet diameter of the substrate (without necessarily initially contacting the substrate), dispensing a droplet followed by pulling away to cause release (if necessary) or followed by subsequent droplet release without pulling away 900C3, (d) deposition via initially setting a separation distance to be greater than a droplet diameter, dispensing a droplet and relatively moving the dispenser toward the substrate to a distance that is less than or equal to a droplet diameter to cause contact with the substrate, and then separating the substrate and the dispenser to cause release (if necessary) or followed by subsequent droplet release without pulling away 900C4, (e) following one of the above procedures, or a modification thereof, use of detection to determine one or more of droplet contact with the substrate or droplet release from a dispenser and wherein such detection is used, alone or in combination with other calculations or detections, to set next operational steps (e.g., steps to take for dispensing a next droplet) 900C5, (e1) while moving downward or while dispensing a droplet, detecting a change from no electrical continuity to detecting electrical continuity between the dispenser (e.g., needle) and the substrate to conclude that a droplet has bridged a gap between the dispenser and the substrate 900C5-1, (e2) after dispensing a droplet, detecting a change from electrical conductivity to no electrical conductivity to conclude that a droplet bridged the gap and then was released 900C5-2, (e3) using a force sensor, contact sensor, or other sensor to detect contact between the substrate and the needle or release of such contact 900C5-3, and (e4) using visual imaging to detect bridging and/or release 900C5-4.

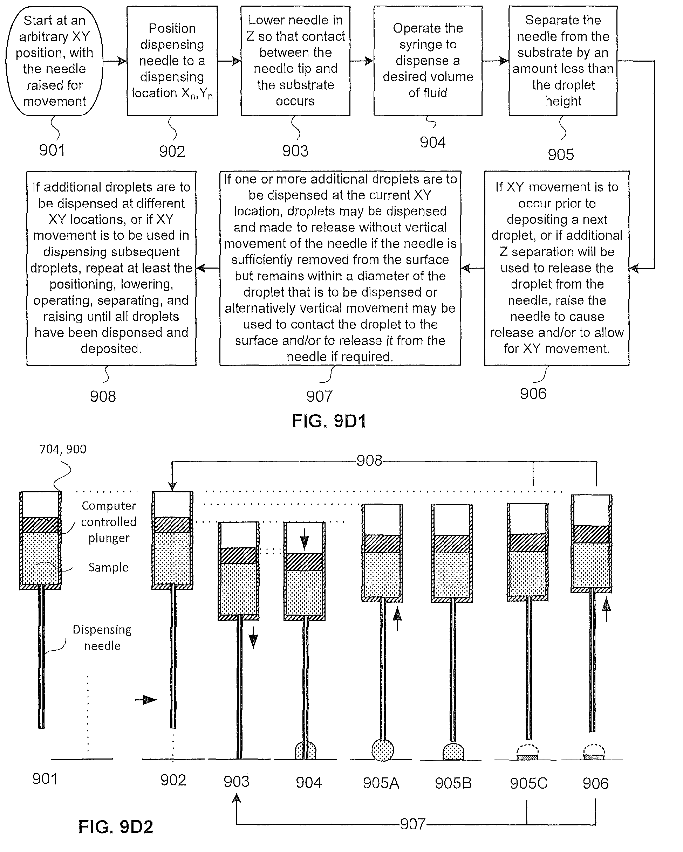

FIGS. 9D-1 and 9D-2 to 9G-1 to 9G-2 provide both block diagrams and schematic representations of four example processes for controllably dispensing known volumes of material in the form of individual droplets from a computer-controlled syringe 900 (FIG. 9D2) or 704 (of FIG. 7).

FIG. 9D1 provides a flowchart of a first droplet dispensing process, while FIG. 9D2 provides a schematic illustration of a device performing that droplet dispensing process, wherein at the time of syringe operation (to dispense a given volume as a droplet), a needle tip of the syringe is in contact with a dispensing substrate, and after dispensing, the tip of the needle is raised to a height above the substrate that may be less than or greater than a height of an unreleased droplet, The process then continues to deposit additional droplets as necessary with or without additional dispenser movement in the X, Y, or Z directions. If the dispenser can dispense and release the droplet without being raised from the substrate beyond the droplet diameter, dispensing of additional droplets can be laid over previous droplets without relative movement of the deposition device and the substrate.

The process of FIGS. 9D-1 and 9D-2 is shown in eight Blocks 901 - 908 with the block operations and the corresponding schematic depictions provided with the same reference numbers. The process starts with Block 901 and with the computer-controlled syringe at an arbitrary XY position with the tip of the needle located sufficiently above a substrate to allow XY movement without risk of collision. The process then calls for the syringe to move to an XₙYₙ dispensing location (i.e., a needle location for dispensing droplets), as set forth in Block 902. Next, the tip of the syringe is lowered in the Z-direction until contact is made with the substrate (detection can occur in a number of different ways) as set forth in Block 903. The syringe is operated to dispense a precisely known volume of material with the needle touching the substrate such that the amount of material is released but remains joined to the substrate and the needle as set forth in Block 904. After the dispensing operation, the syringe is lifted in the Z-direction to a height above the substrate that is less than a droplet height as set forth in Block 905, with a hope of releasing the droplet immediately or after some evaporation has occurred. The attached droplet is stripped from the needle (by a preference to cling to the substrate) and remains in contact with the substrate, whereafter solvent holding the material evaporates, leaving behind a dried deposit of material of an unknown amount, but from a known volume. The process then moves to Block 906, which calls for raising the needle if XY movement is to occur (to remove any danger of collision), or if necessary to release the droplet from the needle. Next, the process moves to block 907, which indicates that if one or more additional droplets are to be dispensed at the current XY location, droplets may be dispensed and made to release without vertical movement, so long as the needle tip is sufficiently close to the surface to allow contact between the hanging volume and the surface, but sufficiently removed to cause separation. Otherwise, vertical movement may be used to cause contact and/or release. Next, the process moves to Block 908, which provides for additional droplets to be dispensed at different XY locations, which repeats the prior operations, as necessary. FIG. 9D2 illustrates the X, Y, and Z movements of the nozzle along with droplet dispensing and release according to the operations of Blocks 901 - 906, but with the release of Block 905 broken into three illustrations, in which 905A shows initial separation less than a droplet height, 905B shows the possibility of the droplet self-releasing, and 905C shows the analyte remaining after evaporation of the droplet has occurred. If the release of 905B occurs, the operation(s) of Block 906 may be eliminated.

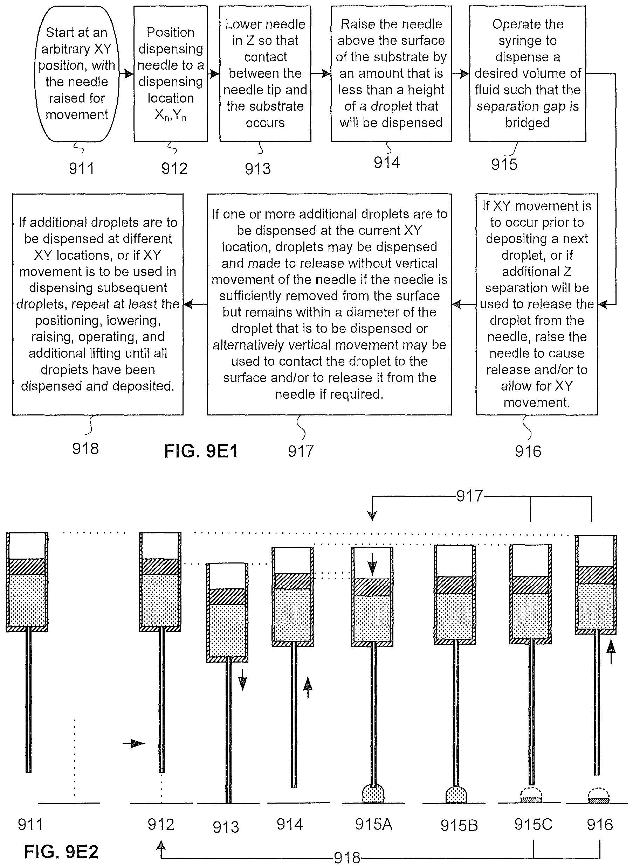

FIG. 9E1 provides a flowchart of a second droplet dispensing process, while FIG. 9E2 provides a schematic illustration of a device performing the droplet dispensing process, wherein at the time of syringe operation (to dispense a given volume as a droplet), a tip of the dispensing needle of the syringe is above a substrate surface by an amount smaller than an unreleased droplet height, where the distance is set by first touching the substrate with the needle (prior to dispensing). Then, the needle is retracted a desired amount to a height above the substrate that is less than a height of an unreleased droplet once dispensed. After, release of a droplet will be made to occur with or without additional Z-movement, which is followed by dispensing of additional droplets, as necessary, with or without additional relative dispenser movement in the X, Y, or Z directions.

The process of FIG. 9E1 and 9E-2 is shown in eight Blocks 911 - 918 with the block operations and the corresponding schematic depictions provided with the same reference numbers. The process starts with Block 911 and with the computer-controlled syringe at an arbitrary XY position with the tip of the needle located sufficiently above a substrate to allow XY movement without risk of collision. The process then, in Block 912, calls for the syringe to move to an XₙYₙ dispensing location (i.e., a needle location for dispensing a droplet). Next, the tip of the syringe is lowered in the Z direction until contact is made with the substrate (detection can occur in different ways) as set forth in Block 913. The syringe is then lifted in the Z direction from the substrate by an amount less than the height of a droplet that will be dispensed as set forth in Block 914. The syringe is operated to dispense a precisely known volume of material such that a droplet while being dispensed, and still clinging to the needle, makes contact with the substrate which may or may not result in the release of the material from the needle as set forth in Block 915. The process then moves to Block 916, which calls for raising the needle if XY movement is to occur (to remove any danger of collision) or if necessary to release the droplet from the needle. Next, the process moves to block 917 which indicates that if one or more additional droplets are to be dispensed at the current XY location, droplets may be dispensed and made to release without vertical movement, so long as the needle tip is sufficiently close to the surface to allow contact between the hanging volume and the surface, but sufficiently removed to cause separation otherwise vertical movement may be used to cause contact and/or release. Next, the process moves to Block 918, which provides for additional droplets to be dispensed at different XY locations, which repeats the prior operations, as necessary. FIG. 9E2 illustrates the X, Y, and Z movements of the nozzle along with droplet dispensing and release according to the operations of Blocks 911 - 918, but with the dispensing of Block 915 and possible release of the droplet broken into three illustrations, in which 915A shows initial separation less than a droplet height along with dispensing of the droplet, 915B shows the possibility of the droplet self-releasing, and 915C shows the analyte remaining after evaporation of the droplet has occurred. If the release of 915B occurs, the operation(s) of Block 916 may be eliminated.

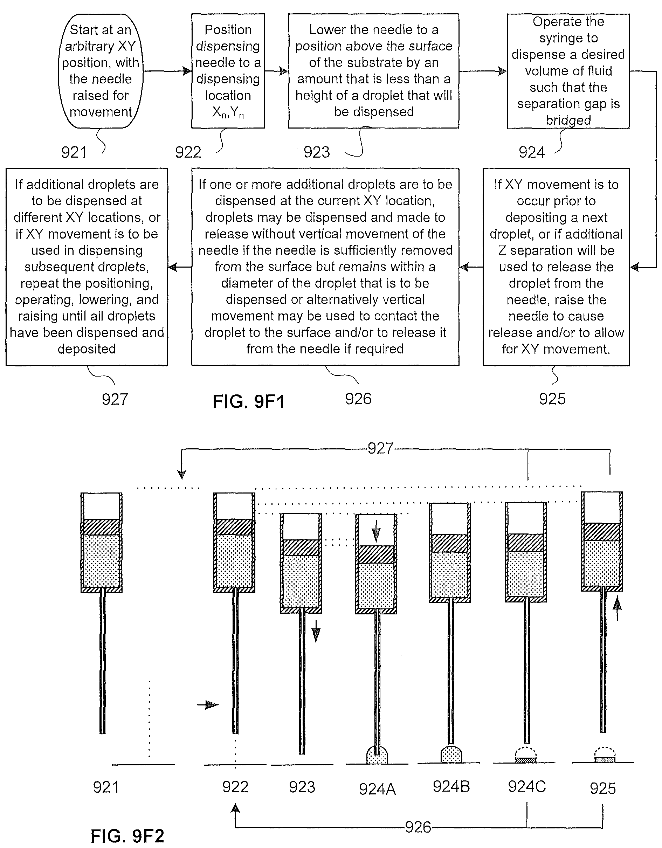

FIG. 9F1 provides a flowchart of a third droplet dispensing process, while FIG. 9F2 provides a schematic illustration of a device performing the droplet dispensing process, wherein at the time of dispensing a given volume (as a droplet) from a tip of a syringe dispensing needle. The tip is above a substrate surface by an amount smaller than a droplet height, where the distance is set by moving the needle downward and stopping movement prior to the needle making contact with the substrate. After dispensing the droplet, it may or may not be released without upward movement of the dispenser in the Z-direction, which is followed by dispensing of additional droplets as necessary with or without additional relative dispenser movement in the X, Y, or Z directions.

The process of FIG. 9F1 and 9F-2 is shown in seven Blocks 921 - 927 with the block operations and the corresponding schematic depictions provided with the same reference numbers. The process starts with Block 921 and with the computer-controlled syringe at an arbitrary XY position with the tip of the needle located sufficiently above a substrate to allow XY movement without risk of collision. The process then calls for the syringe to move to an XₙYₙ dispensing location (i.e., a needle location for dispensing a droplet) as set forth in Block 922. Next, the tip of the syringe is lowered in the Z-direction until it is located above the surface of the substrate, but closer to the substrate than a height of a droplet that will be dispensed as set forth in Block 923. The syringe is operated to dispense a precisely known volume of material with the needle located above the substrate within the height of the droplet that is being dispensed such that it bridges the gap between the needle tip and the substrate, as set forth in Block 924. The process then moves to Block 925 which calls for raising the needle if XY movement is to occur (to remove any danger of collision) or if necessary to release the droplet from the needle. Next, the process moves to block 926 which indicates that if one or more additional droplets are to be dispensed at the current XY location, droplets may be dispensed and made to release without vertical movement so long as the needle tip is sufficiently close to the surface to allow contact between the hanging volume and the surface, but sufficiently removed to cause separation otherwise vertical movement may be used to cause contact and/or release. Next, the process moves to Block 927, which provides for additional droplets to be dispensed at different XY locations, which repeats the prior operations, as necessary. FIG. 9F2 illustrates the X, Y, and Z movements of the nozzle along with droplet dispensing and release according to the operations of Blocks 921 - 927. The release of Block 924 is broken into three illustrations, in which 924A shows initial separation less than a droplet height and the dispensing of the droplet, 924B shows the possibility of the droplet self-releasing, and 924C shows the analyte remaining after evaporation of the droplet has occurred. If the release of 924B occurs, the operation(s) of Block 925 may be eliminated.

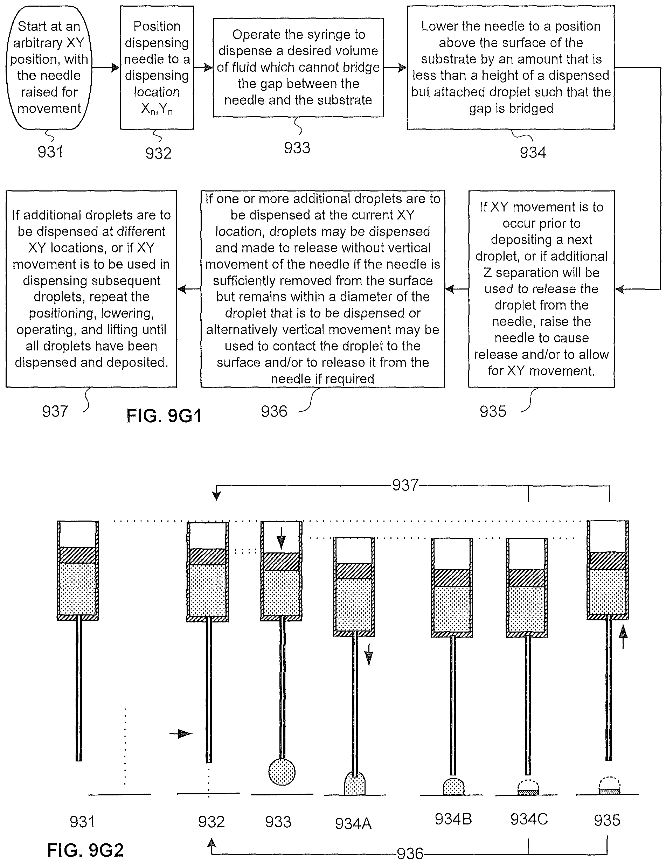

FIG. 9G1 provides a flowchart of a fourth droplet dispensing process, while FIG. 9G2 provides a schematic illustration of a device performing the droplet dispensing process, wherein at the time of dispensing a known droplet volume from a tip of a syringe dispensing needle, the tip is above a substrate surface by an amount greater than an unreleased droplet height, and where after dispensing, the needle tip is lowered to a position above the substrate less than a height of the unreleased droplet. This will cause contact of the droplet to the substrate, which may or may not cause release of the droplet without subsequent movement of the dispenser in the Z-direction. This is followed by dispensing of additional droplets as necessary with or without additional relative dispenser movement in X, Y, or Z directions.

The process of FIG. 9G1 and 9G2 is shown in seven Blocks 931 - 937 with the block operations and the corresponding schematic depictions provided with the same reference numbers. The process starts with Block 931 and with the computer-controlled syringe at an arbitrary XY position with the tip of the needle located sufficiently above a substrate to allow XY movement without risk of collision. The process then calls for the syringe to move to an XₙYₙ dispensing location (i.e., a needle location for dispensing a droplet) as set forth in Block 932. The syringe is operated to dispense a precisely known volume of material with the needle located above the substrate by an amount greater than a height of the dispensed but unreleased droplet which cannot reach the substrate without dropping from the needle as set forth in Block 933. Next, the needle is lowered to within the height of an unreleased droplet from the substrate, which causes the droplet to bridge the gap between the needle and the substrate, and may or may not result in the release of the droplet from the needle as set forth in Block 934. The process then moves to Block 935, which calls for raising the needle if XY movement is to occur (to remove any danger of collision) or if necessary to release the droplet from the needle. Next, the process moves to Block 936, which indicates that if one or more additional droplets are to be dispensed at the current XY location, droplets may be dispensed and made to release without vertical movement so long as the needle tip is sufficiently close to the surface to allow contact between the hanging volume and the surface, but sufficiently removed to cause separation. Otherwise, vertical movement may be used to cause contact and/or release. Next, the process moves to Block 937, which provides for additional droplets to be dispensed at different XY locations which repeats the prior operations, as necessary. FIG. 9G2 illustrates the X, Y, and Z movements of the nozzle along with droplet dispensing and release according to the operations of Blocks 931 - 937, but with the release of Block 934 broken into three illustrations. In these illustrations, 934A shows initial separation less than a droplet height, 934B shows the possibility of the droplet self-releasing, and 934C shows the analyte remaining after evaporation of the droplet has occurred. If the release of 934B occurs, the operation(s) of Block 935 may be eliminated.

FIGS. 9H1 to 9L3 illustrate four hypothetical droplets of different sizes and resulting analyte deposits that might occur from single deposits and from multiple deposits including deposit combinations resulting from different sized droplets.

FIG. 9H1 provides two hypothetical images of a relatively large droplet with an upper image providing a sideview of the droplet before impinging on a substrate and the lower image providing a side view of the droplet after impacting the substrate and spreading out before the liquid of the droplet evaporates.

FIG. 9H2 shows two hypothetical images of the deposited droplet of FIG. 9H1 with an upper image providing a top view of the analyte forming a ring-like shape after the liquid from the droplet has evaporated and with a lower image providing a central cross-sectional view of the analyte along the cut line shown in the upper image.

FIG. 9H3 provides a hypothetical image of a cross-sectional view similar to that of the lower image of FIG. 9H2 but after two droplets have been deposited and the liquid evaporates such that the analyte deposit has thickened.

FIGS 9I1 - 9I3, 9J1 - 9J3, and 9K1 - 9K3 provide similar hypothetical images to those of FIGS 9H1 - 9H3, respectively, but where the droplets are smaller with each successive group of figures and where the images of FIGS 9I3, 9J3, and 9K3 are, respectively formed from 4 droplets, 8 droplets, and 16 droplets to roughly approximate the analyte deposition height of FIG. 9H3.

FIG. 9L provides a hypothetical view of an analyte deposition that might result if the depositions of each of FIG. 9H3, 9I3, 9J3, and 9K3 are targeted at the same location to provide an even more concentrated amount of deposited analyte.

FIGS. 9M1 - 9M4 provide hypothetical top views and cut views of a single droplet for FIG. 9M1 to five droplets for FIG. 9M4 where successive droplets are dispensed at slightly offset positions (for FIGS. 9M2 to 9M4) to illustrate another example of analyte stacking.

FIGS. 9N1 - 9N3 provides another example of analyte stacking but also provides an example of how lateral analyte concentration may be made to occur wherein FIGS. 9N1 provides a top view and cross-sectional view of relative large droplet that has evaporated to leave a ring-like deposition of analyte material while FIG. 9N2 provide16 rotated top views of a smaller liquid droplet that is swept over part of the analyte from the larger droplet to pull it toward the center where the smaller droplet is dropped and dried, whereby it is believed that the sweeping of the smaller droplets can pull the analyte material of the larger droplet inward toward the center to provide a smaller deposition of enhanced concentration as can be seen in the top and cross-sectional view of the FIG. 9N3 when compared to the original distribution of the analyte of FIG. 9N1.

FIG. 10A provides a schematic illustration of a multi-source modular liquid handling system 1000A that can be used in the system of FIG. 7 that can supply 1 to N liquids to 1 to N different reservoirs with each reservoir accessible by one or more dispensing systems. The liquid handling system includes multiple independent modules with each providing a reservoir, one or more optional filters, one or more optional pumps (one is shown on the inlet line), a drain, an outlet (separate from the drain), a flushing source, and control valves and pump controls that can be used to control fluid flow and thus functionality achieved by each module whether it be for filling the reservoir with a continuous or discontinuous flow of fresh liquid to be tested, or purging the system of contaminates and particulates.

As illustrated the system includes a first module 1007A-1 for handling liquid from a first source 1041A-1, a second module 1007A-2 for handling liquid from a second source 1041A-2, and an Nth module 1007A-N for handling liquid from an Nth source 1041A-N. Each of these modules includes the components noted above with a connection configuration as illustrated in FIG. 10A. Each module in the present embodiment includes four values which ensure that liquid flow is limited to appropriate paths. The valves shown in the example of FIG 10A include: (1) a flush line valve for each module (VF1, VF2, and VFN) that controls flow of flushing liquid into one or more of the a drain line, an inlet line and an outlet line; (2) a reservoir value (VR1, VR2, and VRN) that controls flow of liquid to or from the reservoir and thus to or from the inlet line; (3) a drain line value (VD1, VD2, and VDN) which controls flow into the drain line; and (4) an outlet line valve which may which control the flow of fluid into the outlet. Depending on which valves are opened, which pumps are operating, and the flow direction enforced by those pumps, different fluid flows are possible. The flows may be generally of a continuous nature, or may be of a discontinuous nature. Periodically, a normal or operation flow may be reversed to allow for filter purging, reservoir flushing, and the like.

For example, in normal operation, the reservoir value may be open along with the outlet value, while the other values are closed. The pump may (i.e., Pump 1, Pump 2, or Pump N) may be on moving liquid from the process chamber to the reservoir to provide a continuous refreshed stream of liquid that can be accessed by a dispenser for testing purposes with excess liquid being returned to the process chamber via the open outlet value.

In an alternative approach the reservoir valve may be opened along with the drain valve while the other values are closed. The pump may be supplying process chamber liquid to the reservoir and excess liquid out the drain.

In another functional mode, the flush valve and the reservoir value may be open (with or without the outlet value and drain valve being open) to allow the flushing fluid to backflow through the reservoir pump and inlet filter to clean out any buildup of particulates in the inlet filter. In another example the inlet and outlet values may be open with the other values closed and with the reservoir pump may be operating in the reverse direction to clear out the outlet filter. Though 3 modules are specifically illustrated in this example, additional modules may be added, or fewer modules may be included depending on how the handling system will be used.

FIG. 10B provides a simplified module 1007B with its outlet line and associated components removed in favor of using the drain for handling all excess liquid. The remaining components are similar to those set forth in FIG. 10A with functionalities remaining the same, mutatis mutandis. The module of FIG. 10B may be used in place of any of the modules of FIG. 10A.

FIG. 10C illustrates another example of a multi-source liquid handling system that uses the simplified module of FIG. 10B as a starting point and a single reservoir for holding liquid from different sources at different times. Valves are included in this system not only to allow the functionalities noted in FIG. 10A, but also to control which of the multiple process or storage chambers are feeding material into the single reservoir of this system at any given time. In addition to incorporation of a reservoir valve V2, a flush value V1, and a first drain valve V3, a second drain valve is provided that allows for purging of inlet line fluid as well as inlet storage valves (e.g., VS1, VS2 and VSN) for each of the storage process chambers 1041C-1, 1041C-2, and 1041C-N so that independent access to the contents of each chamber may be provided to the reservoir, loaded into a dispenser, deposited, and analyzed. Valve control functions are similar to those noted regarding FIG. 10A, mutatis mutandis.

Numerous variations to the fluid handling systems of FIGS. 10A - 10C are possible. In some such variations filters and/or pumps may be located in other positions relative to other system components or even outside a system housing. Additional pumps or fewer pumps may be used. Pumps and/ filters may be located in proximity to primary sources of the liquid such as processes chambers, natural or manmade fluid systems such as lakes, tanks, or other storage systems, rivers, channels, pipes, or other fluid transfer systems. The pumps may be used to pull liquid from a source and/or be used to push liquid to a reservoir that is accessible by a dispensing system. Since pulling a liquid may have suffer limitations associated with vaporization or other vacuum limits taking such effects into consideration when locating one or more or more pumps may be important. Pumps may also be used to pull liquid from the reservoir that is accessible by a dispensing system. In other embodiments, fluid flow may occur with or without pumping via gravity or some other motivating force.

FIGS. 10D1 - 10D4-2 illustrate five different example reservoirs that might be used in variations of the embodiments of FIGS. 10A - 10C or other embodiments requiring reservoirs. FIG. 10D1 provides a simple reservoir with an open top, and inlet and an outlet where a dispensing device (e.g., a needle of a micropipette or syringe) can access the reservoir from above to draw out liquid for deposition and testing. FIG. 10D2 provides a similar reservoir but with a weir to provide a controlled fluid height. FIG. 10D3 provides a reservoir with two primary differences: (1) the reservoir is capped by a septum through which a dispensing needle must penetrate to access the liquid within the reservoir, and (2) the inclusion of a stirring device (e.g., a magnetic stirrer) near the bottom of the reservoir. The dispensing needle used with the reservoir of FIG. 10D3 may penetrate the septum directly, or it may be inserted into a larger diameter non-coring needle to allow penetration and filling with liquid. Thereafter, the dispensing needle can be extracted from the non-coring needle prior to being used to dispense material that is to be tested. In some configurations, the dispensing needle may have a non-coring configuration. FIG. 10D4-1 and 10D4-2 illustrate a reservoir with a lid that can be closed and opened as necessary to allow dispenser access.

FIG. 10E provides a different type of reservoir and fluid transfer system that is also capable of handling liquids from multiple sources. In this embodiment, multiple process chambers are provided along with multiple reservoirs where material may be extracted from the chambers by scoping and deposited into the reservoirs by pouring (shown), large scale pipetting (not shown), vertical movement of the reservoir to allow access to a below surface chamber valve (not shown), or the like. The chamber access area and the reservoir may be located above a drain trap so that any spilled or excess material may be automatically removed (as shown). Once material is extracted from the reservoir the reservoir may be emptied by tilting (as shown) or by opening a valve at the bottom of the reservoir (not shown), or the like.

FIG. 10F illustrates the relationship between a dispensing system and multiple reservoirs that can be accessed by the dispensing system as may be implemented in system such as that of FIG. 7. As illustrated, the dispenser in this example includes a computer-controlled plunger that can push liquid out of a dispensing reservoir through a microneedle such that very small, controlled volumes of material can be dispensed. The dispensing system is held by a computer-controlled vertical stage, which in turn is held by a computer controlled horizontal stage that can move the needle of the dispenser to horizontal and vertical positions to access liquids whether they be for testing, cleaning, calibrating, or other functions and to access substrates for dispensing. Other hardware and functional components may be included such as ultrasonic vibrators, magnet stirrers, heaters, radiation sources, shielding, ozone producers, mounting structures, reservoir release or emptying mechanisms (such as valves tilting systems, directable rinsing hoses and nozzles), reservoir covers along with mechanical or computer-controlled opening or access mechanisms (e.g., those that provide sliding, tilting, rotating, or puncturing).

FIGS. 11A1 - 11A2 provide a top view and a side cross-sectional view, respectively, of a first example of a chemical biological disk (CBD) 1171A having a deposition surface 1171-S that may be used in some embodiments of the invention.

FIGS. 11B1 - 11B2 provide a top view and a side cross-sectional view, respectively, of another example of a CBD 1171B that includes a top surface 1171B-S onto which depositions of material can be made wherein the CBD also include registration notches and surfaces 1171B-R1, 1171B-R2, and 1171B-R3 for engaging a mounting structure that provides for upright loading into the system as well as proper rotational loading or alignment.

FIGS. 11C1 - 11C2 provide a top view and a side cross-sectional view, respectively, of another example of a CBD 1171C that may be used in some embodiments of the invention where alignment or registration features 1171C-R1, 1171C-R2, and 1171C-R3 are marked on a deposition surface 1171C-S when the disk is first loaded or formatted for use.

FIGS. 11D1 - 11D2 provide a top view and a side cross-sectional view, respectively, of another example CBD 1171D that includes registrations marks 1171D-R1, 1171D-R2, 1171D-R3, and 1171D-R4, as well as deposition location areas 1171D-S that take the form of indentations into which deposits can be made and analytes retained.

FIGS. 11E1 - 11E2 provide a top view and a side cross-sectional view, respectively, of a further example CBD 1171E that includes registrations marks 1171E-R1, 1171E-R2, and 1171E-R3 but where the substrate is formed of a hydrophilic material that defines deposition areas 1171E-S that are surrounded by a coating of a hydrophobic material that defines non-deposition areas 1171-B where the hydrophobicity of the non-deposition areas may help to ensure preferential placement of droplets and associated analyte deposits.

FIGS. 11F1 - 11F2 provide a top view and a side cross-sectional view, respectively, of a CBD 1171F that includes registrations marks 1171F-R1, 1171F-R2, and 1171F-R3 and where the substrate is hydrophobic that but has deposition locations 1171E-S that are provided with a hydrophilic or less hydrophobic coating wherein the more hydrophobic regions define non-deposition areas 1171F-B.

FIGS. 11G1 - 11G2 provide a top view and a side cross-sectional view, respectively, of a CBD 1171G that includes registrations marks 1171G-R1, 1171G-R2, and 1171G-R3 and where the substrate is hydrophilic and has etched regions that receive hydrophobic material such that the hydrophilic (or less hydrophobic) regions define deposition areas 1171G-S and the hydrophobic regions define non-deposition regions 1171G-B.

FIGS. 11H1 - 11H2 provide a top view and a side cross-sectional view, respectively, of a CBD 1171H that includes registrations marks 1171H-R1, 1171H-R2, and 1171H-R3, wherein the substrate is hydrophobic and has selected regions that are etched that receive a hydrophilic material such that the hydrophilic regions provide preferential regions for deposition while the hydrophobic regions define regions that are less preferential for deposition of droplets and analyte material. Alternatively, the selected regions may be regions where structured plasmonic features will be formed on the surface to produce signal enhancement.

FIG. 11I provides a top view and a side cross-sectional view of a CBD 11711 that includes a protective lip 1171I-L around its perimeter to help ensure that deposits are not damaged by inadvertent scrapping of the surface, wherein the CBD is otherwise similar to that of the CBD of FIGS. 11H1 and 11H2 with the exception of registration marks not being shown and some of the deposition locations having been eliminated in favor of the protective lip. In some alternative embodiments, the perimeter wall or lip may extend to interior regions of the substrate.

FIG. 11J provides a top view and a side cross-sectional view of a CBD 1171J that includes not only a raised protective perimeter 1171J-L like that of FIG. 11I, but where each deposition location has its own elevated wall 1171J-W that protects it during formation of neighboring deposits and against inadvertent contact. In alternative embodiments, the walls, for example, may border the deposition areas while in other embodiments the walls may be set back somewhat from the deposition regions.

FIGS. 11K provides a top view and a side cross-sectional view of a CBD 1171K with a simple cover or lid 1171K-C that protects the deposition surface from contamination when not in use.

FIGS. 11L provides a top view and a side cross-sectional view of another example the CBD 1171L that is protected not only by an upper lid 1171L-C1, but also by a lower base structure 1171L-C2 and side walls, wherein the lid and possibly the base can be removed, displaced, or separated when it is necessary to access to the CBD surface when necessary.

FIGS. 11M1 - 11M3 provide side cross-section views of three additional example CBDs 1171M1, 1171M2, 1171M3, which include different rotatable protective lids or cover structures A1 or A2 for 1171M1, B and A1 or A2 for 1171M2, and A1, A2 and B for 1171M3.

FIG 11M4-1 provides a top view of the CBD without any lid, but where a rotational shaft or post for supporting rotation of a lid can be seen while FIGS 11M4-2 to 11M4-4 provide three different types of example lids that may be used alone or in combination. In other alternatives, other lid and substrate configurations are possible include configurations with sliding stops and locks that must be released to allow opening or exposure of the substrate. In some such variations the locking may be mechanical in nature while in other situations it may be magnetic

FIG. 11M4-2 shows a top view of an opaque lid with a radial slot that exposes a portion of the disk wherein the disk and lid can be relatively rotated to provide access to any portion of the deposition surface of the CBD for deposition or reading. In alternative configurations, the lid may itself include a second lid or partial lid that provides a cover for the slotted opening where the second lid or cover may be spring biased to a closed configuration that can be opened upon loading of the disk into a reading or writing system or it can be triggered to open via a read or write command being issued.

FIG. 11M4-3 shows a top view of a transparent lid (e.g., formed of quartz or other material that can pass excitation and emission radiation in required wavelength ranges) that is otherwise similar to the lid of 11M4-2 such that the during deposition of samples the surface can be exposed but during subsequent reading the surface can remain coved and protected. In some variations of this embodiment as well as other embodiments, the substrate may also include a slot-like through hole or recessed surface that aligns with the slot of the lid during non-deposition periods. In some variations, a perimeter lip may trace a matching slot like region of the substrate such that when the slots are aligned the working part of the substrate is completely covered, shielded, and perhaps even sealed.

FIG. 11M4-4 illustrates a top view of a lid with an alternative opening in the form of a curved slot that can be paired with a lid of one or both of FIG 11M4-1 and FIG. 11M4-2 to provide access to any single deposition location on the disk for deposition or reading purposes by appropriate relative rotations of the lids and the substrate.

Other CBD variations and lid or cover variations may be utilized in the alternative, for example, some such variation may include CBDs with non-circular shapes, such as squares, rectangles, or hexagons.

### Further Remarks

Some embodiments provide methods and apparatus for concentration of analyte samples in a liquid matrix or medium to improve limits of detection utilizing Raman or resonance Raman along with fluorescence spectroscopy methods ranging from the deep UV above 200 nm to the far IR. In some embodiments, excitation wavelengths below 250 nm have special appeal. Some methods and apparatus use single stage reduction of a carrier matrix while others use sequential concentration of analytes within a sample volume by sequential reduction of the fluid volume in which an analyte is suspended or dissolved while providing little or no reduction or loss of analyte. Liquid samples can be extracted, collected, or otherwise captured during in-line, on-line, at-line, or off-line sampling. Offline samples may be removed for remote testing in industrial, commercial, or research and development applications. All samples or duplicate copies may undergo more extensive remote testing by either transporting a liquid sample or by transporting an analyte sample after its creation and use locally.

Liquid samples can be contained within a flow cell or deposited in single or multiple sequential small volume droplets deposited on a surface. For detection within a flow cell, the cell volume needs to only be large enough to capture the optical excitation volume from the light source and its related optics. The flow cell can be part of the carrier liquid elimination mechanism used in single or sequential processes to achieve high levels of analyte concentration. For detection of droplets originating from a liquid sample, individual droplet sizes can range from a microliter or more but more preferably have volumes of a few nanoliters to tens of nanoliters. The droplets are deposited on non-background producing substrates with a size ideally close in diameter to the excitation light source spot size to lower the limits of detection. Stacking droplets one on top of another with the potential to stack hundreds of droplets all within the original or near original droplet diameter can improve signal to background values by several hundred times or more and yield limits of detection that extend down into the ppt and ppq range for many analytes.

Integration of a sequentially concentrating flow cell into a Raman and/or fluorescence spectrometer includes a flow cell and concentrator configuration which is automated and allows high levels of concentration to be achieved in less than 1 hour; less than 30 minutes; less than 10 minutes, or potentially less than 5 minutes or even 1 minute.

Integration of the droplet deposition device is in most embodiments implemented in the form of an automated device that creates highly localized drop and dry deposits for concentration at the beginning of which, during stacking, or at the end of which Rayleigh, Raman, and/or fluorescence spectral data are collected in less than 1 hour, less than 30 minutes, less than 10 minutes, even less than 5 minutes or even led than 1 minute. The excitation illumination spot needs to be co-located with the droplet deposition stack to obtain optical spectra. Upon completion of a test sequence, in some embodiments, an automated stage moves a substrate surface to a new location to enable a new droplet deposition stack to be formed and the next optical measurement to be taken. In some embodiments, a dispensing device may move while the deposition substrate remains stationary, while in till others relative motion may be achieved in other ways. For example, with a droplet size about 0.1 mm in diameter, a 0.1 mm laser beam spot size may be used, and a separation of droplet stacks, or pitch, of 0.5 mm may be used with enough space available on a 100 mm diameter movable coupon or substrate surface to collect hourly data for over three years before the coupon would need to be replaced or cleaned and reused. This substrate coupon may function as, or enable, a single 100 mm diameter by 1 mm thick "chemical recorder" or physical chemical "hard drive" disc as a permanent archive enabling future retaking of data using improved instruments or software or by other instrumental methods. Of course, in some embodiments, different diameters, different thicknesses, and even different shaped deposition substrates may be used.

Preferred excitation wavelengths are below 10,000 nm, below 5000 nm, below 2000 nm; below 1000 nm, below 600 nm, below 400 nm, below 300 nm, and most preferably below 250 nm. Optical sources include all forms of lasers in any of the above wavelength ranges and include non-laser sources for application to fluorescence or phosphorescence spectrometers.

Some embodiments will preferentially process analyte samples using UV Raman and/or fluorescence spectroscopy with an excitation wavelength that is less than 400 nm, less than 300nm, less than 275 nm, and even more preferably less than 250 nm.

Some embodiments will preferentially process analyte samples using an IR or visible excitation wavelengths that are less than 1600 nm, less than 700 nm, less than 600 nm, and in some cases less than 500 nm.

In some embodiments liquid samples can come directly from one or more on-line, in-line, off-line, or from grabbed samples taken from primary liquid based sources. Liquid samples can also come from samples taken from surfaces using swabs or wipes which are vortexed and/or sonicated to remove material into a liquid medium prior to testing. Liquid samples can also come from aerosol samples collected on filters or aerosol scalpers or concentrators and into liquid medium prior to testing.

In some embodiments, using UV excitation wavelengths (as noted above), emission detections may make use of more than one of Rayleigh, Raman, resonance Raman, and fluorescence as part of a single instrument that may also include one or more of spatial mapping, context imaging, and internal spectral calibration.

Some embodiments avoid sample damage issues by using excitation at low powers, such as less than 1W of laser power (e.g., 0.1W) applied over longer periods, such as greater than 1 microsecond (us) or even 10 us. Some embodiments will also apply the pulses for less than 1 millisecond (ms), 0.5 ms, or even 50 us which is hundreds or even thousands of times (e.g., 15,000 times) less than peak powers of Q-switched or excimer lasers to produce very little sample heating, even for delicate materials such as biological microbes.

Some embodiments provide desiccated mass determinations via calibration from correlating reading from analyte samples being tested with readings from samples of known material deposited with known masses or concentration levels that are provided on substrates that may be the same or different from the CB Hard Drives on which the samples are unknown samples are located.

Some embodiments provide real time analysis and conclusions by providing those conclusions within tens of seconds to tens of minutes (e.g., under 20 minutes) or by providing near real-time results within a fraction of an hour (e.g. 1/3 of hour) to within several hours (e.g. less than 8 hours and more preferably less than four hours or even less than two hours) from the time of initially depositing a volume of a sample to the time of providing a result. More preferably the processing time includes the time from obtaining the liquid sample to the time results are provided. In some embodiments, for the purpose of this disclosure, trace amounts are considered to have concentration levels between 100 parts per billion and 1 part per billion while ultra-trace amounts are considered to have concentrations of under 1 part per billion. In some embodiments, a determination of real-time and near real time may be based on the definitions of such terms as used in the respective industries from which the samples are taken. In some cases, real-time and near-real time are those times which provide results within a useful period of time that allow the determination to aid in setting process parameters for optional efficiency to be within 1%, 2%, 5% or even 10% of an optimal efficiency of a control parameter of interest (real-time) or within 10% - 20% of optimal efficiency for a control parameter of interest (near-real time). Examples of control parameters of interest include: (1) amount of energy usage, (2) amount of energy usage that is more than a minimum or optimal usage, (3) cost of energy used, (4) excess cost of energy used, (5) amount or cost of one process consumable (e.g. chemical or biologic treatment materials), (6) excess amount or cost of such consumable that is above an optimal if the process were operating with maximum reasonable efficiency), (7) amount of one or more pollutants that are generated, and/or (8) an extra amount of such pollutants that are generated.

Though the teachings of the present application are applicable to a large range of spectroscopic inspection methods, apparatus, and systems, and though some claims may be directed to this large range, some implementations are believed to provide enhanced benefits. Some of these implementations include detection, identification, and quantification of materials in the various applications noted above while using by deep UV Raman, deep UV resonance Raman, and/or deep UV fluorescence spectroscopy with additional methods to reduce limits of detection, using chemical concentration, background reduction, or enhanced detection techniques, and methods to provide quantification of unknown analytes. Other implementations include the providing/creating of a physical archive of chemical and biological (CB) samples on a CB hard drive-type storage structure or device that enables further analysis in the future possibly using enhanced equipment, programs, data comparison sources, or facilities. Other implementations may apply the concentration methods to detection methods using near UV, visible, and/or infrared (IR), including near, mid, and far IR.

Preferred instrumentation to perform these tests with sizes, weights, power consumption, cost profile, reliability, and weeks to months of unattended autonomous operation that allows effective cost recovery profiles and limited space usage.

In the following, additional embodiments are described:
Embodiment 1. A method of spectroscopic analysis, comprising:
   (a) obtaining a liquid sample to be tested from at least one source;
   (b) providing a substrate;
   (c) depositing a total volume of the liquid sample to a predefined location on the substrate;
   (d) allowing or causing at least a portion of the liquid to evaporate from the sample to provide an analyte sample for testing;
   (e) directing excitation radiation on to the analyte sample;
   (f) detecting resulting emission radiation coming from the analyte sample; and
   (g) analyzing emission radiation spectra to provide a determination concerning the presence of any biological or chemical analyte of interest.
Embodiment 2. The method of embodiment 1, wherein the depositing of (c) comprises depositing a defined volume of the liquid to a predefined location on the substrate where the defined volume is less than the total volume to be deposited and thereafter repeating the depositing operation one or more times to deposit the total volume on to the predefined location on the substrate, and wherein the allowing or causing of (d) occurs between depositing operations or after the total volume has been deposited.
Embodiment 3. The method of embodiment 1, wherein the depositing of (c) comprises (i) making one or more deposits; and (ii) allowing or causing different analytes to spatially separate after deposition to create a distributed analyte sample,
   wherein the detecting of (f) comprises detecting resulting emission radiation coming from different regions of the distributed analyte sample, and
   wherein the analyzing of (g) comprises analyzing emission radiation spectra from the different regions of the distributed analyte sample to provide an integrated determination concerning the presence of any biological or chemical analytes of interest.
Embodiment 4. The method of embodiment 1, further comprising:
   (h) repeating the depositing of (c) and the allowing or causing of (d) to form a plurality of additional liquid samples wherein the depositing of each liquid sample will occur at a different defined location on the substrate relative to previously formed analyte samples and wherein the substrate with its plurality of analyte samples will form a permanent chemical and biological storage device or disc (CBD) that may be accessed in the future for providing updated analysis.
Embodiment 5. The method of embodiment 1, wherein the depositing the total volume of (c) comprises depositing a known volume of the liquid sample to a predefined location on a substrate, wherein the known volume of the liquid sample is selected from the group consisting of: (A) a volume equal to a total volume to be deposited, and (B) a volume less than the total volume to be deposited and repeating the deposition as necessary until the total volume is deposited,
   wherein the allowing or causing of (d) comprises allowing time between depositing operations or after the total volume is deposited;
   wherein different analytes in the deposited volume have positionings selected from a group consisting of: (A) substantially overlapping positions or symmetric positioning driven primarily by deposition and drying and (B) spatially offset positions driven by a separating motive force in combination with deposition and drying,
   wherein the detecting of (f) comprises detecting resulting emission radiation coming from the analyte sample as a whole or separately from multiple regions of the sample; and
   wherein the method further comprises:
   (h) repeating at least operations of the depositing of (c) and allowing of (d) for a plurality of additional liquid samples wherein the depositing of each liquid sample occurs at a different defined location on the substrate relative to previously dispensed analyte sample locations and wherein the substrate with its plurality of analyte samples form a permanent chemical and biological storage device or disc (CBD).
Embodiment 6. The method of embodiment 1, wherein the substrate is capable of receiving a plurality of spatially separated deposits of liquids from samples to be tested,
   wherein the depositing of the total volume of (c) occurs via deposition of a plurality of sub-microliter volumes which are overlaid on locations of previously deposited volumes,
   wherein the allowing or causing of (d) comprises providing a drying time between dispensing successive droplets to allow for at least partial evaporation of the liquid so that liquid at the dispensing location does not continue to increase in volume with each successive overlaid volume,
   wherein the overlaid volumes provide thickening of the analyte deposition wherein a diameter of analyte deposition is between 50 µm and 500 µm,
   wherein the excitation radiation of (e) comprises radiation selected from the group consisting of deep ultraviolet, near ultraviolet, visible, and infrared;
   wherein the detected emission radiation of (f) comprises radiation selected from the group consisting of Rayleigh, Raman, resonance Raman, native fluorescence, and photoluminescence emission radiation.
Embodiment 7. The method of any of embodiments 1 - 5, wherein the determination comprises a quantification of any biological or chemical analytes of interest.
Embodiment 8. The method of any of embodiments 1 - 5 or 7, wherein (g) provides a preliminary determination and wherein the method additionally comprises repeating operations at least once and analyzing the emission radiation spectra from the repeated operations to provide a refined determination concerning the presence and quantification of at least one biological or chemical analyte of interest, wherein the repeated operations comprise operations selected from the group consisting of (A) at least (e) and (f); (B) at least (d), (e), and (f); and (C) at least (c), (d), (e), and (f).
Embodiment 9. The method of embodiment 8, wherein at least one repeating of (f) comprises detection of a different type of emission radiation than used during the initial operation of (f), and wherein at least one repeating of (e) comprises use of a different wavelength range of excitation radiation than was used during the initial operation of (e).
Embodiment 10. The method of any of embodiments 1 - 5, 7, 8, or 9, wherein the excitation radiation comprises radiation selected from the group consisting of: (A) between 200 nm and 12 microns, (B) between 1046 nm and 200 nm, (C) less than 400 nm, (D) less than 300 nm, (E) less than 275 nm, and (F) less than 250 nm; and wherein the detecting of emission radiation comprises detection of radiation selected from the group consisting of: (A) Raman emission, (B) resonance Raman emission, (C) surface plasmon resonance enhancement Raman emission, (D) native fluorescence emission, (E) Rayleigh emission, and (F) phosphorescence emission.
Embodiment 11. The method of any of embodiments 1 - 5 or 7 - 10, wherein substantially complete evaporation of the liquid from the sample has occurred at a time of detecting.
Embodiment 12. The method of any of embodiments 1 - 5 or 7 - 10, wherein at a time of detecting, the amount of liquid remaining in the analyte sample (as compared to the original amount of liquid present) being tested is selected from the group consisting of: (A) less than 50%, (B) less than 20%, (C) less than 10%, (D) less than 5%, (E) less than 2%, (F) less than 1%, (G) less than 0.5 percent, (H) less than 1%, and (I) less than 0.1%.
Embodiment 13. The method of any of embodiments 1, 3 - 5, or 7 - 12, wherein the total volume is deposited in a single deposition.
Embodiment 14. The method of any of embodiments 1, 3 - 5, or 7 - 12, wherein the deposited volume is created from a plurality of separate depositions wherein at least one deposition has a volume selected from the group consisting of: (A) less than 1 nL, (B) less than 2 nL, (C) less than 5 nL, (D) less than 10 nL, (E) less than 20 nL, (F) less than 50 nL, (G) less than 100 nL, (H) less than 200 nL, (I) less than 500 nL, (J) less than 1 uL, and (K) less than 2 uL.
Embodiment 15. The method of any of embodiments 1, 3 - 5, 7 - 12 or 14, wherein the deposited volume is created from a plurality of separate depositions and wherein a number of the plurality of depositions is selected from the group consisting of: (A) at least 2 depositions, (B) at least 4 depositions, (C) at least 8 depositions, (D) at least 16 depositions, (E) at least 32 depositions, (F) at least 64 depositions, (G) at least 128 depositions, and (H) at least 256 depositions.
Embodiment 16. The method of either of embodiments 14 or 15, wherein successive depositions have targeted deposition locations that are selected from the group consisting of (A) more than 90% of subsequent droplets have deposition areas that overlay the combination of areas occupied by prior depositions by an amount selected from the group consisting of: (i) more than 50%, (ii) more than 70%, (iii) more than 90%, (iv) more than 95%, and (v) more than 99%, and (B) more than 90% of subsequent droplets have central droplet locations selected from the group consisting of (i) within 8 um of the average center location for previously dispensed droplets, (ii) within 4 um of the average center location for previously dispensed droplets, (iii) within 2 um of the average center location for previously dispensed droplets, (iv) within 1 um of the average center location for previously dispensed droplets.
Embodiment 17. The method of any of embodiments 14 - 16, wherein at least one successive deposition has a substantially smaller volume than that of a previous deposition, where the smaller volume is selected from the group consisting of: (A) no more than 70% of that of the previous deposition, (B) no more than 50% of that of the previous deposition, (C) no more than 30% of that of the selected previous deposition.
Embodiment 18. The method of any of embodiments 1 - 5 or 7 - 17, wherein the substrate has a property selected from the group consisting of: (A) a smooth surface, (B) depressions defining potential droplet locations, (C) regions where analyte depositions are to exist have higher hydrophilicity than surrounding than surrounding peripheral regions, (D) regions where analyte depositions are to exist have lower hydrophobicity than surrounding peripheral regions, (E) no production of interfering background emissions during exposure so excitation radiation, (F) regions where analyte deposition are intended have chromatographic surfaces, and (G) structured plasmonic features.
Embodiment 19. The method of any of embodiments 1 - 5 or 7 - 18, wherein the liquid comprises an aqueous solution.
Embodiment 20. The method of any of embodiments 1 - 5 or 7 - 19, wherein the analyte sample comprises a plurality of analyte samples.
Embodiment 21. The method of embodiment 20, wherein the plurality of analyte samples are extracted from a material undergoing a process and wherein the determination made for each analyte sample is used, at least in part, to make a decision selected from the group consisting of: (A) leaving processing parameters and operations unchanged, (B) making a change to at least one processing parameter, (C) making a change to at least one processing operation, (D) making a determination as to when to perform a subsequent test, (E) making a determination concerning ending the process.
Embodiment 22. The method of embodiment 3, wherein the spatial separation comprises chromatographic separation.
Embodiment 23. The method of any of embodiments 1 - 5 or 7 - 22, wherein the sample comprises water extracted during a wastewater treatment stage.
Embodiment 24. The method of any of embodiments 1 - 5 or 7 - 23, wherein the liquid sample comprises a plurality of samples which originate from a plurality of different sources.
Embodiment 101. A system for spectroscopic analysis, comprising:
   (a) a substrate;
   (b) means for depositing a total volume of a liquid sample to a predefined location on the substrate;
   (c) means for allowing or causing at least a portion of the liquid to evaporate from the liquid sample to provide an analyte sample for testing;
   (d) means for directing excitation radiation on to the analyte sample;
   (e) means for detecting resulting emission radiation coming from the analyte sample; and
   (f) means for analyzing emission radiation spectra to provide a determination concerning the presence of at least one biological or chemical analyte of interest.
Embodiment 102. The system of embodiment 101, wherein the means for depositing of (b) comprises means for depositing a defined volume of a liquid to a predefined location on the substrate where the defined volume is less than a total volume to be deposited and means for operating the means for depositing multiple times to deposit the total volume on to the predefined location on the substrate while allowing time between depositing operations or after the total volume has been deposited to allow at least a portion of the liquid to evaporate to leave an analyte sample.
Embodiment 103. The system of embodiment 101, additionally comprising means for allowing or causing different analytes to spatially separate after deposition to create a distributed analyte sample, wherein the means of depositing of (b) comprises means for depositing a known volume of the liquid sample one or more times to deposit the total volume to a predefined location on the substrate,
   wherein the means for detecting of (e) comprises means for detecting resulting emission radiation coming from different regions of the distributed analyte sample; and
   wherein the means for analyzing or (f) comprises means for analyzing emission radiation spectra from the different regions of the distributed analyte sample to provide an integrated determination concerning the presence of any biological or chemical analytes of interest.
Embodiment 104. The system of embodiment 101, further comprising:
   (g) means for operating the means of depositing of (b) and the means for allowing or causing of (c) a plurality of times to create a plurality of additional analyte samples at different defined locations on the substrate relative to previously created analyte samples wherein the substrate with its plurality of depositions comprises a permanent chemical and biological storage device or disc (CBD).
Embodiment 105. The system of embodiment 101, wherein the means for depositing of (b) comprises means for depositing a known volume of the liquid sample one or more times to deposit the total volume to a predefined location on the substrate wherein positions of different analytes in the deposited volume is selected from a group consisting of: (A) substantially overlapping positions or symmetric positions driven primarily by deposition and drying, and (B) spatially offset positions driven by a separating motive force in combination with deposition and drying,
   wherein the means for detecting of (e) comprises means detecting emission radiation coming from the analyte sample as a whole or from multiple regions of the sample, and
   wherein the system additionally comprises:
      (g) means for operating the means of (b) and (c) a plurality of times to create a plurality of additional analyte samples at different defined locations on the substrate relative to previously created analyte samples wherein the substrate with its plurality of depositions comprises a permanent chemical and biological storage device or disc (CBD).
Embodiment 106. The system of embodiment 101, wherein the substrate of (a) is capable of receiving a plurality of spatially separated deposits of liquids from samples to be tested;
   wherein the means for depositing of (b) comprises means for depositing a first sub-microliter volume of liquid and a means for sequentially depositing a plurality of at least partially overlaying additional volumes upon previously dispensed deposits,
   wherein the means for allowing or causing of (c) provides a drying time between successive deposits that allows for at least partial evaporation of the liquid so that liquid at the dispensing location does increase in volume with each successive deposition, and wherein the overlaying of depositions provides for thickening of the analyte deposition wherein a diameter of analyte deposition resulting from the deposition of the total volume is between 50 µm and 500 µm,
   wherein the means for directing excitation radiation of (d) provides radiation having a wavelength in the deep ultraviolet, near ultraviolet, visible, or infrared portions of the spectrum, and
   wherein the means for detecting detects at least one of Rayleigh, Raman, resonance Raman, native fluorescence, or photoluminescence emission radiation.
Embodiment 107. The system of any of embodiments 101 - 105, wherein the system additionally comprises means for receiving a testable liquid which can be accessed by the means for dispensing and can thereafter be deposited by the means for dispensing.
Embodiment 108. The system of any of embodiments 101 - 105 or 107, wherein the means for determining is further configured to quantify an amount of at least one detected biological or chemical analyte of interest.
Embodiment 109. The system of any of embodiments 101 - 105, 107 or 108, wherein the determination of the means of (f) provides a preliminary determination and the system is configured to repeat operations at least once to provide a refined determination concerning the presence and quantification of at least one biological or chemical analyte of interest wherein the means to undergo repeated operation comprise means selected from the group consisting of (A) at least (e) and (f); (B) at least (d), (e), and (f); and (C) at least (c), (d), (e), and (f) .
Embodiment 110. The system of embodiment 109, wherein the system is configured such that at least one repeating of the operation of the means of (e) comprises use of a different type of emission radiation than used during a previous operation of the means of (e) and at least one repeating of the operation of the means of (d) comprises providing a different wavelength range of excitation radiation than was used during a previous operation of the means of(d).
Embodiment 111. The system of any of embodiments 101 - 105 or 107 - 110, wherein the means of (d) is configured to provide excitation radiation selected from the group consisting of: (A) between 200 nm and 12 microns, (B) between 200 nm and 1046 nm, (C) less than 400 nm, (D) less than 300 nm, less than 275 nm, and (E) less than 250 nm, and wherein (e) is configured to detect emission radiation selected from the group consisting of: (A) Raman emission, (B) resonance Raman, (C) surface plasmon resonance enhancement Raman, (D) fluorescence emission, (E) Rayleigh emission, (F) phosphorescence emission.
Embodiment 112. The system of any of embodiments 101 - 105 or 107 - 111, wherein the system is configured such that substantially complete evaporation occurs prior to an operation of the means of (d) and (e).
Embodiment 113. The system of any of embodiments 101 - 105 or 107 - 111, wherein the system is configured to operate the means of (d), (e), and (f) when, the amount of liquid remaining in the analyte sample (as compared to the original amount of liquid present) being tested is selected from the group consisting of: (A) less than 50%, (B) less than 20%, (C) less than 10%, (D) less than 5%, (E) less than 2%, (F) less than 1%, (G) less than 0.5 percent, (H) less than 1%, and (I) less than 0.1%.
Embodiment 114. The system of any of embodiments 101, 103 - 105, or 107 - 113, wherein the system is configured to operate the means of (b) only once to provide the total volume from a single deposition.
Embodiment 115. The system of any of embodiments 101, 103 - 105, or 107 - 113, wherein the system is configured to operate the means of (b) a plurality of times to provide a deposited volume that is created from a plurality of depositions, wherein at least one of the plurality of depositions is configured to have a volume selected from the group consisting of: (A) less than 1 nL, (B) less than 2 nL, (C) less than 5 nL, (D) less than 10 nL, (E) less than 20 nL, (F) less than 50 nL, (G) less than 100 nL, (H) less than 200 nL, (I) less than 500 nL, (J) less than 1 uL, and (K) less than 2 uL.
Embodiment 116. The system of any of embodiments 101, 103 - 105, 107 - 113 or 115, wherein the system is configured such that a plurality of depositions is used in depositing the total volume, wherein the plurality is selected from the group consisting of: (A) at least 2 depositions, (B) at least 4 depositions, (C) at least 8 depositions, (D) at least 16 depositions, (E) at least 32 depositions, (F) at least 64 depositions, (G) at least 128 depositions, and (H) at least 256 depositions.
Embodiment 117. The system of either of embodiments 115 or 116, wherein the system is configured to provide successive depositions having targeted deposition locations that are selected from the group consisting of (A) more than 90% of subsequent droplets have deposition areas that overlay the combination of areas occupied by prior depositions by an amount selected from the group consisting of: (i) more than 50%, (ii) more than 70%, (iii) more than 90%, (iv) more than 95%, and (v) more than 99%, and (B) more than 90% of subsequent droplets have central droplet locations selected from the group consisting of (i) within 8 um of the average center location for previously dispensed droplets, (ii) within 4 um of the average center location for previously dispensed droplets, (iii) within 2 um of the average center location for previously dispensed droplets, (iv) within 1 um of the average center location for previously dispensed droplets.
Embodiment 118. The system of any of embodiments 115 - 117, wherein the system is configured such that the at least one successive deposition has a substantially smaller volume than that of a selected previous deposition, where the smaller volume is selected from the group consisting of: (A) no more than 70% of that of the previous deposition, (B) no more than 50% of that of the previous deposition, (C) no more than 30% of that of the previous deposition.
Embodiment 119. The system of any of embodiments 101 - 105 or 107 - 118, wherein the substrate has a property selected from the group consisting of: (A) a smooth surface, (B) depressions defining potential droplet locations, (C) regions where analyte depositions are to exist having higher hydrophilicity than the surrounding peripheral regions, (D) regions where analyte depositions are to exist have lower hydrophobicity than surrounding peripheral regions, (E) no production of interfering background emissions during exposure to excitation radiation, (F) at least some regions where analyte deposition are intended have chromatographic surfaces, and (G) structured plasmonic features.
Embodiment 120. The system of any of embodiments 101 - 105 or 107 - 119, wherein the liquid comprises an aqueous solution.
Embodiment 121. The system of any of embodiments 101 - 105 or 107 - 120, wherein the analyte sample comprises a plurality of analyte samples.
Embodiment 122. The system of embodiment 121, wherein the analyte sample is extracted from a material undergoing a process and wherein the system is configured such that the determination made for the analyte sample is used, at least in part, to make a decision selected from the group consisting of: (A) leaving processing parameters and operations unchanged, (B) making a change to at least one processing parameter, (C) making a change to at least one processing operation, (D) making a determination as to when to perform a subsequent test, (E) making a determination concerning ending the process.
Embodiment 123. The system of embodiment 103, wherein the means for allowing or causing provides for chromatographic separation.
Embodiment 124. The system of any of embodiments 101 - 105 or 107 - 123, wherein the spectroscopic analysis system is incorporated into a wastewater treatment facility and wherein a sample to undergo spectroscopic analysis comprises water extracted during a wastewater treatment operation.
Embodiment 125. The system of any of embodiments 101 - 105 or 107 - 124, wherein the system is configured such that the sample to be tested comprises a plurality of samples which originate from a plurality of different sources.
Embodiment 201. A spectroscopic analysis apparatus, comprising:
   (a) a substrate;
   (b) a computer-controlled dispenser for depositing a total volume of a liquid sample to a predefined location on the substrate;
   (c) an evaporation promoter selected from the group consisting of at least one of: (1) a substrate heater, (2) an air heater, (3) an air chiller, (4) a desiccant, (5) at least one air filter, (6) an exhaust fan, (7) an intake fan, and (8) a wait time for promoting evaporation of the liquid from the deposited liquid sample to provide an analyte sample for testing;
   (d) a source of excitation radiation controlled to direct the excitation radiation at the predefined location on the substrate when the analyte sample is present;
   (e) at least one optical component and a detector for capturing emission radiation in a plurality of selected spectral bands arising from an interaction between the excitation radiation and the analyte sample; and
   (f) a computer programmed to compare detected emission radiation with spectral emission data from samples of known materials of interest to determine presence of at least one material of interest in the analyte sample.
Embodiment 202. The apparatus of embodiment 201, wherein the computer-controlled dispenser of (a) is configured for depositing a plurality of overlaid known volume droplets to obtain a total deposited volume wherein a time between dispensing of successive droplets is controlled, and
   wherein the evaporation promoter of (c) comprises a promoter selected from the group consisting of at least one of: (1) a substrate heater, (2) an air heater, (3) an air chiller, (4) a desiccant, (5) at least one air filter, (6) an exhaust fan, and (7) an intake fan, and (8) a controlled wait time for promoting evaporation of the deposited liquid to provide an analyte sample for testing.
Embodiment 203. The apparatus of embodiment 201, wherein the substrate of (a) comprises at least one of an analyte separation promoter, and
   wherein the evaporation promoter and the analyte separation promoter are configured to operate in conjunction to provide an analyte sample with reduced or eliminated liquid and with a desired level of separation of different analytes.
Embodiment 204. The apparatus of embodiment 201, further comprising:
   (g) at least one reservoir for holding at least one liquid sample to be tested,
   (h) at least one computer-controlled stage for relatively positioning the substrate and the dispenser for aligning the dispenser with a predefined substrate location and thereafter depositing a known volume of the liquid sample to the predefined location using one or more deposition operations to deposit the total volume;
   (i) a drain for receiving excess liquid sample material from the dispenser after dispensing the total volume; and
   (j) a computer control system configured for loading sample liquid from the reservoir into the dispenser, for depositing the fresh liquid samples at predefined locations on the substrate using the dispenser, for operating the evaporation promoter, for operating the source of excitation radiation, and for operating the at least one detector, and
   wherein the substrate of (a) is capable of permanently holding a plurality of spaced apart, deposited analyte samples.
Embodiment 205. The apparatus of embodiment 201, wherein the substrate of (a) comprises a substrate capable of receiving a plurality of spatially separated deposits of liquids from samples to be tested;
   wherein the dispenser of (b) comprises a digital micro syringe or pipette with a dispensing needle for depositing defined sub-microliter volumes of liquid,
   wherein the apparatus additionally comprises a programmed controller for providing dispensing commands to the dispenser and for controlling the evaporation promoter to allow at least partial drying of deposited liquid between dispensing successive droplets so that liquid at the predefined location does continue to increase in volume with the dispensing of each successive droplet;
   wherein a diameter of analyte deposition is between 50 µm and 500 µm after dispensing the total volume,
   wherein the source of excitation radiation of (d) provides at least one of deep ultraviolet, near ultraviolet, visible, or infrared radiation, and
   wherein the emission radiation comprises at least one of Rayleigh, Raman, resonance Raman, native fluorescence, or photoluminescence emission radiation wherein the at least one optical component aids in directing the emission radiation along an optical path from the analyte deposition location to the detector.
Embodiment 206. The apparatus of embodiment 201, wherein the apparatus additionally comprises a liquid flow system that provides a liquid sample from a process chamber or a storage chamber to a reservoir that allows access to the liquid by the dispenser such that the dispenser can be loaded with the liquid and thereafter made to deposit the liquid onto the selected location of the substrate.
Embodiment 207. The apparatus of any of embodiments 201 - 204 or 206, wherein the computer is further programmed to quantify an amount of detected biological or chemical analytes of interest.
Embodiment 208. The apparatus of any of embodiments 201 - 204 or 206, wherein the determination of (f) provides a preliminary determination concerning a material of interest and wherein the apparatus further comprising a controller programmed to operate at least one of the detector and the computer multiple times to provide a refined determination concerning the presence and quantification of any biological or chemical analytes of interest.
Embodiment 209. The apparatus of any of embodiments 201 - 204 or 206 - 208, wherein the determination of (f) provides a preliminary determination concerning a material of interest and wherein the apparatus comprises a controller programmed to operate at least the source, the detector, and the programmed computer multiple times to provide a refined determination concerning the presence and quantification of any biological or chemical analytes of interest.
Embodiment 210. The apparatus of any of embodiments 201 - 204 or 206 - 208, wherein the determination of (f) provides a preliminary determination concerning a material of interest and wherein the apparatus comprises a controller that is programmed to operate at least the dispenser, the source, the detector, and the programmed computer multiple times to provide a refined determination concerning the presence and quantification of any biological or chemical analytes of interest.
Embodiment 211. The apparatus of any of embodiments 201 - 204 or 206 - 208, wherein the apparatus is configured such that a repeating of operation of the detector at least once comprises detection of a different type of emission radiation than used during a previous operation of the detector.
Embodiment 212. The apparatus of any of embodiments 201 - 204 or 206 - 208, wherein the apparatus is configured such that a repeating of the operation of the source and the detector comprises the source providing a different wavelength range of excitation radiation than was used during a previous operation of the source and the detector.
Embodiment 213. The apparatus of embodiment 201, wherein the source provides excitation radiation selected from the group consisting of: (A) between 200 nm and 12 microns, (B) between 1046 nm and 200 nm, (C) less than 400 nm, (D) less than 300 nm, less than 275 nm, and (E) less than 250 nm, and wherein the at least one optical element and the detector are configured to detect emission radiation selected from the group consisting of: (A) Raman emission, (B) resonance Raman, (C) surface plasmon resonance enhancement Raman, (D) fluorescence emission, (E) Rayleigh emission, and (F) phosphorescence emission.
Embodiment 214. The apparatus of any of embodiments 201 - 204 or 206 - 213, wherein the apparatus is configured such that substantially complete evaporation occurs prior to an operation of the source of (d) and the detector of (e).
Embodiment 215. The apparatus of any of embodiments 201 - 204 or 206 - 214, wherein the apparatus is configured to operate the source, the detector, and the programmed computer when, the amount of liquid remaining in the analyte sample (as compared to the original amount of liquid present) being tested is selected from the group consisting of: (A) less than 50%, (B) less than 20%, (C) less than 10%, (D) less than 5%, (E) less than 2%, (F) less than 1%, (G) less than 0.5 percent, (H) less than 1%, and (9) less than 0.1%.
Embodiment 216. The apparatus of any of embodiments 201, 203, or 206, wherein the apparatus is configured to operate the dispenser only once to provide a deposited total volume.
Embodiment 217. The apparatus of any of embodiments 201, 203 - 204, or 206 - 215, wherein the apparatus is configured to operate the dispenser a plurality of times to provide a deposited total volume that is created from a plurality of smaller depositions, wherein at least one of the plurality of depositions is configured to have a volume selected from the group consisting of: (A) less than 1 nL, (B) less than 2 nL, (C) less than 5 nL, (D) less than 10 nL, (E) less than 20 nL, (F) less than 50 nL, (G) less than 100 nL, (H) less than 200 nL, (I) less than 500 nL, (J) less than 1 uL, and (K) less than 2 uL.
Embodiment 218. The apparatus of any of embodiments 201 - 204 or 206 - 217, wherein the apparatus is configured such that a plurality of depositions is used in depositing the total volume, wherein the plurality is selected from the group consisting of: (A) at least 2 depositions, (B) at least 4 depositions, (C) at least 8 depositions, (D) at least 16 depositions, (E) at least 32 depositions, (F) at least 64 depositions, (G) at least 128 depositions, and (H) at least 256 depositions.
Embodiment 219. The apparatus of either of embodiments 217 or 218, wherein the apparatus is configured to provide successive depositions having targeted deposition locations that are selected from the group consisting of (A) more than 90% of subsequent depositions have deposition areas that overlay the combination of areas occupied by prior depositions by an amount selected from the group consisting of: (i) more than 50%, (ii) more than 70%, (iii) more than 90%, (iv) more than 95%, and (v) more than 99%, and (B) more than 90% of subsequent depositions have central droplet locations selected from the group consisting of (i) within 8 um of the average center location for previously dispensed droplets, (ii) within 4 um of the average center location for previously dispensed droplets, (iii) within 2 um of the average center location for previously dispensed droplets, (iv) within1 um of the average center location for previously dispensed droplets.
Embodiment 220. The apparatus of any of embodiments 217 - 219, wherein the apparatus is configured such that at least one successive deposition has a substantially smaller volume than that of a selected previous deposition, where the smaller volume is selected from the group consisting of: (A) no more than 70% of that of the previous deposition, (B) no more than 50% of that of the previous deposition, (C) no more than 30% of that of the previous deposition.
Embodiment 221. The apparatus of any of embodiments 201 -204 or 206- 220, wherein the substrate has a property selected from the group consisting of: (A) a smooth surface, (B) depressions defining potential droplet locations, (C) regions where analyte depositions are to exist having higher hydrophilicity than the surrounding peripheral regions, (D) regions where analyte depositions are to exist have lower hydrophobicity than surrounding peripheral regions, (E) no production of interfering background emissions during exposure to excitation radiation, (F) at least some regions where analyte deposition are intended have chromatographic surfaces, and (G) structured plasmonic features.
Embodiment 222. The apparatus of any of embodiments 201 -204 or 206- 221, wherein the liquid comprises an aqueous solution.
Embodiment 223. The apparatus of any of embodiments 201 -204 or 206- 222, wherein the analyte sample comprises a plurality of analyte samples.
Embodiment 224. The apparatus of embodiment 223, wherein the plurality of analyte samples are extracted from a material undergoing a process and wherein the apparatus is configured such that the determination made for the analyte sample is used, at least in part, to make a decision selected from the group consisting of: (A) leaving processing parameters and operations unchanged, (B) making a change to at least one processing parameter, (C) making a change to at least one processing operation, (D) making a determination as to when to perform a subsequent test, (E) making a determination concerning ending the process.
Embodiment 225. The apparatus of embodiment 203, wherein the substrate provides for chromatographic separation.
Embodiment 226. The apparatus of any of embodiments 201- 225, wherein the spectroscopic analysis apparatus is incorporated into a wastewater treatment facility and wherein a sample to undergo spectroscopic analysis comprises water extracted during a wastewater treatment stage.
Embodiment 227. The apparatus of any of embodiments 201- 226, wherein the apparatus is configured such that the sample to be tested comprises a plurality of samples which originate from a plurality of different sources.

## Claims

1. A method of spectroscopic analysis, comprising:
(a) obtaining a liquid sample to be tested from at least one source;
(b) providing a substrate;
(c) depositing a total volume of the liquid sample to a predefined location on the substrate;
(d) allowing or causing at least a portion of the liquid to evaporate from the sample to provide an analyte sample for testing;
(e) directing excitation radiation on to the analyte sample;
(f) detecting resulting emission radiation coming from the analyte sample; and
(g) analyzing emission radiation spectra to provide a determination concerning the presence of any biological or chemical analyte of interest.

2. The method of claim 1, wherein the depositing of (c) comprises depositing a defined volume of the liquid to a predefined location on the substrate where the defined volume is less than the total volume to be deposited and thereafter repeating the depositing operation one or more times to deposit the total volume on to the predefined location on the substrate, and wherein the allowing or causing of (d) occurs between depositing operations or after the total volume has been deposited.

3. The method of claim 1, wherein the depositing of (c) comprises (i) making one or more deposits; and (ii) allowing or causing different analytes to spatially separate after deposition to create a distributed analyte sample,
wherein the detecting of (f) comprises detecting resulting emission radiation coming from different regions of the distributed analyte sample, and
wherein the analyzing of (g) comprises analyzing emission radiation spectra from the different regions of the distributed analyte sample to provide an integrated determination concerning the presence of any biological or chemical analytes of interest.

4. The method of claim 1, further comprising:
(h) repeating the depositing of (c) and the allowing or causing of (d) to form a plurality of additional liquid samples wherein the depositing of each liquid sample will occur at a different defined location on the substrate relative to previously formed analyte samples and wherein the substrate with its plurality of analyte samples will form a permanent chemical and biological storage device or disc (CBD) that may be accessed in the future for providing updated analysis.

5. The method of claim 1, wherein the depositing the total volume of (c) comprises depositing a known volume of the liquid sample to a predefined location on a substrate, wherein the known volume of the liquid sample is selected from the group consisting of: (A) a volume equal to a total volume to be deposited, and (B) a volume less than the total volume to be deposited and repeating the deposition as necessary until the total volume is deposited,
wherein the allowing or causing of (d) comprises allowing time between depositing operations or after the total volume is deposited;
wherein different analytes in the deposited volume have positionings selected from a group consisting of: (A) substantially overlapping positions or symmetric positioning driven primarily by deposition and drying and (B) spatially offset positions driven by a separating motive force in combination with deposition and drying,
wherein the detecting of (f) comprises detecting resulting emission radiation coming from the analyte sample as a whole or separately from multiple regions of the sample; and
wherein the method further comprises:
(h) repeating at least operations of the depositing of (c) and allowing of (d) for a plurality of additional liquid samples wherein the depositing of each liquid sample occurs at a different defined location on the substrate relative to previously dispensed analyte sample locations and wherein the substrate with its plurality of analyte samples form a permanent chemical and biological storage device or disc (CBD).

6. The method of claim 1, wherein the substrate is capable of receiving a plurality of spatially separated deposits of liquids from samples to be tested,
wherein the depositing of the total volume of (c) occurs via deposition of a plurality of sub-microliter volumes which are overlaid on locations of previously deposited volumes,
wherein the allowing or causing of (d) comprises providing a drying time between dispensing successive droplets to allow for at least partial evaporation of the liquid so that liquid at the dispensing location does not continue to increase in volume with each successive overlaid volume,
wherein the overlaid volumes provide thickening of the analyte deposition wherein a diameter of analyte deposition is between 50 µm and 500 µm,
wherein the excitation radiation of (e) comprises radiation selected from the group consisting of deep ultraviolet, near ultraviolet, visible, and infrared;
wherein the detected emission radiation of (f) comprises radiation selected from the group consisting of Rayleigh, Raman, resonance Raman, native fluorescence, and photoluminescence emission radiation.

7. The method of claim 1, wherein (g) provides a preliminary determination and wherein the method additionally comprises repeating operations at least once and analyzing the emission radiation spectra from the repeated operations to provide a refined determination concerning the presence and quantification of at least one biological or chemical analyte of interest, wherein the repeated operations comprise operations selected from the group consisting of (A) at least (e) and (f); (B) at least (d), (e), and (f); and (C) at least (c), (d), (e), and (f).

8. The method of claim 1, wherein the excitation radiation comprises radiation selected from the group consisting of: (A) between 200 nm and 12 microns, (B) between 1046 nm and 200 nm, (C) less than 400 nm, (D) less than 300 nm, (E) less than 275 nm, and (F) less than 250 nm; and wherein the detecting of emission radiation comprises detection of radiation selected from the group consisting of: (A) Raman emission, (B) resonance Raman emission, (C) surface plasmon resonance enhancement Raman emission, (D) native fluorescence emission, (E) Rayleigh emission, and (F) phosphorescence emission.

9. The method of claim 1, wherein at a time of detecting, the amount of liquid remaining in the analyte sample (as compared to the original amount of liquid present) being tested is selected from the group consisting of: (A) less than 50%, (B) less than 20%, (C) less than 10%, (D) less than 5%, (E) less than 2%, (F) less than 1%, (G) less than 0.5 percent, (H) less than 1%, and (I) less than 0.1%.

10. The method of claim 1, wherein the deposited volume is created from a plurality of separate depositions and wherein a number of the plurality of depositions is selected from the group consisting of: (A) at least 2 depositions, (B) at least 4 depositions, (C) at least 8 depositions, (D) at least 16 depositions, (E) at least 32 depositions, (F) at least 64 depositions, (G) at least 128 depositions, and (H) at least 256 depositions.

11. The method of claim 10, wherein successive depositions have targeted deposition locations that are selected from the group consisting of (A) more than 90% of subsequent droplets have deposition areas that overlay the combination of areas occupied by prior depositions by an amount selected from the group consisting of: (i) more than 50%, (ii) more than 70%, (iii) more than 90%, (iv) more than 95%, and (v) more than 99%, and (B) more than 90% of subsequent droplets have central droplet locations selected from the group consisting of (i) within 8 um of the average center location for previously dispensed droplets, (ii) within 4 um of the average center location for previously dispensed droplets, (iii) within 2 um of the average center location for previously dispensed droplets, (iv) within 1 um of the average center location for previously dispensed droplets.

12. The method of claim 1, wherein the substrate has a property selected from the group consisting of: (A) a smooth surface, (B) depressions defining potential droplet locations, (C) regions where analyte depositions are to exist have higher hydrophilicity than surrounding than surrounding peripheral regions, (D) regions where analyte depositions are to exist have lower hydrophobicity than surrounding peripheral regions, (E) no production of interfering background emissions during exposure so excitation radiation, (F) regions where analyte deposition are intended have chromatographic surfaces, and (G) structured plasmonic features.

13. A spectroscopic analysis apparatus, comprising:
(a) a substrate;
(b) a computer-controlled dispenser for depositing a total volume of a liquid sample to a predefined location on the substrate;
(c) an evaporation promoter selected from the group consisting of at least one of: (1) a substrate heater, (2) an air heater, (3) an air chiller, (4) a desiccant, (5) at least one air filter, (6) an exhaust fan, (7) an intake fan, and (8) a wait time for promoting evaporation of the liquid from the deposited liquid sample to provide an analyte sample for testing;
(d) a source of excitation radiation controlled to direct the excitation radiation at the predefined location on the substrate when the analyte sample is present;
(e) at least one optical component and a detector for capturing emission radiation in a plurality of selected spectral bands arising from an interaction between the excitation radiation and the analyte sample; and
(f) a computer programmed to compare detected emission radiation with spectral emission data from samples of known materials of interest to determine presence of at least one material of interest in the analyte sample.

14. The apparatus of claim 13, wherein the computer-controlled dispenser of (a) is configured for depositing a plurality of overlaid known volume droplets to obtain a total deposited volume wherein a time between dispensing of successive droplets is controlled, and
wherein the evaporation promoter of (c) comprises a promoter selected from the group consisting of at least one of: (1) a substrate heater, (2) an air heater, (3) an air chiller, (4) a desiccant, (5) at least one air filter, (6) an exhaust fan, and (7) an intake fan, and (8) a controlled wait time for promoting evaporation of the deposited liquid to provide an analyte sample for testing.

15. The apparatus of claim 13, wherein the plurality of analyte samples are extracted from a material undergoing a process and wherein the apparatus is configured such that the determination made for the analyte sample is used, at least in part, to make a decision selected from the group consisting of: (A) leaving processing parameters and operations unchanged, (B) making a change to at least one processing parameter, (C) making a change to at least one processing operation, (D) making a determination as to when to perform a subsequent test, (E) making a determination concerning ending the process.
